# EUROPEAN PATENT APPLICATION

(11) **EP 1 702 983 A2**
(43) Date of publication of application: **20.09.2006**
(21) Application number: 06001413.1
(22) Date of filing: 13.04.2001
(51) Int. Cl.: C12N 15/11, C12N 15/74

(54) **Tissue-specific and pathogen-specific toxic agents, ribozymes, DNAzymes and antisense oligonucleotides and methods of use thereof**

(30) Priority: 13.04.2000 US 548449; 07.12.2000 US 251810 P
(62) Divisional of application: 01926973.7
(71) Applicant: MEDICAL UNIVERSITY OF SOUTH CAROLINA, Charleston, South Carolina 29425 (US); THE PENN STATE RESEARCH FOUNDATION, University Park, PA 16802 (US)
(72) Inventor: Norris, James, S., Mount Pleasant, SC 29464 (US); Clawson, Gary, A., Bethesda, MD 20816 (US); Westwater, Caroline, Hollywood, SC 29449 (US); Schofield, David, Hollywood, SC 29449 (US); Schmidt, Michael, G., Mount Pleasant, SC 29464 (US); Hoel, Brian, Charleston, SC 29401 (US); Dolan, Joseph, Mount Pleasant, SC 29646 (US); Pan, Wei-Hua, Hummelstown, PA 17036 (US)
(74) Representative: Ford, Timothy James

(57) **Abstract**

The present invention relates to the discovery, identification and characterization of toxic agents which are lethal to pathogens and methods for targeting such toxic agents to a pathogen or pathogen infected cells in order to treat and/or eradicate the infection. In particular, the present invention relates to toxic agents which target bacteria at different stages of the bacterial life cycle, which are delivered alone or in combination to bacteria or bacteria-infected cells. The invention relates to toxic agents which are lethal to diseased cells and methods for targeting such toxic agents to a diseased cell in order to treat and/or eradicate the disease. The present invention relates to promoter elements which are pathogen-specific or tissue-specific and the use of such promoter elements to achieve pathogen-specific or tissue-specific expression of the toxic agent(s) and/or ribozyme(s) of the present invention. Specifically, the invention relates to the delivery of one or more toxic gene products, antisense RNAs, or ribozymes, or combination thereof. The invention provides a novel system by which multiple pathogenic targets may be simultaneously targeted to cause the death of a pathogen, or cell infected with a pathogen. Further, the invention has important implications in the eradication of drug-resistant bacterium and bacterial pathogens. The invention provides a novel system by which multiple targets may be simultaneously targeted to cause the death of a diseased cell. The invention has important implications in the eradication of drug-resistant pathogens (such as antibiotic resistant bacteria) and drug-resistant diseased cells (such as drug-resistant cancer cells).

### Annex to the application documents - subsequently filed sequences listing

SEQUENCE LISTING

<110> MEDICAL UNIVERISTY OF SOUTH CAROLINA AND THE PENN STATE RESEARCH
FOUNDATION

<120> TISSUE SPECIFIC AND PATHOGEN SPECIFIC TOXIC AGENTS, RIBOZYMES,
DNAZYMES, AND ANTISENSE OLIGONUCLEOTIDES, AND METHODS OF USE
THEREOF

<130> P34849EP1/TF

<140> EP 06001413.1
<141> 2001-04-13

<150> 60/251,810
<151> 2000-12-07

<150> 09/548,449
<151> 2000-04-13

<160> 85

<170> PatentIn version 3.0

<210> 1

<211> 66

<212> DNA

<213> Artificial

<220>

<223> LEASHI promoter

<220>

<221> misc_feature

<223> LEASH1 promoter

<400> 1
gatcctcaga aaattatttt aaatttccaa ttgacattgt gagcggataa caatataatg 60

tgtgga 66

<210> 2

<211> 74

<212> DNA

<213> Artificial

<220>

<223> modified rrnb promoter

<400> 2
agaaagcaaa aataaatgct tgacactgta gcgggaaggc gtataatgga attgtgagcg 60

gataacaatt caca 74

<210> 3

<211> 492

<212> DNA

<213> Artificial

<220>

<223> proC promoter

<400> 3
actcgcggat catcttcacc atcggccgca actcctgcgg gatatcctcg tcctcctcct 60

ccaccggcac ccccatggta gcggccagct cgcgccctgc ctgggaaagc tgtacatgct 120

gatcggcggc gtcggtgccg gcggccgggt cttccgcctg ctcggcggtg ccggtccgtg 180

cggccttggc gtccgcggcg gcgcgcgatg agggcggcac ctgggtggtg atccagccac 240

tgagggtcaa cattccagtc actccgggaa aaatggaatt cttccattgg atcggcccac 300

gcgtcgcgaa cttgagcccc cttttcgtcg ccccttgaca gggtgcgaca ggtagtcgca 360

gttgtttgac gcaagtcact gattggaaac gccatcggcc tgtcagaaat ggtcgttgcc 420

agacctatgg ctggcacccg catcgcggct gcgttaccct tactcctgtt gtgcctttaa 480

cctagcaagg ac 492

<210> 4

<211> 1113

<212> DNA

<213> Artificial

<220>

<223>  proC promoter

<400>  4
aattcctcga agtccttgcg ctgcttgtcg ttcatgatgt cgtagatcag cgcatgcacc      60

tgcttgtgtt ccagcggtgg caggttgatc cggcgtacat cgccatccac ccggatcatg     120

ggtggcaggc cggcggagag gtgcaggtcc gaagcgccct gtttggcact gaaggcgagc     180

agctcggtaa tatccatggg actccccaat tacaagcaag caggtagaat gccgccaaag     240

ccgccgtctc ggacaaggaa aacaccggat gagccagggt gcttccagga cacgcgtggt     300

gtcctgcgcc agacgcggaa cctcgacact ggaacaggaa gatggccatc gaggccggcg     360

gtttcgaggg cgtcgagccg acgccgaccg cacttccata gggcgcaggt aatgtccacg     420

atagcagaga atattgcaaa ggttgccgcg cgcatccgtg aggcagcgca agctgcgggg     480

cgcgatccgg ccacggtcgg cctgctcgcc gtgagcaaga ccaagcccgc cgccgcggtg     540

cgcgaggcgc acgccgccgg ccttcgcgac ttcggcgaaa actacctgca ggaggccctc     600

ggcaagcagg ccgaactggc cgacctgccc ttgaactggc acttcatcgg ccccatccag     660

tcgaacaaga cgcggcccat cgccgagcat ttccagtggg tgcactcggt ggaccggttg     720

aagatcgcgc agcgcctgtc ggagcaacgc ccggccgggc tgccgcccct gaatgtctgc     780

ctgcaggtca acgtcagcgg cgaagccagc aagtccggct gcgcccccga ggacctgccg     840

gccctggccg aggccgtgaa gcaactgccc aacctccgat tgcgtggcct gatggccatc     900

cccgaaccca ccgccgaacg cgccgcgcaa cacgccgcgt tcgcccgcct gcgcgaactg     960

ctgctggacc tgaaccttgg cctggacacc ctgtccatgg gcatgagcga cgacctcgag    1020

gcagccatcg gcgaaggtgc gacctgggtc cgcatcggta ccgccctgtt cggcgcccgc    1080

gactacggcg cgccggcttc ttgaatgaat ccc                                 1113

<210>  5

<211>  66

<212>  DNA

<213>  Artificial

<220>

<223>  ARC promoter

<400>  5
ctagagctat tgatgtggat caacattgtc cactagccgc tgccgcctaa tctccagaat      60

tgtgag                                                                  66

<210>  6

<211>  2120

<212>  DNA

<213>  Staphylococcus aureus

<400>  6
ttatttagca ggaataatta gccagattat cgagggagtt ccagggcaat ccaaacattg      60

ttatatatgc atttataaaa ttttcaagat aatttattat tcataccctt gccctttgtt     120

tcaaaattat gccctttttt tgcccttgga aacaaccaca ctcctaaatt aataggtggt     180

gtggtttgat catttataat ataacataaa aacaaccacc cagtaactag tatgagtggc     240

gtagcgacta taacaactct atgttatcaa gatatatgta tatgagtgat gacaaggaag     300

atgtctcctg tgagaccaac agccagatat atggcctctt gccgggctat atagttcact     360

cctactatat acacatgtaa ttataacata aaaaaataga caagtaccga agtacctgcc     420

taaataacaa caagattaac atgtgaataa tggaaataaa aagtcagccc gaaggctaac     480

ttacgaatag atgaaaattt gaacacattg ctgtgtctaa aatgattata gcataaataa     540

cgaatatttc cagctcgaaa ttaatatatt gtaataataa tattttatat ctttgttaat     600

aattatttaa ttgatttaca taaataataa ttgtaaaatt aatttgtaat cgattgcaaa     660

taagttatag gagaaaataa aatgaataaa aaactattaa caaaaacatt gatagcaagt     720

gctttagttt taacaacagt aggttcaggt tttcattctt cttcaaatta taatggtatt     780

aataacgttg aaaaagctga gcaaacgaca gataacgcat tgtggaaaaa tgtaagagac     840

gctttaaaag acgcgaatat tatcgataaa acagataatg aaaatgtcaa ggttacgtat     900

aaaatagaaa atggtggaga aaataccata gaaggaacag ttaatttaga aaatattagt     960

acttcaaaca atcctaaaat aaaccctcaa aatgttacaa aaattaatat aactagaaaa    1020

aatccgaact accctaatat tgatgctaat aatacatgga aaaaattacc agaaaaattg    1080

aaagaaaaaa atatagtgga acaacggcga caatgtttca atcttaagta cagaccctaa    1140

agatgagact gtattcggta aagtaggaga agataaatca aacgtaagca atagatacat    1200

caatcctaaa gatataaatg aattcaaatc actaaaaata cttttttccg aggcagatta    1260

ctcctgcctc tttctttgaa cagtgatatc ttctgatcta tgtaacactc aattacttca    1320

gattctttac ctttaacttc ctttaattca tttctctcta tctcctcaaa aagttgtgct 1380

ttttgatttg tgattggagt tgggcgtttt ttcatcgcgt tgtttcaatt cctttttaag 1440

gtattctaat tctcttctag tcatatcaat tgttttttta cttctcacct ttagtgaaat 1500

actcttatcc tttctcttct tgcgttaatg ttgctaatta gtataaaata catgcgccca 1560

tatattccaa tggtaggaca tttaattctg gattttcagc tattttcata aatctattat 1620

ctgataattt gcttaatcca attttcaagc catagcctaa attccccatc cactaagtca 1680

ttttgtttca tatggtttta atctacggcc aatctcaaag atagattgac cagcgatgtt 1740

taaagtcata tttcacggat ccacatttac gataaacata tctagttaca caatattatc 1800

ccttactgca acacaggacg tttctcagcg taaaaaacac cactagaaag tgactttaaa 1860

gaatataact aattcaaact tatattaatt aatattcttt aaatgaccac tcacactttg 1920

ttttttgcta tttgtaactt taaaatgttg tttgaaatct atattttttt gatatagctc 1980

cctatgtaac aaacaatttt taattaatat atatttaaac aagtcaattt agagatcggt 2040

taattcgatt catttaaata atatttatac attctatatg taaacgttta cacatttgaa 2100

gtaaggagaa ttaaaaatga 2120

<210> 7

<211> 177

<212> DNA

<213> Artificial

<220>

<223> P1 pac site

<400> 7
ccactaaaaa gcatgatcat tgatcactct aatgatcaac atgcaggtga tcacattgcg 60

gctgaaatag cggaaaaaca aagagttaat gccgttgtca gtgccgcagt cgagaatgcg 120

aagcgccaaa ataagcgcat aaatgatcgt tcagatgatc atgacgtgat cacccgc 177

<210> 8

<211> 45

<212> DNA

<213> Artificial

<220>

<223>  DicF1 molecule

<400>  8
caggcgacag gtatagtttc tctccgattt gtgcctgtcg cctgc                    45


<210>  9

<211>  14

<212>  DNA

<213>  Artificial



<220>

<223>  DNAZyme

<220>

<221>  misc_feature

<223>  n=  a, t, g or c


<400>  9
ggaggtgnnn natg                                                      14


<210>  10

<211>  15

<212>  DNA

<213>  Artificial



<220>

<223>  dnazyme

<400>  10
ggctagctac aacga                                                     15


<210>  11

<211>  23

<212>  RNA

<213>  Artificial

<220>

<223>  hbv rna

<400>  11
uuucguccuc acggacucau cag                                              23


<210>  12

<211>  67

<212>  RNA

<213>  Artificial



<220>

<223>  hbvrz881

<400>  12
gguuccagga uccaagagag ucugaugagu ccgugaggac gaaacuccac agugaauucc      60

aaggguc                                                                67


<210>  13

<211>  15

<212>  DNA

<213>  Artificial



<220>

<223>  HBV sRz 469

<400>  13
ttctcggggg cttgg                                                       15


<210>  14

<211>  15

<212>  DNA

<213>  Artificial



<220>

<223>  HBV sRz 469

<400> 14
gggcgcacct cttta 15

<210> 15

<211> 14

<212> DNA

<213> Artificial

<220>

<223> HBV sRz 777

<400> 15
tctgcctaat ctct 14

<210> 16

<211> 15

<212> DNA

<213> Artificial

<220>

<223> HBV sRz 885

<400> 16
tggagttact cgttt 15

<210> 17

<211> 15

<212> DNA

<213> Artificial

<220>

<223> HBV m1RZ 247

<400> 17
cgcagcaggt ggagc 15

<210> 18

<211> 15

<212>  DNA

<213>  Artificial

<220>

<223>  HBV m1RZ 355

<400>  18
cgcgggacgc tttgt                                                        15

<210>  19

<211>  15

<212>  DNA

<213>  Artificial

<220>

<223>  Pol I sRZ70

<400>  19
tcgcaatgtc atact                                                        15

<210>  20

<211>  15

<212>  DNA

<213>  Artificial

<220>

<223>  Pol I sRz 353

<400>  20
tcatgctgac ccgtc                                                        15

<210>  21

<211>  15

<212>  DNA

<213>  Artificial

<220>

<223>  Pol I sRz458

<400>  21
ccatgctgca aagat                                                        15


<210>  22

<211>  15

<212>  DNA

<213>  Artificial



<220>

<223>  Pol I sRz 595

<400>  22
atatcctcag ctcag                                                        15


<210>  23

<211>  15

<212>  DNA

<213>  Artificial



<220>

<223>  PTEN sRz 281

<400>  23
tgaagaccaa cccac                                                        15


<210>  24

<211>  15

<212>  DNA

<213>  Artificial



<220>

<223>  PTEN sRz425

<400>  24
tttattgcag gggca                                                        15

<210>  25

<211>  16

<212>  DNA

<213>  Artificial

<220>

<223>  PTEN sRz499

<400>  25
aaaagggaga caattt                                                    16

<210>  26

<211>  15

<212>  DNA

<213>  Artificial

<220>

<223>  PTEN sRz774

<400>  26
atatattccc aattc                                                     15

<210>  27

<211>  14

<212>  DNA

<213>  Artificial

<220>

<223>  PTEN sRz774

<400>  27
gtagagttcc caca                                                      14

<210>  28

<211>  15

<212>  DNA

<213>  Artificial

<220>

<223>  PTEN sRz127

<400>  28
cagaaagacg aaggt                                                        15

<210>  29

<211>  15

<212>  DNA

<213>  Artificial

<220>

<223>  PTEN m1Rz439

<400>  29
ggaacaatag atgat                                                        15

<210>  30

<211>  15

<212>  DNA

<213>  Artificial

<220>

<223>  PTEN m1Rz760

<400>  30
gcaaatttta aggca                                                        15

<210>  31

<211>  15

<212>  DNA

<213>  Artificial

<220>

<223>  PTEN m1Rz760

<400>  31
gtggtgataa aagta                                                        15


<210>  32

<211>  15

<212>  DNA

<213>  Artificial



<220>

<223>  PTEN m1Rz304

<400>  32
tgagagacaa taaca                                                        15


<210>  33

<211>  15

<212>  DNA

<213>  Artificial



<220>

<223>  PTEN m1Rz414

<400>  33
tagaacttaa aaccc                                                        15


<210>  34

<211>  15

<212>  DNA

<213>  Artificial



<220>

<223>  PTEN m1Rz414

<400>  34
atttgtgcat ttatt                                                        15


<210>  35

<211>  15

<212>  DNA

<213>  Artificial

<220>

<223>  PTEN m2Rz961

<400>  35
tactcaccca caaaa                                                        15

<210>  36

<211>  31

<212>  DNA

<213>  Artificial

<220>

<223>  HPV16

<400>  36
tgtggtaagg ctagctacaa cgatttctgg g                                      31

<210>  37

<211>  17

<212>  DNA

<213>  Artificial

<220>

<223>  DNAzyme

<400>  37
cccagaaagt taccaca                                                      17

<210>  38

<211>  17

<212>  DNA

<213>  Artificial

<220>

<223>  DNAzyme

<400>  38
ttaccacagt tatgcac                                                    17


<210>  39

<211>  17

<212>  DNA

<213>  Artificial



<220>

<223>  DNAzyme

<400>  39
gacgtgaggt atatgac                                                    17


<210>  40

<211>  17

<212>  DNA

<213>  Artificial



<220>

<223>  DNAzyme

<400>  40
catagtatat agagatg                                                    17


<210>  41

<211>  17

<212>  DNA

<213>  Artificial



<220>

<223>  DNAzyme

<400>  41
attagtgagt atagaca                                                    17

<210> 42

<211> 17

<212> DNA

<213> Artificial

<220>

<223> DNAzyme

<400> 42
tatagtttgt atggaac 17

<210> 43

<211> 17

<212> DNA

<213> Artificial

<220>

<223> DNAzyme

<400> 43
tataaggggt cggtgga 17

<210> 44

<211> 17

<212> DNA

<213> Artificial

<220>

<223> DNAzyme

<400> 44
gtggaccggt cgatgta 17

<210> 45

<211> 17

<212> DNA

<213>  Artificial

<220>

<223>  DNAzyme

<400>  45
gtgcttttgt gtgtctg                                                    17

<210>  46

<211>  17

<212>  DNA

<213>  Artificial

<220>

<223>  DNAzyme

<400>  46
gcctctgcgt ttaggtg                                                    17

<210>  47

<211>  17

<212>  DNA

<213>  Artificial

<220>

<223>  DNAzyme

<400>  47
gcctccacgt ctgcaac                                                    17

<210>  48

<211>  17

<212>  DNA

<213>  Artificial

<220>

<223>  DNAzyme

<400>  48
gccgttgtgt gaaatag                                                    17


<210>  49

<211>  17

<212>  DNA

<213>  Artificial



<220>

<223>  DNAzyme

<400>  49
gtggaagggt cgttgct                                                    17


<210>  50

<211>  17

<212>  DNA

<213>  Artificial



<220>

<223>  DNAzyme

<400>  50
ttctcggggt cgcttgg                                                    17


<210>  51

<211>  17

<212>  DNA

<213>  Artificial



<220>

<223>  DNAzyme

<400>  51
tctgcctaat catctct                                                    17


<210>  52

<211>  17

<212>  DNA

<213>  Artificial

<220>

<223>  DNAzyme

<400>  52
tggagttact ctcgttt                                                    17

<210>  53

<211>  17

<212>  DNA

<213>  Artificial

<220>

<223>  DNAzyme

<400>  53
tgcaatgttt caggacc                                                    17

<210>  54

<211>  17

<212>  DNA

<213>  Artificial

<220>

<223>  DNAzyme

<400>  54
gccactgtgt cctgaag                                                    17

<210>  55

<211>  17

<212>  DNA

<213>  Artificial

<220>

<223>  DNAzyme

<400>  55
gtggaccggt cgatgta                                                    17


<210>  56

<211>  17

<212>  DNA

<213>  Artificial



<220>

<223>  DNAzyme

<400>  56
aaagatgcct ccacgtc                                                    17


<210>  57

<211>  17

<212>  DNA

<213>  Artificial



<220>

<223>  DNAzyme

<400>  57
tgtgtgaaat agaaaaa                                                    17


<210>  58

<211>  17

<212>  DNA

<213>  Artificial



<220>

<223>  DNAzyme

<400>  58

gtggaagggt cgttgct 17

<210> 59

<211> 17

<212> DNA

<213> Artificial

<220>

<223> DNAzyme

<400> 59
ctttgaggat ccaacac 17

<210> 60

<211> 17

<212> DNA

<213> Artificial

<220>

<223> DNAzyme

<400> 60
accgttgaat ccagcag 17

<210> 61

<211> 17

<212> DNA

<213> Artificial

<220>

<223> DNAzyme

<400> 61
tgggcactat agaggcc 17

<210> 62

<211> 17

<212>  DNA

<213>  Artificial

<220>

<223>  DNAzyme

<400>  62
cccagaaagt taccaca                                                    17

<210>  63

<211>  17

<212>  DNA

<213>  Artificial

<220>

<223>  DNAzyme

<400>  63
ttaccacagt tatgcac                                                    17

<210>  64

<211>  17

<212>  DNA

<213>  Artificial

<220>

<223>  DNAzyme

<400>  64
catagtatat agagatg                                                    17

<210>  65

<211>  17

<212>  DNA

<213>  Artificial

<220>

<223>  DNAzyme

<400>  65
attagtgagt atagaca                                                    17


<210>  66

<211>  17

<212>  DNA

<213>  Artificial



<220>

<223>  DNAzyme

<400>  66
tatagtttgt atggaac                                                    17


<210>  67

<211>  31

<212>  DNA

<213>  Artificial



<220>

<223>  DNAzyme

<400>  67
tgtggtaagg ctagctacaa cgatttctgg g                                    31


<210>  68

<211>  31

<212>  DNA

<213>  Artificial



<220>

<223>  DNAzyme

<400>  68
gtgcataagg ctagctacaa cgatgtggta a                                    31

<210> 69

<211> 31

<212> DNA

<213> Artificial

<220>

<223> DNAzyme

<400> 69
gtcatatagg ctagctacaa cgactcacgt c 31

<210> 70

<211> 31

<212> DNA

<213> Artificial

<220>

<223> DNAzyme

<400> 70
catctctagg ctagctacaa cgaatactat g 31

<210> 71

<211> 31

<212> DNA

<213> Artificial

<220>

<223> DNAzyme

<400> 71
tgtctatagg ctagctacaa cgatcactaa t 31

<210> 72

<211> 31

<212> DNA

<213> Artificial

<220>

<223>  DNAzyme

<400>  72
gttccatagg ctagctacaa cgaaaactat a                                    31

<210>  73

<211>  31

<212>  DNA

<213>  Artificial

<220>

<223>  DNAzyme

<400>  73
tccaccgagg ctagctacaa cgacccttat a                                    31

<210>  74

<211>  31

<212>  DNA

<213>  Artificial

<220>

<223>  DNAzyme

<400>  74
tacatcgagg ctagctacaa cgacggtcca c                                    31

<210>  75

<211>  31

<212>  DNA

<213>  Artificial

<220>

<223>  DNAzyme

<400> 75
cagacacagg ctagctacaa cgaaaaagca c 31

<210> 76

<211> 31

<212> DNA

<213> Artificial

<220>

<223> DNAzyme

<400> 76
cacctaaagg ctagctacaa cgagcagagg c 31

<210> 77

<211> 31

<212> DNA

<213> Artificial

<220>

<223> DNAzyme

<400> 77
gttgcagagg ctagctacaa cgagtggagg c 31

<210> 78

<211> 31

<212> DNA

<213> Artificial

<220>

<223> DNAzyme

<400> 78
ctatttcagg ctagctacaa cgaacaacgg c 31

<210> 79

<211> 31

<212>  DNA

<213>  Artificial

<220>

<223>  DNAzyme

<400>  79
agcaacgagg ctagctacaa cgaccttcca c                                    31

<210>  80

<211>  31

<212>  DNA

<213>  Artificial

<220>

<223>  DNAzyme dz879

<400>  80
gagagtaagg ctagctacaa cgatccacag t                                    31

<210>  81

<211>  31

<212>  DNA

<213>  Artificial

<220>

<223>  DNAzyme mDz879

<400>  81
gagagtaagc ctagctacta cgatccacag t                                    31

<210>  82

<211>  31

<212>  DNA

<213>  Artificial

<220>

<223>  DNAzyme LS1

<400>  82
tgccgggagg ctagctacaa cgatcggggc t                                    31

<210>  83

<211>  31

<212>  DNA

<213>  Artificial

<220>

<223>  DNAzyme LS2

<400>  83
cacagaaagg ctagctacaa cgaagactga a                                    31

<210>  84

<211>  31

<212>  DNA

<213>  Artificial

<220>

<223>  DNAzyme LS3

<400>  84
tatagaaggg ctagctacaa cgaagccctg c                                    31

<210>  85

<211>  31

<212>  DNA

<213>  Artificial

<220>

<223>  DNAzyme mLS2

<400>  85
cacagaaagc ctagctacta cgaagactga a                                    31

## Description

### 1. INTRODUCTION

The present invention relates to the discovery, identification, and characterization of toxic agents which are lethal to pathogens and methods for targeting and delivering such toxic agents to a pathogen or pathogen infected cell in order to treat and/or eradicate an infection. In particular, the present invention relates to toxic agents which target bacteria at different stages of the bacterial life cycle, which are delivered alone or in combination to bacteria or bacteria infected cells. In particular, the invention relates to a phage delivery vehicle approach for the treatment of bacterial infections in humans and animals. The invention also relates to toxic agents which are lethal to diseased cells and methods for targeting such toxic agents to a diseased cell in order to treat and/or eradicate the disease. The present invention relates to promoter elements which are pathogen-specific. The invention also relates to promoter elements which are used to achieve pathogen-specific or tissue specific expression of the toxic agent(s) and/or ribozyme(s) of the present invention. Specifically, the invention relates to the delivery of one or more toxic gene products, antisense RNAs, or ribozymes, or combination thereof. The invention provides a novel system by which multiple pathogenic targets may be simultaneously targeted in order to kill a pathogen or pamogen-infeeted cell or render it less fit. Further, the invention has important implications in the eradication of drug-resistant bacterium and bacterial pathogens. The invention provides a novel system by which multiple targets may be simultaneously targeted to cause the death of a diseased cell or render it less fit. The invention has important implications in the eradication of drug-resistant pathogens (such as antibiotic resistant bacteria) and drug-resistant diseased cells (such as drug-resistant cancer cells). The present invention also relates to DNAzymes, antisense oligonucleotides and ribozymes useful in pharmaceutical compositions for the treatment of viral infections including papillomavirus and hepatitis B.

### 2. BACKGROUND

### 2.1. ANTIMICROBIAL AGENTS

Infectious diseases sicken or kill millions of people each year. Each year in the United States alone, hundreds of thousands of people are infected with resistant bacterial strains that are no longer treatable with drugs like penicillin and vancomycin (Hiramatsu et al, 1997, Morbidity and Mortality Weekly Report 46:624-26). Infections associated with antimicrobial resistance include those acquired in hospitals (nosocomial), such as pneumonia particularly in the young, elderly and immunocompromised), typhoid fever, bacterial meningitis, and tuberculosis. Around the world, nearly 1.5 billion people carry various types of the tuberculosis bacteria and depending on the country, up to 40 percent have proven to be resistant to antibiotics (see, Boyce et al,1997, Epidemiology and prevention of nosocomial infections. In The Staphylococcus in Human Disease. Crassly and Archer E's, Churchill Livingston Inc., New York, NY). It is estimated that in some developed countries, up to 60% of all nosocomial infections result from bacteria resistant to antibiotics. For example, *Pseudomonas aeruginosa,* is of the most common gram-negative bacterium associated with nosocomial infections and outbreaks in bum units. Infections by this organism are associated with high mortality (60%), which is attributed to the high intrinsic resistance of members of this genus to many structurally unrelated antibiotics. Gram-positive bacteria also have a significant impact on infectious diseases. For example, *Staphylococcus aureus,* is a Gram-positive organism which is responsible for about 260,000 hospital acquired infections in the United States which subsequently causes between 60,000 and 80,000 deaths annually (see, Boyce et al, supra).

Although, numerous antimicrobial therapies have been designed to target one or several infectious agents, many therapies show varying degrees of success in eradicating infection. Only a very limited number of new antibiotics have come onto the market in the last decade, yet the number of deadly bacteria that are resistant to these drug therapies has soared. For example, vancomycin is one of the last effective antimicrobial available for the treatment of methicillin-resistant *S. aureus* infection (MRSA). However, vancomycin resistant isolates *S aureus* have now emerged (Hiramatsu et al, *1997,* Morbidity and Mortality Weekly Report 46:62A-26). Additionally, the failure of many of these therapies to target specific infectious agents has lead to overuse or inappropriate use of the therapies, which in turn has lead to the development of drug resistant microbes. The development of drug resistance in many infectious agents has reduced the efficacy and increased the risk of using the traditional antimicrobial therapies.

Accordingly, there is need in the art for novel molecules and novel combinations of molecules that can act as lethal agents in bacteria and which may be delivered to a pathogen, without causing toxicity to the infected host. Further, there is a need in the art for novel methods of targeting particular species of pathogens while leaving the host's beneficial flora intact. The present invention provides such novel products, therapeutics, and methods for delivery which may be used as toxic agents against pathogens such as bacteria.

### 2.2. ANTISENSE

Antisense technology seeks to use RNA molecules which are complementary to (or antisense to) a cellular RNA, for the purpose of inhibiting a cellular RNA from being translated into the encoded protein. In this way, the expression of a specific protein is targeted for down regulation. However, a large number of difficulties exist in the art surrounding antisense technology. Commonly, delivery of an exogenous antisense molecule to the target cell is difficult or impossible to achieve. Further, antisense molecules do not consistently lead to a decrease in protein expression. For example, it has been shown that the expression of antisense RNA in transgenic mice did not invariably lead to a reduction in target RNA molecules, and when reduction in target RNA molecules did occur, it was not predictably paralleled by a reduction in protein. Even when protein levels were reduced sometimes no biological effect was detected (Whitton, J. Lindsay "Antisense Treatment of Viral Infection" *Adv. in Virus Res.* Vol: 44,1994). Thus, there is a need in the art for a delivery system in which antisense molecules may be efficiently delivered to a target cell such as a bacterial pathogen.

### 2.3. RIBOZYMES

A ribozyme is a catalytic RNA molecule that cleaves RNA in a sequence specific manner. A key technical concern in the use of ribozymes as antimicrobial agents is that the ribozyme must be introduced into and expressed by the targeted microbe so that the ribozyme(s) can cleave the targeted RNA(s) inside the microorganism. A second important concern is the tight coupling of transcription and translation in microorganisms which can prevent binding to and cleavage of the bacterial RNA targets. Additionally, bacterial RNAs often have a shorter half life than eukaryotic RNAs, thus lessening the time in which to target a bacterial RNA. The invention described herein addresses these concerns and proves novel therapeutic treatments of bacterial infections using combinations of ribozymes and toxic agents.

### 3. SUMMARY OF THE INVENTION

The present invention provides toxic agents and methods for specifically targeting toxic agents to bacteria or bacteria-infected cells or other pathogens. Toxic agents of the present invention are directed to one or more targets and thus can be used alone or in combination to eradicate bacteria. The invention relates to the delivery of toxic gene products or the combination of ribozymes and toxic gene products for the eradication of a pathogen or diseased cell. Specifically, the invention provides the delivery of one or more toxic proteins, antisense RNA, multi-ribozymes, or nucleic acids encoding the same, or a combination thereof, to a cell, tissue, or subject containing an infectious bacteria or pathogen in order to eradicate such bacteria or pathogen.

The present invention further encompasses the use of a toxic agent and/or ribozymes of the present invention for the treatment of disease, viral infection, parasitic infection and microbial infection. The present invention further relates to a method of treating a subject having a proliferative disease of a specific tissue by inhibiting cell proliferation in the tissue, comprising administering to the subject a toxic agent and/ar ribozyme operably linked to a tissue-specific promoter sequence, which promoter is specific for the diseased tissue, and whereby the ribozyme and/or toxic agent encoded by the nucleic acid is expressed, cell proliferation is inhibited, and the proliferative disease is treated.

The present invention further relates to a method of treating a subject having a pathogenic infection or disease, by inhibiting replication of the pathogen, comprising administering to the subject a toxic agent and/or ribozyme operably linked to a pathogen-specific promoter, whereby the ribozyme and/or toxic agent encoded by the nucleic acid is expressed, the pathogen is inhibited from replicating or is killed or rendered less fit, and the infection or disease is treated. In specific embodiments of the invention, the toxic agents of the invention are useful to treat microbial infections associated with severe burns, cystic fibrosis, cancer, or other immunocompromising conditions. The present invention encompasses the toxic agent(s) and/or ribozyme(s) of the present invention in pharmaceutical formulations.

The present invention further encompasses the use of the toxic agents and/or ribozymes of the present invention for research and screening purposes. In one embodiment of the present invention, the ribozymes and/or toxic agents may be used to screen for viral, microbial, prokaryotic, or eukaryotic gene products or molecules to be targeted in order to effectively inhibit the selected virus or microbial agent or selected cell.

In yet another embodiment, the present invention relates to a novel vector encoding the toxic agent(s) and/or ribozyme(s). The novel vectors of the present invention may be used to engineer a wide variety of toxic agents and/or ribozymes including, but not limited to, tissue-specific, pathogen-specific, Promoter-specific, antimicrobial specific, antiviral specific, anticancer specific, antitumor specific, or target-specific.

In one embodiment, the invention relates to toxic agents which specifically target gene products essential for the survival or life cycle of a pathogen (such as replication, packaging, etc). In one embodiment, the present invention relates to naturally occurring bactericidal addiction system toxins which have been modified to be expressed in the absence of their corresponding addiction system antidote. In another embodiment, the present invention relates to naturally occurring addiction system toxins which have been modified to be expressed at higher levels than their corresponding addiction system antidote. In one example, an addiction system toxin (*e.g., doc, chpBK, kicB,* or *gef*) is used as a toxic agent and is uncoupled from its antidote. In specific embodiments, the invention provides for delivery of toxic agents such as bactericidal proteins (or nucleic acids encoding such toxic agents) by a bacteriophage delivery system. In other specific embodiments, the invention provides novel transfer plasmids encoding toxic agents which may be used in combination with a bacteriophage delivery system in order to treat a bacterial infection in a host.

The invention also relates to antisense RNA which targets essential nucleotide sequences, such as *DicF1* or a *DicF1-*like antisense molecule that specifically target a nucleotide sequence encoding a protein essential for replication or survival.
Further, the invention relates to modified antisense structures with increased stability which act as lethal agents when expressed in bacteria. The invention also relates to toxic sense molecules designed to target essential antisense molecules.

The present invention relates to multi-ribozymes and their use to target RNA in a tissue-specific or pathogen-specific manner for the treatment of disease (such as pathogen infection or cancer). The invention provides multi-ribozymes containing one or more internal trans-acting ribozyme. Trans-acting ribozymes act in a target-specific manner and therefore may act as a toxic agent to a pathogen (such as bacteria) or a selected cell (such as a diseased cell), In accordance with the present invention, the multi-ribozyme may comprise a) a trans-acting ribozyme flanked by 5' and 3' autocatalytically cleaving ribozymes or enhanced autocatalytically cleaving ribozymes; b) a trans-acting ribozyme flanked by either a 5' or 3' autocatalytically cleaving ribozyme; or c) multiple transacting ribozymes, flanked by one or both 5' and 3' autocatalytically cleaving ribozymes or enhanced autocatalytically cleaving ribozymes. Multi-ribozymes of the invention may also be used to deliver one or more toxic agents to a pathogen cell or tissue. Ribozymes useful in the present invention include those described in U.S. Patent 5,824,519 and PCT publications No.WO98/24925, WO97/17433, WO98/24925, WO99/67400, which are incorporated by reference herein in their entirety. In accordance with the present invention the multi-transacting ribozymes may be targeted to the same site on the same RNA, different sites on the same RNA or different RNAs. In accordance with the present invention the multiple toxic agents may be targeted to the same site on the same target (such as a cellular RNA or protein),different sites on the same target or different targets. For example, in certain embodiments a toxic agent (such as an antisense nucleic acid or nucleic acid encoding a toxic protein) may be engineered into a multi-ribozyme in place of a trans-acting ribozyme, or in addition to a trans-acting ribozyme. In this embodiment, the toxic agent is flanked by a 5' and/or 3' autocatalytically cleaving ribozyme.

The invention additionally provides nucleic acids and expression cassettes which encode the toxic agent and/or ribozymes of the invention. These nucleic acids can be used to express the toxic agent(s) and/or ribozyme(s) of the invention at the selected site.

At the molecular genetic level the coding sequence for a toxic agent, ribozyme, or multi-ribozyme of the invention may be placed under the control of one or more of the following genetic elements: a naturally occurring strong, intermediate, or weak constitutively expressed or regulated promoter from the targeted microorganism, or an artificially contrived constitutively expressed or regulated promoter containing either a strong, intermediate or weak consensus sequence that accords the desired levels of ribozyme and/or toxic agent expression. The present invention relates to promoter elements which are pathogen-specific. The invention relates to promoter elements which are used to achieve pathogen-specific expression of the toxic agents of the present invention. The present invention also relates to promoter elements which are tissue-specific. The invention relates to promoter elements which are used to achieve tissue-specific expression of the toxic agents of the present invention.

In one embodiment, the nucleic acids comprise a tissue-specific promoter operably linked to a sequence encoding one or more toxic agent(s). In another embodiment the nucleic acids comprise a pathogen-specific promoter operably linked a sequence encoding one or more toxic agent(s). In accordance with the present invention, toxic agents of the invention may act on the same or different targets.

The present invention relates to a toxic agent and/or a trans-acting ribozyme which targets any cellular, viral, bacterial, fungal, or other single cellular or multicellular organism from any known taxonomic family, genus, or species. Another embodiment of the invention relates to a toxic agent which is lethal or toxic to a pathogen such as a bacteria, fungus, yeast, diseased cell.

The targets of the antimicrobial ribozyme therapeutics described herein are the RNAs of invading or normal flora microorganisms. The targets of the antimicrobial toxic agent therapeutics described herein include RNAs, proteins, genes and other molecules of invading or normal flora microorganisms. The invention provides the delivery of a series of ribozymes and/or toxic agents directed towards essential, housekeeping, or virulence genes of one or a series of candidate microorganisms, Inactivation of essential proteins and virulence determinants render the invading microbes inactive or slow their growth, while at the same time, the essential processes of the host are not significantly affected.

The present invention also relates to the delivery of the toxic agents of the invention to cell or pathogen by abiologic or biologic systems. In a specific embodiment, a toxic agent of the invention is delivered to a bacterial cell by a modified bacteriophage capable of infecting a pathogenic bacteria. In a further embodiment, bacteriophage are selected for their ability to infect a particular species or genera of bacteria, and are used to deliver a toxic agent for the eradication of such bacterial species or genera from a host. In a preferred embodiment, the delivery vehicle or nucleic acids native to the delivery vehicle are modified such that they contain insufficient genetic information for the delivery of nucleic acids native to the delivery vehicle. Thus, the modified delivery vehicle *(e.g.,* virion or bacteriophage) can serve as a molecular vehicle that delivers the ribozyme(s) and/or toxic agent(s) of the invention to the target cell or pathogen, but does not deliver replicable nucleic acids native to the delivery vehicle. Alternatively, an abiologic delivery system *(e.g.,* liposomes) can be used to package nucleic acid carrying the genetic elements necessary and sufficient for the proper expression of the ribozyme(s) and/or toxic agent(s). In one embodiment, delivery of a toxic agent to a pathogen is by use of a bacteriophage or other delivery vehicle which targets the pathogen of interest. In one embodiment, a recombinant bacteriophage delivers the toxic agent or nucleic acids encoding the toxic agent to the pathogen.

The present invention provides compositions of matter which has resulted from the development of methods and compositions for the delivery of one or more ribozymes and/or toxic agents directed against fundamental and essential cellular processes specific to a targeted microorganism through an inactivated, altered, virus (virion), bacteriophage, or abiologic delivery vehicles, capable of delivering a nucleic acid comprising the toxic agent(s) and/or ribozyme(s) into the targeted microorganism. The microorganisms may be any virus, nonvirus, bacterium, or lower eukaryotes such as fungi, yeast, parasites, protozoa, or other eukaryotes that may be considered pathogens of humans, animals, fish, plants, or other forms of life. Thus, the invention has important implications in human and veterinary medicine.

In certain preferred embodiments, a toxic agent of the invention is used as an antimicrobial therapeutic. A toxic agent may be used alone, or in combination with one or more other toxic agents. Thus, delivery of a toxic agent to an invading microorganism, kills or render it less fit. A toxic agent may also be used in combination with one or more ribozymes. Further, a combination of ribozymes and toxic agents may be used as an antimicrobial therapeutic.

The toxic agent approaches of the invention offer advances for antimicrobial therapeutics including but not limited to: (1) the bypass of de novo or built-in drug resistance, which sophisticated microbes may have or develop (2) the decreased ability of cells to counteract ribozymes or toxic agents delivered into them, (3) the use of broad RNA targets and non-RNA targets available in microbes that can be attacked in simultaneously (4) the flexibility of custom design of the present delivery vehicle can be readily tailored to different families of organisms or different species of organisms, (5) the ease of assembly construction and manufacture of the modified delivery vehicle, (6) the availability of a variety of methods of administration of the pharmaceutical preparations of the invention such as topically, or via injection, inhalation, or ingestion, etc. (7) the ability to lyophilize the pharmaceutical preparation and thus confer stability to the antimicrobial therapeutic, (8) the reduced immunogenicity of the therapeutic preparations, and (9) the availability of animal test systems that enable the evaluation of the ribozymes and/or toxic agents of the invention. Therefore, the unique delivery approach and an aggressive mechanism for depriving the pathogen essential or important gene products can achieve the timely defeat of pathogen within the infected host Accordingly, the invention has important implication in the eradication of drug-resistant pathogens.

The present invention is also directed to a purified preparation of at least one nucleic acid molecule that specifically hybridizes under physiological conditions to mRNA. encoding at least one viral protein associated with transformation or plasmid copy number control or which hybridizes to a viral polyadenylation signal. A further embodiment of the present invention is directed to a purified preparation of at least one nucleic acid molecule wherein said mRNA is B6/B7 mRNA. A still further embodiment of the invention is directed to a purified preparation of at least one nucleic acid molecule wherein said mRNA is papilloma viral RNA or hepatitis B viral RNA The present invention provides a purified preparation of one or more antisense nucleic acids that specifically hybridize under physiological conditions to papillomavirus B6/E7 mRNA or papillomavirus polyadenylation signal wherein said antisense nucleic acids are antisense oligonucleotides, ribozymes or DNAzymes.

A further embodiment of the present invention is directed to a purified preparation of at least one nucleic acid molecule that specifically hybridizes under physiological conditions to a sequence selected from the group consisting of SEQ ID NOs:AA-BD or a homologous sequence in a related HPV or HBV strain. A homologous sequence in a related HPV or HBV strain is preferably at least about 70% or 75% or 80% or 85% or 86% or 87% or 88% or 89% or 90% or 91% or 92% or 93% or 94% or 95% or 96% or 97% or 98% or 99% identical to a target site identified in the instant disclosure.

A further embodiment of the present invention is directed to a purified preparation of at least one nucleic acid molecule as described above wherein said nucleic acid molecule is a DNAzyme or an antisense oligonucleotide or a ribozyme, either alone or in combination.

A further embodiment of the present invention is directed to a purified preparation of any of the preceding nucleic acid molecules, wherein said nucleic acid molecules are made resistant to nuclease degradation by any means known in the art.

In a preferred embodiment, nucleic acid molecules of the present invention are modified at their 3' ends to resist nuclease degradation by inclusion of a 3'-3' inverted T at the 3' ends. The modification at the 3' ends may be to include a 3'-3' inverted thymidine, adenine, guanine or cytosine (inverted T, inverted A, inverted G, inverted C, respectively) at the 3' ends.

A further embodiment of the present invention is directed to pharmaceutical compositions comprising any of the preceding nucleic acid molecules and a pharmaceutically acceptable carrier. In a still further embodiment, any of the pharmaceutical compositions maybe formulated as cosmetic formulations and/or formulations for topical administration. The topical formulations may be in the form of ointments, salves, gels, creams or lotions. Any of the pharmaceutical compositions may be formulated such that the nucleic acid molecules are formulated into a liposome preparation or a lipid preparation. In a further embodiment, the liposome is capable of tissue-specific uptake in the liver. In a still further embodiment said liposome is modified using asialofetuin or one or more sugars.

A still further embodiment is directed to pharmaceutical compositions wherein the nucleic acid molecules of the present invention are formulated in amounts sufficient to produce cytotoxic or cytostatic effects in cells infected with papillomavirus or heptatis B virus. The cells may also be transformed by a papillomavirus or transformed by a hepatitis B virus.

A further embodiment of the present invention is directed to methods of treating papillomavirus-induced conditions or hepatitis-induced conditions comprising administering to a subject any of the preceding pharmaceutical compositions. In a further embodiment said papillomavirns-induced condition is selected from the group consisting of warts (*e.g*., warts of the hands, feet, larynx, and/or flat cervical warts), cervical carcinoma, laryngeal papilloma" condylomata acuminata, epidermodysplasia verruciformis, cervical intraepithelial neoplasia, or any other infection involving a papillomavirus. The methods of the instant invention include the treatment of epithelial cells such as squamous epithelia, cutaneous epithelia and mucosal epithelia, or any other cell infected or which may become infected with a papillomavirus.

A further embodiment of the present invention is directed to methods of administration of any of the preceding pharmaceutical compositions comprising topical application. The administration may be to the cervix or the epidermis. In a further embodiment said administration is to epithelial cells such as squamous epithelia, cutaneous epithelia and mucosal epithelia.

The instant invention is also directed to methods of treating papillomavirus-induced conditions comprising: administering to a subject, by topical application to cells infected with said papillomavirus, one or more of the antisense oligonucleotides, DNAzymes or ribozymes described herein. The method of treating papillomavirus-induced conditions includes cervical application or dermal or epidermal application.

In a still further embodiment of the invention, the methods of treating papillomavirus include treatments of conditions induced by human papillomavirus (HPV).

In a further embodiment, the pharmaceutical compositions of the antisense nucleic acids of the invention may be cosmetic formulations.

The pharmaceutical compositions may be formulated such that the nucleic acids of the invention are present in amounts sufficient to produce a cytotoxic or cytostatic effect in cells infected with a wart virus or viruses. In a specific embodiment, the wart virus is a papillomavirus. In a still further embodiment, the cells are transformed by a papillomavirus.

In a preferred embodiment, pharmaceutical compositions of DNAzymes, antisense oligonucleotides or ribozymes of the instant invention are formulated for topical administration Such formulations include ointments, salves, gels, creams, lotions or suppositories.

In a still further embodiment of the instant invention, the pharmaceutical composition of the nucleic acids of the present invention may be formulated into a liposome preparation or a lipid preparation.

A pharmaceutical composition may comprise one or more nucleic acids of the invention selected from the group consisting of antisense oligonucleotides, DNAzymes or ribozymes, wherein said nucleic acids specifically hybridize under physiological conditions to HPV E6/E7 mRNA or an HPV polyadenylation signal, and/or wherein said antisense oligonucleotides or DNAzymes have a 3'-3' inverted thymidine at their 3' ends, and/or wherein said pharmaceutical composition is fomnulated for topical application as an ointment, salve, gel, cream or lotion.

In another preferred embodiment, a purified preparation of one or more nucleic acids of the invention that specifically hybridize under physiological conditions to hepatitis B viral (HBV) RNA are provided, wherein said one or more nucleic acids are autissense oligonucleotides, DNAzymes or ribozymes.

### 4. BRIEF DESCRIPTION OF THE FIGURES

- Figure 1A: Diagram depicts the components of the lacI-regulated broad spectrum promoter.
- Figure 1B: The sequence of the LEASHI promoter (SEQ ID NO:1).
- Figure 1C: The sequence of a modified rmB promoter (SEQ ID NO:2)
- Figure 1D: The sequence of the *Anr* promoter (SEQ ID NO:3).
- Figure 1E: The sequence of the *Proc* promoter (SEQ ID NO:4).
- Figure 1F: The sequence of the *Arc* promoter (SEQ ID NO:5).
- Figure 1G: The sequence of the *TSST-1* promoter (SEQ ID NO:6).
- Figure 2: Diagram of a β-lactamase reporter plasmid.
- Figure 3A-B: Expression vectors for cloning Toxic Agents.
- Figure 4: Assay for lethality of Toxic Agents
- Figure 5: Growth of *E. coli* harboring a *doc* expression plasmid.
- Figure 6A-B: Structure of a Transfer Plasmid.
- Figure 7: Delivery Efficiency of the Transfer Plasmid by the P1 bacteriophage vehicle to *E. coli.*
- Figure 8: Scheme for generation of the P1 pac site knockout
- Figure 9: Identification and confirmation of the P1 *pac* site knockout by PCR screening.
- Figure 10: Diagram of the pacABC Complementing plasmid.
- Figure 11: Recombination between the P1 pac mutant and the pacABC Complementing plasmid.
- Figure 12: Sequence of the minimal P1 *pac* site (SEQ IS NO:7).
- Figure 13: Immunogenicity of replicating phage in mice.
- Figure 14: Comparison of original and long-circulating P1 phage persistence *in vivo.*
- Figure 15: Treatment of *P. aeruginara* (PA01) infections in embryonated hen eggs.
- Figure 16: In vitro killing of *E*. *coli* EC-4 bacterial cells.
- Figure 17: Treatment of *E*. *coli* EC-4 infection in embryonated hen eggs.
- Figure 18: Sequence of the *DicF1* molecule (SEQ ID N0:8).
- Figure 19: Diagram and nucleotide sequence of the pClip ribozyme cassette.
- Figure 20: Diagram and nucleotide sequence of the pChop ribozyme cassette.
- Figure 21: Schematic diagram of the pSnip ribozyme cassette. pSnip includes sequences of the pClip triple ribozyme cassette, catalytic core targeted ribozymes comprising two linked trans-acting ribozymes, and sequences from the pChop triple ribozyme cassette.

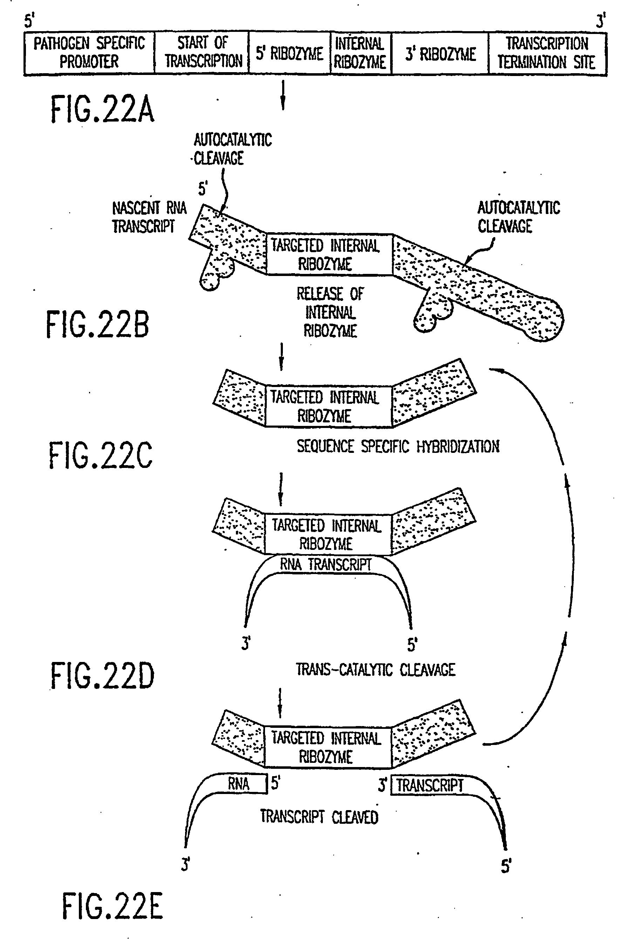

- Figure 22A: A schematic of DNA encoding the ribozyme used in the molecular sequence of events in ribozyme maturation and action.
- Figure 22B: The primary RNA transcript. Autocatalytic cleavage takes place upon completion of transcription.
- Figure 22C: The release of the trans-acting ribozyme. As a direct result of cleavage of the two cis-acting ribozymes, the internal ribozyme containing a reverse and complementary sequence to the mRNA target is released.
- Figure 22D: The sequence specific hybridization of the ribozyme. The internal or trans-acting ribozymes comprise two trans-acting ribozymes linked by a short nucleotide "spacer". Each of the two trans-acting ribozymes contain a sequence that is reverse complementary to the targeted message of the same or at different sites. The ribozyme is synthesized at a concentration sufficient to locate and hybridize to all or substantially all targeted transcripts.
- Figure 22E: The trans-catalytic cleavage. Upon hybridization of the internal trans-acting ribozyme to the targeted mRNA transcript, the internal ribozyme achieves a catalytic topology and cleaves the targeted message. Upon cleavage the trans-acting ribozyme is released and its activity and function are recycled.

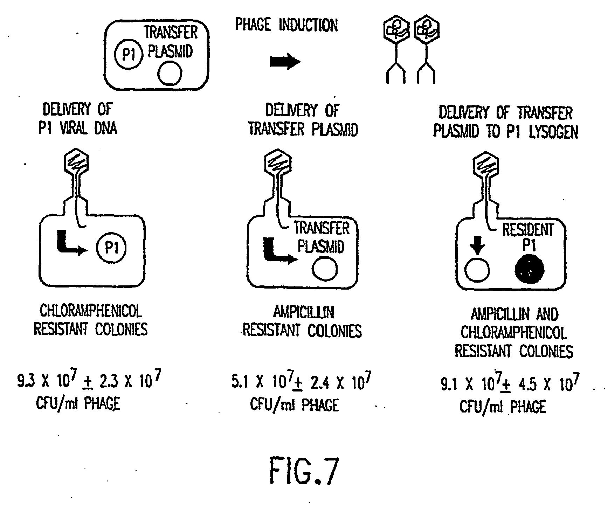

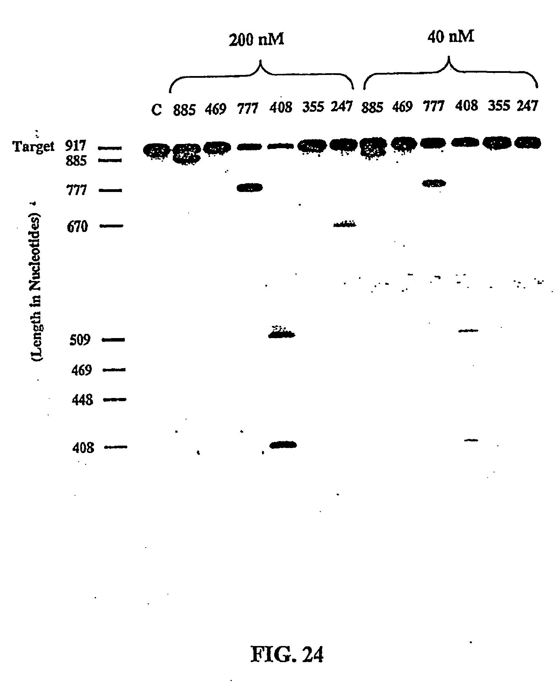

- Figure 23: *In vitro* cleavage analyses of HBV-targeted Rz. The locations of the 6 Rz tested are as shown in Figure 7 of priority provisional application no. 60/251,810 filed December 7, 2000, incorporated by reference herein. Individual Rz were constructed and transcribed in vitro, and mixed with [³²P]-labeled HBV target RNA (917 nt). Incubations were for 30 min at 37 C in 20 mM Tris-HCl (pH 7.4), 5 mM MgCl₂. Following cleavage, products were separated by denaturing PAGE, and results were quantitated using a Phosphor-Imager. The size of the cleavage products is shown to the right, and the concentration ofRz (40 mM or 200 mM) is shown at the top.
- Figure 24: Cleavage analyses of HBV-targeted Rz. Rz and HBV-target RNA were mixed at various ratios (40:1, 40:10, or 40:100, as shown at the top), and incubated as described for various periods (20 sec, 40 sec, 1 min, 3 min, 10 min, 30 min, or 2 h for each ratio, shown from left to right, respectively). After incubation, products were analyzed by denaturing PAGE and quantitated using a Phosphor-Imager.
- Figure 25: Effects of topical application of DNAzymes on papilloma growth in cottontail rabbits. DNAzymes targeted to Shope Papilloma Virus mRNA sites were applied alone (Group L1, Group L2, Group L3) or in combination (Group L1/L2/L3). Results showed that the combination therapy was effective in reducing papilloma volume. Catalytically inactive DNAzyme (Group mL2) was ineffective in reducing papilloma volume.

### 5. DETAILED DESCRIPTION OF THE INVENTION

The present invention provides toxic agent(s) and/or ribozyme(s) and their use in a tissue-specific, target-specific, or pathogen-specific manner for the treatment of disorders and disease related to bacterial, parasitic or viral infections or to cellular proliferation, and cancers. The ribozymes and/or toxic agents of the present invention may be engineered to target one or more specific RNAs contained in a specific cell or tissue in the host. The ribozymes of the present invention may also be engineered to target one or more specific RNAs encoded by a specific pathogen, virus, or microbial agent. The toxic agents of the present invention may also be engineered to target one or more specific RNAs, proteins, or molecules of a specific pathogen, virus, or microbial agent.

The present invention also provides toxic agents which are lethal or toxic to a selected pathogen. In one embodiment of the invention, the toxic agents of the invention comprise toxic proteins which cause lethality to a pathogen or selected cell (*e.g*., a diseased cell) or which render the pathogen or selected cell less fit. In one embodiment, such toxic proteins of the invention are lethal when overexpressed in a pathogen or selected cell. In other embodiments, a toxic protein is an exogenous protein that is toxic when expressed in a pathogen or selected cell. A toxic protein of the invention may further be engineered to have increased toxicity. For example, many methods are known in the art for introducing mutations, deletion, insertions etc. into a known sequence. Thus, optimization of a toxic protein is provided. The invention also provides methods for inhibiting the toxicity of a toxic protein, so that the toxic protein may be produced or manufactured in a producing cell. Inhibiting the toxicity may be performed by any methods known in the art, for example, the toxic protein may be expressed from an inducible promoter which allows expression to be turned on/off under appropriate conditions. A toxic protein may be expressed in a cell without causing lethality in the cell by overexpressing an antidote protein in the same cell. Other methods will be apparent to one skilled in the art and are within the scope of the invention.

The present invention provides toxic agents and methods for specifically targeting toxic agents to bacteria or bacteria-infected cells or other pathogens. Toxic agents of the present invention are directed to one or more targets and thus can be used alone or in combination to eradicate bacteria. Specifically, the invention provides the delivery of one or more toxic proteins, antisense RNAs, multi-ribozymes, or nucleic acids encoding the same, or a combination thereof, to a cell, tissue, or subject containing an infectious bacteria or pathogen in order to eradicate such bacteria or pathogen.

The present invention further encompasses the use of the toxic agents and/or ribozymes of the present invention as therapeutics and pharmaceutical compositions. In specific embodiments of the invention, the toxic agents of the invention are useful to treat microbial infections associated with severe burns, cystic fibrosis, cancer, or other immunocompromising conditions.

The present invention further encompasses the use of the toxic agents and/or ribozymes of the present invention for research and screening purposes. In one embodiment of the present invention, the ribozymes and/or toxic agents may be used to screen for viral, microbial, prokaryotic, or eukaryotic gene products or molecules to be targeted in order to effectively inhibit the selected virus or microbial agent or selected cell.

### 5.1. PATHOGEN-SPECIFIC AND TISSUE-SPECIFIC TOXIC AGENTS

The invention provides specific nucleic acids which act as or encode toxic agents and are therefore useful as antimicrobial agents. A variety of toxic agents are within the scope of the invention. For clarity, the toxic agents of the invention are described herein below in several sub-types. The toxic agents of the invention include but are not limited to antisense nucleic acids, toxic gene products, sense nucleic acids.

### 5.1.1. TOXIC GENE PRODUCTS

The present invention relates to the use of toxic gene products or toxic proteins as toxic agents for the treatment of disorders and disease related to bacterial, parasitic, fungal, or viral infections or to cellular proliferation, and cancers, or to diseased cells. A toxic gene product of the invention is any gene product (such as DNA, RNA or protein), which is toxic to a pathogen or selected cell (such as a diseased cell). Such toxic gene products may be naturally occurring (endogenous), or may be non-naturally occurring (exogenous) in the target pathogen or selected cell. A toxic agent of the invention may be a chromosomally encoded, plasmid encoded, pathogen encoded, synthetic, or encoded in any other nucleic acid or nucleotide sequence. The present invention provides toxic agents which are endogenous toxic gene products that are expressed in a pathogen or selected cell which kill or render the pathogen or selected cell less fit. The present invention also provides toxic agents which are exogenous toxic gene products that are introduced into or expressed in a pathogen or selected cell which kill or render the pathogen or selected cell less fit. A pathogen or selected cell which is less fit is one which is weakened, or which is more susceptible to chemical treatment (such as drugs, toxins, phacmaceuticals, mutagens, solvents, etc.), or which is more susceptible to physical stress (such as temperature), or which is more susceptible to genetic alterations (such as by radiation or UV), or is more susceptible to environmental changes (such as available nutrients).

In several embodiments, the present invention provides the use of a plasmid addiction system protein as a toxic agent when expressed in bacteria or a selected cell. For example, in certain types of bacteriophage, the lysogenic (dormant) pathway is manifested by a bacterial cell maintaining only a single copy of the bacteriophage DNA in the form of a plasmid. In order to assure that both daughter bacterial cells receive a copy of the plasmid, a "plasmid addiction system" or ''post-segregation system" is used by the cells which ensures that only bacterial cells which receive a copy of the plasmid will survive.

In one embodiment of the invention, a post-segregation system or plasmid addiction system toxin, is used as a toxic agent to a pathogen (such as bacteria) by overexpression of the toxin. Such overexpression of the toxin uncouples the toxin and the antidote, leading to toxicity, and preferably lethality, in the cell containing the overexpressed toxic agent

For example, in one embodiment, the invention provides toxic agents which specifically target gene products essential for the survival or life cycle of a pathogen (such as replication, packaging etc). In one embodiment, the present invention provides naturally occurring addiction system toxins which have been modified to be expressed in the absence of the addiction system antidote. In another embodiment, the present invention provides naturally occuning addiction system toxins which have been modified to be expressed at higher levels than the addiction system antidote. In one example, an addiction system toxin *(e.g., .doc, chpBK, kicb, or gef)* is used as a toxic agent and is uncoupled from its antidote. In another embodiment of the invention, a chromosomally encoded toxic gene product (such as *chpBK, kicB,* or *gef)* is used as a toxic agent to a pathogen by overexpression of the toxic gene product

In certain embodiments, toxic agents include but are not limited to Shiga-like toxins of *E. coli,* cholera toxin of *Vibrio cholerae,* and cytotoxins of P. *aeruginosa.* For example, phage K139 confers to *V. cholera* a gene product that enhances enzymatic activity of cholera toxin. Such toxins are within the scope of the invention and may be used as a toxic agent in association with the methods and compositions of the invention.. In certain embodiments of the invention, the bacariocidat toxic agent is derived from a bacterium including but not limited to *Staphylococcus aureus, Enterococcus faecalis* or *Pseudomonas aeruginosa.*

In another embodiment, the antidote of a toxin is the target of a trans-acting ribozyme or toxic agent of the invention. Thus, when the antidote is inactivated by the trans-acting ribozyme or toxic agent, the toxin is no longer neutralized or inactivated by the antidote, thus leading to toxicity, and preferably lethality to the pathogen.

In yet another embodiment, when the antidote is itself an antisense RNA, a sense RNA may be synthesized as a toxic agent and delivered to inactivate the antisense antidote. Thus, when the antidote is inactivated by the sense RNA, the antidote is no longer available to inactivate the toxin, thus leading to toxicity, and preferably lethality.

One example of an addiction system toxin that may be used in connection with the invention is *doc* (death on curing; Lehnherr H, et al., 1993, J. Mol. Biol. 233:414-28). The protein encoded by *doc* is lethal or toxic in both Gram-negative and Gram-positive organisms *(e.g., E. coli, P. aeruginosa, Staphylococcus aureus,* and *Enterococcus faecalis). doc* acts as a bacterial cell toxin to which *Phd* (prevention of host death) is the antidote. Accordingly, the invention provides for plasmids expressing doc which can be delivered to a bacterial pathogen in order to render the pathogen less fit, and preferably eradicate the pathogen. A particular advantage of *doc* is that *doc* has little to no toxicity to eukaryotic cells, and thus may be administered safely to a eukaryotic host.

Specific examples of addiction system toxins or chromosomally encoded toxins, or other toxic agents which may be used in connection with the invention include but are not limited to *ccdB, kid, perK, parE, doc, higB, chpAK, chpBK, kicB, hoc, srnB'*, *flmA, pmdA, relF, gef, kilA, kilB, kilC, kilE, traL, traE, sigB, hok, pemK, lysostaphin, and kikA.* Examples of antidotes which may be used as in the methods of the invention include but are not limited to *ccdA, kis, pemI, parD, phd, higA, chpAI, chpBI, kicA, soc, srnC, flrnB, pndB, sof, korA, korB, korC, korD, korE, and korF.* Thus, the invention herein provides a method of using a an addiction system toxin (such as *doc)* or other toxic protein, as a toxic agent of the invention. The invention also provides methods for inhibiting or inactivating antidotes of a toxin. The invention further provide co-expression of a toxin and its corresponding antidote for manufacturing purposes.

In certain specific embodiments, the invention provides toxic agents *chpBK. kicB,* and *gef.* Each of the proteins of kicB, or *gef* are lethal in *E*. *coli but not in P. aeruginosa.* Accordingly, the invention provides for the use of *kicB* or *gef* in the eradication or treatment of bacterial infections of *E*. *coli.* In one embodiment, *kicB* or gef encoding nucleic acids are delivered by a to the *E*. *coli* by a P1 bacteriophage of the invention containing a transfer plasmid, said transfer plasmid encoding the *kicB* or *gef* toxic agents (or both).

In another specific embodiment of the invention, the *chpBK*protein is a toxic agent of the invention and is lethal to *E. coli* and toxic or lethal in *P. aeruginosa.* Accordingly, the invention provides for the use of *chpBK* in the eradication or treatment of infections *of E. coli or P. aeruginosa.* In one embodiment, *chpBK* nucleic acids are delivered by a to *E. coli* or *P*. *aeruginosa* by a P1 bacteriophage of the invention, containing a transfer plasmid, said transfer plasmid encoding the *chpBK* toxic agent The antidote protein that antagonizes *chpBK* function is called *ChpBl.* Accordingly, the invention provides, for the co-expression of the antidote *ChpB1* and *chpBK* for manufacturing purposes.

In several embodiments of the invention, the toxic gene, such as *doc, chpB*K, *kicB,* or *gef,* is placed under the control of an inducible promoter and is uncoupled from the antidote. In one embodiment, the promoter is the P1 lytic promoter P53. In a preferred embodiment, the promoter is the LBASHI promoter. In a preferred embodiment, for the treatment of *P. aeruginosa* infections, the invention provides *P. aeruginosa* specific promoters, *anr, arc orproC.*

In other specific embodiments of the invention, a consensus ribosome binding site (GGAGGTGXXXXATG, wherein X is any nucleotide) may be inserted immediately upstream of the nucleic acids encoding the toxic agent and leads to increased expression of the toxic agent. The provides for the use of a combinafion of a promoter and a ribosome entry site(s) to modulate expression of a toxic agent or ribozyme.

It is also within the scope of the invention that more that one toxic agent may be used to eradicate or treat an infection. For example, it is contemplated that two or more toxic agents may be engineered into a single transfer plasmid for delivery by a bacteriophage. Such bacteriophage could serve to deliver nucleic acids encoding multiple toxic agents to target bacteria. Alternatively, two or more transfer plasmids may be carried by a single bacteriophage, wherein each transfer plasmid encodes different toxic agents. In this embodiment, when more than one transfer plasmid is used, such plasmids are designed such that the two or more plasmids are non-recombinigenic. Such methods of engineering non-recombinigenic sequences are known in the art. Additionally, in this embodiment, the two or more engineered plasmids will preferably have different origins of replication. In this manner, the bacteriophage serves to deliver nucleic acids encoding multiple toxic agents. In yet a third alternative, bacteriophage may be designed to carry multiple toxic agents on multiple transfer plasmids. When two or more toxic agents are encoded within a single bacteriophage, the nucleic acids encoding such toxic agents may be operably linked to the same promoter, or different promoters (*e.g*., see sections 5.4 and 5.4.1 herein).

### 5.1.2. ANTISENSE

The invention provides specific nucleic acids which act as toxic agents and are therefore useful as antimicrobial agents. The invention provides antisense RNA molecules which target an RNA of a pathogen or selected cell. Target RNAs of the invention may be pathogen-specific RNAs, tissue-specific cellular RNAs, or disease-specific RNAs. The invention also provides modified and enhanced antisense nucleic acids which target pathogen-specific RNAs, tissue-specific cellular RNAs, or disease-specific RNAs.

The proposed target of the toxic antisense molecule of the invention is the RNA of a gene which plays a critical role in the survival of the pathogen, or which is essential to the pathogen's life cycle. The present invention also encompasses modifications to naturally occurring antisense molecules which modulate the expression of an essential gene product of a pathogen. For example, as described below, one proposed target of an antisense of the invention is the *ftsZ* gene whose gene product plays a critical role in the initiation of cell division of *E. coli.*

In another embodiment, the toxic agents of the invention comprise antisense molecules designed to have enhanced inhibition of target RNAs. The toxic agents which comprise antisense molecules of the invention are engineered to more specifically bind target RNAs in that the sequences of such toxic antisense molecules are designed to have increased complementarity to a target sequence such as an essential RNA of a pathogen or selected cell. Such toxic antisense molecules are therefore more specific to their targets and hence, have increased efficacy. The invention provides antisense toxic agents and ribozymes which are also modified with a hairpin structure to create a more stable molecule. The antisense toxic agents of the invention may also be expressed to a high level in a target pathogen or cell by any method known or cell by any method known in the art. For example, an antisense toxic agent may be expressed *in trans* from a multi-copy expression plasmid using a strong regulatable promoter. The antisense toxic agent may also be operably linked to a tissue-specific or pathogen-specific promoter such that the antisense molecule is only expressed in a pathogen or cell which uses the same promoter.

Specifically, the invention provides antisense RNAs which target essential nucleotide sequences, such as *DicF1* or a *DicF1-*like antisense molecule that specifically target a nucleotide sequence which encodes a protein essential for replication or survival. Further, the invention provides modified antisense structures with increased stability to act as lethal agents when expressed in bacteria. The invention also provides toxic sense molecules designed to target essential antisense molecules.

In another embodiment of the invention the toxic agents comprise sense RNA molecules targeted to antisense RNAs which are required for the survival of the pathogen or cell. For example, an antidote of a toxic protein (such as an addiction system toxin) maybe in the form of an antisense molecule which regulates the expression of the toxin. Such an antisense antidote allows the pathogen or cell to survive in the presence of such toxin. The invention provides inhibition of the antisense antidote by a toxic agent in the form of a sense RNA molecule.

In certain embodiments a combination of two or more toxic molecules may be delivered to a pathogen (such as *E.* coli, *P. aeruginosa. etc.)* in order to cause lethality. In this embodiment, the toxic antisense may be directed to the same target_{;} or different targets. When different targets of a pathogen or cell are targeted, such targets may be involved in the same biological pathway within the pathogen or different biological pathways.

In a specific embodiment, the antisense sequence is based on *DicF* (Bouche F, et al., 1989, Mol Microbiol. 3:991-4). Such modified *DicF* sequence is referred to as *DicF1* (SEQ ID NO: 8). Naturally occurring *DicF* is part of an intercistronic region that when expressed in *Escherichia coli* causes inhibition of cell division. This inhibition does not require the translation of *DicF* mRNA into protein, instead, *DicF* RNA exerts its inhibitory effect as an antisense molecule.

The proposed target of DicF is the *ftsZ* gene whose gene product plays a critical role in the initiation of cell division of *E. coli.* Temperature sensitive mutations of the *ftsZ* gene indicate that it is essential for viability of *E. coli.* Without limitation as to mechanism, *DicF* RNA is believed to bind specifically to the 5' untranslated region of *ftsZ* mRNA, thereby inhibiting *ftsZ* protein expression. Cells lacking the *ftsZ* protein are unable to divide and ultimately die. *DicF* homologs have been identified in a variety of other bacteria although it is not known whether they exert a similar function.

The present invention provides for modified *DicF* nucleic acids, called *DicF1 or DicF1-*like RNAs, which are used as antimicrobial agents, or toxic agents of the invention. *DicF*1 RNA is a superior antisense molecule as compared to the endogenous *DicF* RNA*.* It has been modified by increasing its complementarity to the *ftsZ* 5' untranslated mRNA It is therefore more specific to its target and hence, has increased efficacy. An auto hairpin structure has further been enhanced to create a more stable molecule. The invention also provides modifications of other naturally occurring antisense molecules, such as nucleotide sequences which have similar functions as *DicF* in modulating the expression of gene products essential to the pathogen's life cycle or survival. Such nucleic acid is referred to as *a DicF1*-like nucleic acid. In contrast to the endogenous *DicF,* the *DicF1 or a DicF1-like* nucleic acid of the invention may be expressed *in trans* from a multi-copy expression plasmid. Further, the *DicF1* or *DicF1*-like nucleic acids may be operably linked to a variety of promoters that may be used to control the strength, timing, or distribution of such expression. *DicF1* or a *DicF1*-like nucleic acid may also be expressed *in trans* from a riboxyme cassette. The combination of these features results in *DicF1* or *DicF1*-like nucleic acid being an effective antimicrobial agent against a pathogen (such as *E.coli.* In other embodiments, modifications to the sequence of an antisense of the invention allows targeting against a variety of other bacteria. In other embodiments, modifications to the sequence of an antisense of the invention allows targeting in a pathogen-specific manner. The invention also provides DicF1-like nucleic acids which may be used as toxic agents in bacteria, bacteria-infected cells, or other pathogens which have complementary RNA targets.

### 5.1.3. ANTISENSE OLIGONUCLEOTIDES

Antisense oligonucleotides that hybridize or anneal under physiological conditions to at least a portion of a target sequence are also provided for use in the compositions and methods of the invention. Such oligonucleotides are typically short in length (*e.g*. any number of contiguous nucleotides or analogues thereof from about six to about fifty) and can be delivered to cells by any methods known in the art *(see e.g.,* exemplary methods of administration set forth in section 5.9 below). Such antisense oligonucleotides include, but are not limited to, polydeoxynucleotides containing 2'-deoxy-D-ribose, polyribonucleotides containing D-ribose, any other type of polynucleotide which is an N-glycoside of a purine or pyrimidine base, or other polymers containing non-nucleotide backbones *(e.g.,* peptide nucleic acids or PNA, see below, and any other synthetic sequence-specific nucleic-acid-like polymer which is commercially available) or nonstandard linkages, providing that the polymers contain nucleotides in a configuration which allows for base pairing and base stacking such as is found in DNA and RNA. They may include double- and single-stranded DNA, as well as double- and single-stranded RNA and DNA:RNA hybrids, as well as all known types of modifications, for example, labels, "caps", methylation, substitation of one or more natural nucleotides with one or more analogues. Additional known modifications include internucleotide modifications such as, for example, various uncharged linkages *(e.g..* methyl phosphonates, phosphorotriesters, phosphoramidates, carbamates, *etc.),* charged linkages or sulfur-containing linkages (*e.g.,* phosphorothioates, phosphorodithioates, *etc.),* pendant moieties (such as, for example, on proteins including nucleases, nuclease inhibitors, toxins, antibodies, signal peptides, poly-L-lysine, etc.) and saccharides (*e.g.*, monosaccharides, *etc.*), intercalators (*e.g.,* acridine, psoralen, etc.), chelators (*e.g.,* metals, radioactive metals, boron, oxidative metals, etc.), alkylating agents, and modified linkages (*e.g.*, alpha anomeric nucleic acids, *etc.).*

Peptide nucleic acid (PNA) is just one example of a uncleic acid derivative or analogue well known in the art which may be used in the compositions and methods of the present invention. PNA was first described in 1991 by Nielsen *et al.* (1991, Science 254, 1497-1500) and has been described as a nucleic acid mimetic with a neutral peptide-like backbone instead of a negatively-charged sugar-phosphate backbone. However, the same nitrogenous bases (i.e. adenine, guanine, cytosine and thymine) are used in PNA as found in DNA and RNA, and PNA undergoes Watson-Crick base pairing with DNA and RNA. PNA is not generally recognized as a substrate for DNA polymerases, nucleic acid binding proteins, or other enzymes, including proteases and nucleascs, although some exceptions exist *(see e.g.* Lutz *et al.*,1997*,* J. Am. Chem. Soc.119, 3177-3178). The chemical structure of PNA consists of repeating units of N-(2-aminoethyl)-glycine linked by amide bonds. The nitrogenous bases are attached to this neutral backbone by methylene carbonyl linkages. Unlike the natural nucleic acid backbone, no deoxyribose, ribose or phosphate groups are present. As a result, PNA binding to a target nucleic acid is stronger than with conventional nucleic acids, and the binding is virtually independent of salt concentration. Quantitatively, this is reflected by a high thermal stability of duplexes containing PNA.

PNA may be synthesized by methods well known in the art using chemistries similar to those used for synthesis of nucleic acids and peptides. The PNA monomers used in such syntheses are hybrids of nucleosides and amino acids. The neutral backbone of a PNA oligomer results in several unique properties. Its properties relative to natural nucleic acids include: (a) higher affinity; (b) faster hybridization; and (c) relative independence of hybridization from salt concentration. In general, a given PNA duplexed with a natural nucleic acid having one or more mismatches will result in a greater change in melting temperature (*i.e.* ΔTₘ). PNA products, services, and technical support are commercially available from PerSeptive Biosystems, Inc., a division of Applied Biosystems (www.pbio.com). For example, PNA may be synthesized using kits or custom PNA synthesis may be ordered. PNA oligomers may also be manually synthesized using either Fmoc or *t*-Boc based monomers and standard peptide chemistry protocols. Standard peptide purification conditions may be used to purify PNA following synthesis.

A variety of extensive PNA reviews have been published, each of which is incorporated by reference herein *(see e.g.,* Nielsen *et al.,* 1992, In *Antisense Research and Applications,* Crooke and Lebleu, eds., CRC Press, pp. 363-372; Nielsen et *al.,* 1993, Anti-Cancer Drug Design 8, 53-63; Buchardt et al, 1993, TIB TECH 11, 384-386; Nielsen *et al.,* 1994, Bioconjugate Chem. 5, 3-7; Nielsen *et al.,* 1996, In *Antisenre Therapeatics* Vol. 4 (ed. Trainor, ed., SECOM Science Publishers B.V., Leiden, pp. 76-84; Nielsen, 1995, Ann. Rev. Biophys. Biomol. Struct. 24,167-183; Hyrup and Nielsen, 1996, Bioorg. Med. Chem. 4, 5-23; Mesmaeker *et al.,* 1995, Curr. Opin. Struct. Biol. 5, 343-355; Dueholm and Nielsen, 1997, New J. Chem. 21, 19-31; Knudsen and Nielsen, 1997, Anti-Cancer Drug 8, 113-118; Nielsen, 1997, Chem. Bur. J. 3, 505-508; Corey, 1997, TIB TECH 15, 224-229; Nelsen and Orum, 1995, In *Molecular Biology: Current Innovations and Future Trends,* Part 2, Horizon Scientific Press, pp. 73-89; Nielsen and Haaima, 1997, Chem. Soc. Rev., 73-78; Ørum *et al.,* 1997, In *Nucleic Acid Amplification Technologies: Application to Disease Diagnostics,* Lee *et al.,* eds., BioTecbniques Books Div., Eaton Publishing, pp. 29-48).

### 5.1.4. DNAZYMES

DNAzymes have been described in various publications, including U.S. Patent 6,159,714 to Usman *et al.,* International Publication WO 00/09673 to Sun *et al.* and U.S. Patent 5,807,718 to Joyce *et al.,* all of which are hereby incorporated by reference in their entirety. As the term is used in this application, DNAzymes (also known in the art as catalytic DNA enzymes, or Deoxyribozymes) are chimeric DNA, or non-RNA-containmg DNA molecules having an enzymatic activity which is able to cleave (preferably, cleave repeatedly) separate target RNA molecules in a nucleotide base sequence specific manner.

DNAzymes act by first binding to a target RNA. Such binding occurs through and with the DNA-binding portion of a DNAzyme, which is held in close proximity to the RNA substrate. The catalytic sequences of the DNAzyme then cleave the target RNA. Thus, the DNAzyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a DNAzyme has bound and cleaved its RNA target, the DNAzyme is released from that RNA and is free to complex with another target. Thus, a single DNAzyme molecule can bind and cleave new targets repeatedly.

Generally then, a DNAzyme is a DNA molecule that has complementarity in a substrate-binding region to a specified gene target sequence, and also is able to cause specific cleavage of the RNA target sequence. That is, the DNAzyme molecule is able to intermolecularly cleave RNA and thereby inactivate a target RNA molecule. The complementarity sequences of a given DNAzyme function to allow sufficient hybridization of the DNAzyme to its target RNA to allow the cleavage to occur. One hundred percent complementarity is preferred, but complementarity as low as 50-75% may also be useful in this invention. In particular, mismatches may be tolerated within the target recognition site that are not adjacent to the cut region.

The catalytic core of a DNAzyme may be selected from any of a number of possible sequences that have enzymatic activity against a RNA substrate. In one embodiment, the catalytic core sequence is 5'-GGCTAGCTACAACGA-3' (SEQ ID NO ). Other catalytic core sequences are disclosed, for example, in U.S. Patent 5,807,718 to Joyce *et al.*

### 5.1.5. ANTISENSE OLIGONUCLEOTIDES, DNAZYMES, AND ANALOGUES MODIFIED AT THEIR 3' AND/OR 5' TERMINI

Antisense oligonucleotides modified at their 3' and/or 5' ends have been described, for example in U.S. Patent 5,750,669 to Rosch *et al.,* which is hereby incorporated by reference in its entirety. The characteristic structural modification of these oligonucleotides is that the *internucleotide* linkage at the 3' end is altered, that is to say, a 3'-3' linkage exists in place of the biological 3'-5' linkage. This minimal structural modification suffices to stabilize such compounds against nuclease degradation. Inverted bases such as thymidine, cystosine, guanosine or adenine may be used, although other bases are possible. This slight structural modification results in a hybridization behavior which is almost identical to that of unmodified oligonucleotides. This modification also results in these compounds being generally utilizable as inhibitors of gene expression. The DNAzymes of the present invention may also have the above- disclosed modifications at their 3' ends.

### 5.1.6. NUCLEIC ACID HOMOLOGY

Homology or identity at the nucleotide or amino acid sequence level may be determined by any method known to the skilled artisan including BLAST (Basic Local Alignment Search Tool) analysis using the algorithm employed by the programs blastp, blastn, blastx, tblastn and tblastx (Karlin *et at.,* 1990, Proc. Natl. Acad. Sci. USA 87, 2264-2268 and Altschul, 1993, J. Mol. EvoL 36,290-300, fully incorporated by reference) which are tailored for sequence similarity searching. The approach used by the BLAST program is to first consider similar segments between a query sequence and a database sequence, then to evaluate the statistical significance of all matches that are identified, and finally to summarize only those matches which satisfy a preselected threshold of significance. For a discussion of basic issues in similarity searching of sequence databases, see Altschul *et al.,* 1994, Nature Genetics 6,119-129) which is fully incorporated by reference. For purposes of the present application, search parameters for histogram, descriptions, alignments, expect (*i.e.,* the statistical significance threshold for reporting matches against database sequences), cutoff, matrix and filter may be used at the default settings. The default scoring matrix used by blastp, blastx, tblastn, and tblastax is the BLOSUM62 matrix (Henikoff et *al.,1992,* Proc. Natl. Acad. Sci. USA 89,10915-10919, fully incorporated by reference). For blastn, the scoring matrix is set by the ratios of M (*i.e*., the reward score for a pair of matching residues) to N (*i.e.,* the penalty score for mismatching residues), wherein the default values for M and N are 5 and -4, respectively. Four blastn parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent Blastp parameter settings were Q=9; R=2; wink=1; and gapw=32. A Bestfit comparison between sequences, available in the GCG package version 10.0, uses DNA parameters GAP=50 (gap creation penalty) and LEN=3 (gap extension penalty) and the equivalent settings in protein comparisons are GAP=8 and LEN=2. A homologous sequence in a related HPV or HBV strain is a sequence that is at least about 70% identical to a target site of the instant disclosure. A homologous sequence in a related HPV or HBV strain is, more preferably, at least about 75% or 80% or 85% or 86% or 87% or 88% or 89% or 90% or 91% or 92% or 93% or 94% or 95% or 96% or 97% or 98% or 99% identical to a target site identified in the instant disclosure. For example, the target sequences identified as SEQ ID NO:AH and SEQ ID NO:AM are homologous sequences in related HPV strains (HPV16 and HPV11, respectively). Consequently, target sites in related strains ofHPV or HBV are identifiable based on their homology. Homologous sites may also be identified by aligning the protein sequences of related viruses and then aligning the underlying nucleic acid sequences to find homologous or corresponding target sites.

### 5.1.7. RIBOZYMES

The present invention provides methods by which a trans-acting ribozyme may be used in addition to the toxic agents of the invention. Further, a multi-ribozyme may be used as an expression system for one or more toxic agents or trans-acting ribozymes. These ribozymes of the invention can be used, for example, to destroy tissue-specific disease, or to treat bacterial, viral, or parasitic infections. The ribozymes of the present invention may comprise one or more multi-ribozymes.

In accordance with the present invention, the multi-ribozyme may comprise one or more ribozymes or one or more nbozyme cassettes. Bach cassette in turn may consist of a catalytic core (*e.g*., containing one or more trans-acting ribozymes or containing one or more toxic agents) and one or more flanking regions. The catalytic core can target a pathogen, by specifically inhibiting a pathogen-specific target. The catalytic core can target a cell (such as a diseased cell), by specifically inhibiting a tissue-specific target (such as disease-specific target). Further, as described in sections below, the multi-ribozymes of the invention also provide a means of delivering toxic agents to a cell, and expressing toxic agents of the invention (including antisense RNA, toxic gene products) in a cell or tissue-specific, or pathogen-specific manner. In one embodiment, the n'bozyme cassette may consist of a 5' autocatalytically cleaving ribozyme sequence, a core catalytic ribozyme comprising a trans-acting ribozyme and a 3' autocatalytically cleaving ribozyme. In another embodiment, the multi-ribozymes comprise a cassette including, the enhanced 5' and 3' autocatalytically cleaving ribozyme sequence. In another embodiment, the multi-n'bozymes contain one or more internal trans-acting ribozymes. Such trans-acting ribozymes may be directed to the same site on the same RNA, different sites on the same RNA, or different RNAs. Thus, trans-acting ribozymes of the invention may target a pathogen-specific RNA or tissue-specific RNA.

The present invention also provides multi-ribozymes and their use to target RNA in a tissue-specific or pathogen-specific manner for the treatment of disease such as bacterial infection. The invention provides multi-ribozymes containing one or more internal trans-acting ribozyme. Trans-acting ribozymes act in a target-specific manner and therefore may, in certain embodiments, act on a pathogen (such as bacteria) or a selected cell (such as a diseased cell) to enhance the use of toxic agent. In accordance with the present invention, the multi-ribozymes may comprise a) a trans-acting ribozyme or toxic agent flanked by 5' and 3' autocatalytically cleaving ribozymes or enhanced autocatalytically cleaving ribozymes; b) a trans-acting ribozyme or toxic agent flanked by either a 5' or 3' autocatalytically cleaving ribozyme; or c) multi-transacting ribozymes and/or multiple toxic agents, flanked by one or both 5' and 3' autocatalytically cleaving ribozymes or enhanced autocatalytically cleaving ribozymes. For example, in a specific embodiment, the invention provides a multi-ribozyme with two trans-acting ribozymes, wherein the first trans-acting ribozyme cleaves an HBV target, and the second trans-acting ribozyme cleaves a HCV target. In this embodiment, it may also be desirable to target the expression of such multi-ribozyme to the liver, *e.g.,* by operative association with a liver-specific promoter. Thus, the multi-ribozymes of the invention may be used to deliver one or more toxic agents to a bacteria or bacteria-infected cell or tissue, In accordance with the present invention the multi-transacting ribozymes may be targeted to the same site on the same RNA, different sites on the same RNA or different RNAs. In accordance with the present invention the multiple toxic agents may be targeted to the same site on the same target (such as a cellular RNA or protein), different sites on the same target or different targets.

The ribozymes of the present invention possesses sufficient catalytic activity to inactivate the RNA of the targeted RNAs. From an antimicrobial perspective, hammerhead-type ribozymes are especially attractive since the molecule inactivates gene expression catalytically through the cleavage of the phosphodiester bond of the mRNA. Furthermore, hammerhead-type ribozymes have been re-engineered to function in an intermolecular or transducer (trans) acting state (Haseloff et al., 1988, Nature 334(6183):585-91; Uhlenbeck. O.C., 1987, Nature 328(6131):59). The catalytic activity of the ribozyme requires a sufficient concentration of the divalent cation, Mg⁺², and substrate. The substrate can have any sequence as long as the cleavages site contains the recognition element NUX, where N represents any nucleotide, U corresponds to uracil, and X is any nucleotide except G (Koizumi et aL, 1989, Nucleic Acids Resonant 17(17):7059-71). Ribozymes have been widely demonstrated to function *in* vivo (Christoffersen et al., 1995, J. Med. Chem. 38(12):2023-37; Inokuchi et al.,1994, J. Biol. Chem. 269(15):11361-6). The present invention improves the initial design of hammerhead-type ribozymes (Taira et al., 1991, NAR 19(9):5125-5130) by constructing multi-ribozymes consisting of ribozyme cassettes. Ribozyme cassettes contain one or more cis-acting hammerhead ribozymes flanking a ribozyme that inactivates the targeted RNA(s) as well as one or more flanking sequences. Upon transcription the targeted ribozyme is released as a 60-70 base transcript which not only improves its specificity by reducing non-specific interactions but also improves its catalytic activity as well. This invention includes modifications to and use of the ribozyme described in U.S. Patent 5,824,519 and PCT publications No.WO98/24925, WO97/17433, WO98/24925, WO99/67400, which are incorporated by reference herein in their entirety.

### 5.2. NUCLEIC ACIDS ENCODING TOXIC AGENTS OR RIBOZYMES

The invention also provides nucleic acids and expression cassettes which encode the ribozymes and/or toxic agents of the invention. These nucleic acids can be used to express the ribozymes or toxic agents of the invention at the selected site. The site can be tissue-specific in the case of treating tissue-specific cancers or disease, or it can be pathogen-specific in the case of ribozymes or toxic agents that prevent replication of infections agents to treat infection (*e.g*., hepatitis, herpes, malaria, tuberculosis, bacterial infections etc.). The invention provides nucleic acids which encode toxic agent(s) and/or ribozyme(s) which are target-specific. The invention also provides nucleic acids which encode toxic agent(s) and/or ribozyme(s) operably linked to a tissue-specific or pathogen-specific promoter.

There are several options for constructing the multi-ribozyme encoding sequences: 1) ribozymes directed to different targets in the same pathogen 2) multiple copies of the same ribozyme 3) multiple ribozymes directed to multiple targets, and 4) multiple ribozymes directed to different sites on the same target. There are also several options for constructing the toxic agent encoding sequences: 1) toxic agents directed to different targets in the same pathogen 2) multiple copies of the same toxic agent 3) multiple toxic agents directed to multiple targets, and multiple toxic agents directed to the same target. Further, toxic agents and ribozymes may be combined in various ways, *e.g.,* a multi-ribozyme and a nucleic acid encoding a toxic agent may be engineered in a single construct under one promoter. The promoter can have the chosen level of specificity as described herein.

The nucleic acids of the invention encode one or more toxic agents of the invention. Thus, nucleic acids encoding toxic proteins of the invention include but are not limited to addiction system toxins. The invention further provides modified and enhanced addiction system toxins which have been engineered to be more toxic or more specific to a particular target pathogen. The present invention provides nucleic acids encoding antisense molecules targeted to RNA of a gene which plays a critical role in the survival of the pathogen, or which is essential to the pathogen's life cycle. The present invention also encompasses nucleic acids comprising modifications to naturally occurring antisense molecules which modulate the expression of an essential gene product of a pathogen.

The nucleic acids of the invention also relate to those encoding antisense molecules of the invention. The invention provides modified and enhanced antisense molecules which have enhanced stability, enhanced complementarity to a target RNA, or enhanced specificity for a target RNA or target pathogen. The invention also provides nucleic acids encoding modified naturally occurring antisense molecules, such as nucleotide sequences which have similar functions as *DicF* in modulating the expression of gene products essential to the pathogen's life cycle or survival.

The nucleic acids of the invention also relate to nucleic acids encoding sense RNA molecules capable of targeting an essential antisense molecule.

The nucleic acid, encoding a toxic agent selected from the group consisting of *ccdB, kid, perK, parE, doc, higB, chpAK, chpBK, kicB, hoc, srnB', flmA, pmdA, relF, gef, kilA, kilB, kilC, kilE, traL, traE, sigB, hok, pemK, lysostaphin, and kikA is provided.* The nucleic acid encoding the toxic agent *DicF1,* or *DicF1-*like, is provided.

In several embodiments, nucleic acids of the invention encode a catalytic multi-ribozyme(s) that contains two separable functional regions including a) a catalytic sequence (also known as the "catalytic core") which cleaves the target RNA, and b) flanking regions which include cis-acting autocatalytically cleaving ribozyme(s). As described above, the catalytic core consists of one or more trans-acting ribozyme(s) and/or one or more toxic agent(s). The present invention provides nucleic acid which encode an internal targeted ribozyme containing two or more trans-acting ribozymes, wherein each of the separate trans-acting ribozymes can be targeted to the same or different target RNA molecules. By nucleic acid complementarity, the binding site directs the ribozyme core to cleave a specific site on the target RNA molecule. The length of flanking sequences have implications not only for specificity, but also for the cleavage efficiency of the individual ribozyme molecules. In the present catalytic ribozyme, the flanking sequences are highly specific for the target RNA, yet allow ready dissociation from the target RNA once cleavage occurs. This permits cycling of the ribozyme and reduces the amount of ribozyme required to be effective. A range of binding/dissociation values from 16-21 Kcal is expected to be effective. The present invention provides nucleic acid which encode a two or more toxic agents, wherein each of the toxic agents can be targeted to the same or different target molecules.

The invention additionally provides nucleic acids and expression cassettes which encode the toxic agent and/or ribozymes of the invention. These nucleic acids can be used to express the toxic agent and/or ribozyme of the invention at the selected site. In one embodiment, the nucleic acid comprise a tissue-specific promoter operably linked to a toxic agent. In another embodiment, the nucleic acids and expression cassettes of the invention comprise a tissue-specific promoter operably linked to a sequence encoding a catalytic ribozyme comprising one or more target RNA-specific trans-acting ribozymes and one or more toxic agents. In another embodiment, the nucleic acids comprise a pathogen-specific promoter from a sequence encoding a toxic agent. In another embodiment, the nucleic acids and expression cassettes of the invention comprise a pathogen-specific promoter operably linked to a sequence encoding a 5' autocatalytically cleaving ribozyme sequence, a catalytic ribozyme comprising one or more target RNA-specific trans-acting ribozymes and/or pathogen-specific toxic agents, and a 3' autocatalytically cleaving ribozyme sequence. In accordance with the present invention, the expression cassettes maybe engineered to express two or more multi-ribozymes containing trans-acting ribozymes which act on the same or different targets. The expression cassettes may also be engineered to express two or more multi-ribozymes containing 5' and 3' autocatalytically cleaving ribozymes with either slow or enhanced cleavage activity.

The expression cassettes of the invention or the nucleic acids encoding the toxic agents of the invention may be placed into any suitable plasmid known in the art (such as a bacteriophage transfer plasmid, bacterial plasmid, or enkaryotic expression plasmid). The invention also provides novel and modified plasmids for use in accordance with the invention.

At the molecular genetic level the coding sequence for a toxic agent, ribozyme, or multi-ribozyme of the invention may be placed under the control of one or more of the following genetic elements: a naturally occurring strong, intermediate, or weak constitutively expressed or regulated promoter from the targeted microorganism, or an artificially contrived constitutively expressed or regulated promoter containing either a strong, intermediate or weak consensus sequence that accords the desired levels of ribozyme and/or toxic agent expression. The present invention provides promoter elements which are pathogen-specific. The invention provides promoter elements which are used to achieve pathogen-specific expression of the toxic agents of the present invention. The present invention provides promoter elements which are tissue-specific. The invention provides promoter elements which are used to achieve tissue-specific expression of the toxic agents of the present invention. Accordingly, the present invention provides nucleic acids encoding promoter elements which are pathogen-specific. The invention provides promoter elements which are used to achieve pathogen-specific expression of the toxic agent(s) and/or ribozyme(s) of the present invention. The present invention provides promoter elements which are tissue-specific. The invention provides promoter elements which are used to achieve tissue-specific expression of the toxic agent(s) and/or ribozyme(s) of the present invention.

In one embodiment, the nucleic acid comprise a tissue-specific promoter operably linked to a sequence encoding one or more toxic agent(s). In another embodiment, the nucleic acids comprise a tissue-specinc or pathogen-specific promoter operably linked to a sequence encoding at least one autocatalytic ribozyme and one or more tans-acting ribozymes. In another embodiment, the nucleic acids comprise a tissue-specific or pathogen-specific promoter operably linked to a sequence encoding at least one or more toxic agents. In another embodiment, the nucleic acids comprise a pathogen-specific promoter operably linked to a sequence encoding at least one autocatalytic ribozyme and one or more trans-acting ribozymes and one or more toxic agents. In accordance with the present invention, the trans-acting ribozymes and/or toxic agents of the invention may act on the same or different targets.

In yet another embodiment, the present invention provides a novel vector or plasmids encoding the toxic agent(s) and/or ribozyme(s) of the invention. The novel vectors of the present invention may be used to engineer a wide variety of toxic agents and/or ribozymes including, but not limited to, tissue-specific, pathogen-specific, promoter-specific, antimicrobial specific, antiviral specific, anticancer specific, antitumor specific, or target-specific. The invention also relates to a vector or plasmid origin of replication which modulates specificity of the replication of a vector or plasmid in a cell or pathogen. The invention also relates to the copy number of a vector or plasmid in a selected cell or pathogen to modulate the dose of the toxic agent and/or ribozyme.

In a specific embodiment, the invention provides novel plasmids which encode a toxic protein. In another specific embodiment, the invention provides novel plasmids which encode a mutant bacteriophage *pac* site or a mutant bacteriophage pacABC sequence.

### 5.2.1. EUCARYOTIC AND PROCARYOTIC EXPRESSION VECTORS

The present invention encompasses expression systems, both eucaryotic and procaryotic expression vectors, which may be used to express the toxic agents and/or multi-ribozymes of the invention. The DNA expression vectors and viral vectors containing the nucleic acids encoding the toxic agents of the present invention may be produced by recombinant DNA technology using techniques well known in the art. Thus, methods for preparing the expression vectors and viral vectors of the invention by expressing nucleic acid encoding a toxic agent and/or multi-ribozyme sequences are described herein. Methods which are well known to those skilled in the art can be used to construct expression vectors containing gene product coding sequences and appropriate transcriptional and translational control signals. These methods include, for example, in *vitro* recombinant DNA techniques, synthetic techniques, and *in vivo* genetic recombination. See, for example, the techniques described in Sambrook et al., 1989, *supra,* and Ausubel et al., 1989, *supra.* Alternatively, nucleic acids capable of encoding a toxic agent and/or ribozyme sequence may be chemically synthesized using, for example, synthesizers. See, for example, the techniques described in "Oligonucleotide Synthesis", 1984, Gait MJ. ed., IRL Press, Oxford, which is incorporated by reference herein in its entirety.

A variety of host expression vector systems maybe utilized to express the selected toxic agent and/or multi-ribozyme of the invention. Such host-expression systems represent vehicles by which the sequences encoding the toxic agents or nbozymes of the invention may be introduced into cells, tissues, or pathogens both *in vivo* and *in vitro* but also represent cells which may, when transformed or transfected with the appropriate nucleotide coding sequences, to express a toxic agent and/or ribozymes of the invention. These include but are not limited to microorganisms such as bacteria transformed with recombinant bacteriophage DNA, plasmid DNA or cosmid DNA expression vectors containing selected toxic agent(s) and/or multi n'bozyme coding sequences; yeast (*e.g*., Saccharomyces, Fichia) transformed with recombinant yeast expression vectors containing the selected toxic agent(s) and/or multi-ribozyme coding sequences; insect cell systems infected with recombinant virus expression vectors (*e.g*., baculovirus) containing the selected toxic agent(s) and/or multi-ribozyme coding sequences; plant cell systems infected with recombinant virus expression vectors (*e.g.*, cauliflower mosaic virus, CaMV; tobacco mosaic virus, TMV) or transformed with recombinant plasmid expression vectors *(e.g.,* Ti plasmid) containing selected toxic agent(s) and/or multi-ribozyme coding sequences; or mammalian cell systems (*e*.*g*., COS, CHO, BHK, 293, 3T3) harboring recombinant expression constructs containing promoters derived from the genome of mainmalian cells (*e*.*g*., metallothionein promoter) or from mammalian viruses (*e.g.,* the adenovirus late promoter, the vaccinia virus 7.5K promoter).

### 5.3. DELIVERY AND EXPRESSION OF TOXIC AGENTS

The invention also provides a novel vehicle for the delivery of toxic agents or ribozymes of the invention. The invention encompasses DNA expression vectors and viral vectors that contain any of the foregoing coding sequences operatively associated with a regulatory element that directs expression of the coding sequences and genetically engineered host cells that contain any of the foregoing coding sequences operatively associated with a regulatory element that directs the expression of the coding sequences or RNAs in the host cell or pathogen. A key to the present invention is the strategy used to deliver the toxic agent and/or ribozyme to the targeted microorganism or pathogen. Two separate classes of delivery systems can be manufactured, one biologic in nature and the other abiologic.

Accordingly, present invention also provides the delivery of the toxic agents of the invention to cell or pathogen by abiologic or biologic systems. The present invention provides compositions of matter which has resulted from the development of methods and compositions for the delivery of one or more ribozymes and/or toxic agents directed against fundamental and essential cellular processes specific to a targeted microorganism through an inactivated, altered, or modified virus (virion) or bacteriophage delivery vehicles. The present invention also provides abiologic delivery vehicles, capable of delivering a nucleic acid comprising the toxic agent(s) and/or ribozyme(s) into the targeted microorganism.

### 5.3.1. BIOLOGIC DELIVERY VEHICLES

The biologic delivery vehicle of the invention takes advantage of the fact that generalized transducing particles lack DNA originating from the viral delivery vehicle or have a reduced capacity to transfer DNA originating from the viral delivery vehicle. In a preferred embodiment of the invention, the viral delivery vehicle is a bacteriophage, or modified bacteriophage. In one embodiment, such viral or bacteriophage particles only contain sequences of host origin. In other embodiments, such particles contain engineered plasmids/vectors encoding the toxic agent(s) or ribozyme(s) to be delivered. In other embodiments, such particles contain engineered plasmids/vectors encoding the toxic agent(s) or ribozyme(s) to be delivered and contain mutations which inactivate the ability of the delivery vehicle to transfer DNA originating from the delivery vehicle. Consaquently, the invention uses a biologic assembly of viral head proteins (packaging elements for the antimicrobial therapeutic) around the nucleic acid containing the necessary genetic elements that will insure the desired level of expression of the toxic agent(s) and/or ribozyme(s). An important features of the present invention are the combination of toxic agents or ribozyme with viral delivery and assembly of the virions using a unique combination of plasmid features.

In one preferred embodiment, the invention provides bacteriophage which deliver a toxic agent of the invention. Bacteriophage of the invention may be constructed to deliver one or more toxic agents of the invention, such as one or more toxic gene products, proteins, antisense RNAs, sense RNAs, or combination thereof In another embodiment of the invention, a host cell is constructed to express a pathogen-specific toxic agent or ribozyme. In yet another embodiment of the invention, a host cell is constructed to express a repressor of a promoter used in the invention.

In other embodiments, a host cell maybe engineered to overexpress an antidote to a toxic agent such that the host cell is protected from toxicity and may be used as a producing strain, or manufacturing strain.

The present invention also encompasses expression systems, which maybe used to express the toxic agents and/or ribozymes such as bacteriophage, viral vectors, etc. For example, a variety of bacteriophage systems may be utilized to express the selected ribozyme(s) and/or toxic agent(s) of the invention. For example, such bacteriophage systems represent vehicles by which the sequences encoding the toxic agent(s) and/or ribozyme(s) may be introduced into target bacteria both *in vivo* and *in vitro.* In several embodiments, the specific bacteriophage which is selected determines the species of bacteria which is targeted and infected by that bacteriophage.

In one embodiment, delivery of a toxic agent to a pathogen is by use of a bacteriophage or other delivery vehicle which targets the pathogen of interest, In one embodiment, the bacteriophage (or delivery vehicle) delivers the toxic agent or nucleic acids encoding the toxic agent to the pathogen. In a specific embodiment, a toxic agent of the invention is delivered to a bacterial cell by a modified bacteriophage capable of infecting a pathogenic bacteria. In a further embodiment, bacteriophage are selected for their ability to infect a particular species or genera of bacteria, and are used to deliver a toxic agent for the eradication of such bacterial species or genera from a host. In a preferred embodiment, the delivery vehicle or nucleic acids native to the delivery vehicle are modified such that they contain insufficient genetic information for the delivery of nucleic acids native to the delivery vehicle. Thus, the modified delivery vehicle *(e.g.,* virion or bacteriophage) can serve as a molecular vehicle that delivers the ribozyme(s) and/or toxic agent(s) of the invention to the target cell or pathogen, but does not deliver replicable nucleic acids native to the delivery vehicle. Alternatively, an abiologic delivery system (e.g., liposomes) can be used to package nucleic acid carrying the genetic elements necessary and sufficient for the proper expression of the ribozyme(s) and/or toxic agent(s).

The toxic agents and/or ribozymes of the invention may be used to treat infection from a variety of pathogens. These include but are not limited to microorganisms such as bacteria, parasites, and fungi. In specific embodiments of the invention, the toxic agents of the invention, delivered by a viral delivery vehicle (such as a modified bacteriophage are useful to treat microbial infections associated with severe burns, cystic fibrosis, cancer, or other immunocompromising conditions.

### 5.3.1.1. DELIVERY & EXPRESSION BY VIRAL VECTORS

In accordance with the present invention, a wide variety of viruses and viral vectors may be used to deliver the nucleotide sequences encoding the toxic agents) and/or ribozymes of the present invention, a few examples of which are described below. In this regard, a variety of viruses may be genetically engineered to express the selected toxic agent(s) and/or ribozymes in order to target a specific pathogen.

The present invention also relates to the delivery of the toxic agents of the invention to cell or pathogen by abiologic or biologic systems. In a specific embodiment, For example, as described herein, a toxic agent of the invention is delivered to a bacterial cell by a bacteriophage capable of infecting a pathogenic bacteria. In a further embodiment, bacteriophage are selected for their ability to infect a particular species of bacteria, and are used to deliver a toxic agent for the eradication of such bacterial species from a host

The invention provides for use of a virion which can also be any bacteriophage which specifically infects a bacterial pathogen of the present invention as well as any virus which can be specifically targeted to infect the pathogen of the present invention. For example, the bacteriophage can include, but is not limited to, those specific for bacterial cells of the following genera: *Bacillus, Campylobacter, Corynebacterium, Enterobacter, Enterococcus, Escherichia, Klebsiella, Mycobacterium, Pseudomonas*, *Salmonella, Shigella, Staphylococcus, Streptococcus, Vibrio, Streptomyces, Yersinia* and the like (see, *e.g.,* the American Type Culture Collection Catalogue of Bacteria and Bacteriophages, latest edition, Rockville, MD), as well as any other bacteriophages now known or later identified to specifically infect a bacterial pathogen of this invention. The invention also provides for the use of a virion which specifically infects a fimgal pathogen. This delivery system consists of a DNA plasmid carrying the nucleic acids coding for the toxic agent(s) and/or ribozyme(s) packaged into viral particles. Specificity is conferred by the promoter driving transcription of the toxic agents and/or ribozymes and by the host specificity of the viral vehicle. Specificity is also conferred by the origin of replication controlling vector replication.

In the virions of the present invention, the non-viral DNA can encode one or more toxic agent(s) and/or one or more ribozyme(s). In the virions, the non-viral DNA can comprises a pathogen-specific or tissue-specific promoter operably linked to a sequence encoding one or more toxic agents or ribozymes.

The nucleic acid delivered bya virion can encode one or more toxic agent(s) and/or one or more ribozyme(s) or a combination thereof. The virion can comprise any nucleic acid encoding a ribozyme or toxic agent, particularly those described herein.

### Bacteriophage P1

The invention provides the use of any virion for the delivery of a toxic agent or ribozyme to a target cell. For example, a common bacteriophage of *E. coli,* P1, is an attractive delivery vehicle for the invention for a number of reasons. First and foremost, P1 has a broad intergenera and interspecies range (Yarmolinsky et al., 1988, Mol. Gen. Genet 113:273-284). The P1 receptor of *E. coli* is the terminal glucose of the lipopolysacchuide (LPS) core lysergic ring of the bacterial outer membrane (Generalized Transduction, p. 2421-2441. In F. Neidhardt (ed.), Escherichia coli and Salmonella:Cellular and Molecular Biology, 2d ed. Vol.2, ASM Press, Washington, DC.). Yarmolinsky and Sternberg report that in addition to *E*. *coli,* this particular phage has the ability to inject its nucleic acid into a large number (>25) of diverse Gram negative bacteria (Yarmolinsky et al., 1988, Mol. Gen. Genet. 113:273-284). Secondly, P1 can accommodate a significant amount of genetic information, over 2% (100,000 bp) of the DNA of *E. coli* (Generalized Transduction, p. 2421-2441. In F. Neidhardt (ed), Escherichia coli and Salmonella:Cellular and Molecular Biology, 2d ed. VoL2, ASM Press, Washington, DC.). Consequently, gene dosage of the ribozymes or toxic agents can be increased through multiplication of the toxic agents and/or ribozymes, thereby increasing the microbicidal activity of the toxic agents and/or ribozymes. Accordingly, bacteriophage *PI* is used as the delivery vehicle or molecular syringe. P1 has advantages over certain with lytic phage therapy which may harbor risk of 1) dissemination of undesirable products (*e.g.,* DNA originating from the P1 bacteriophage) to nonpamogenic indigenous microflora, 2) excessively narrow host range, 3) rapid clearance of the material by the reticuloendothelial system of the host and 4) the concern that a lytic infection could become uncontrolled in commensal bacteria in immunocompromised patients. In certain of these embodiments, the *P1* delivery system is the preferable delivery vehicle for delivery of a toxic agent to a target pathogenic bacterium. An additional advantage of the P delivery vehicle is that phage-mediated transfer of undesirable products may be decreased or avoided when the phage are engineered such that they are incapable of transferring endogenous phage DNA to the host. In this embodiment, the phage particles inject transfer plasmid DNA into target bacterial cells. Expression of the encoded toxic agents may then result in bacterial cell death independent of the bacterium's resistance to antibiotics.

Additionally, a process utilizing *in vitro* packaging is also possible. *In vitro* packaging can be accomplished through the addition of PAC-sites to the genetic information of the toxic agent or ribozyme construct. PI packaging initiates within one of the P1 PAC genes (Steinberg, N.,1987, J. Mol. Biol. 194(3) :469-79). It has been reported that the active PAC site is contained within a 161 base-pair segment of the P 1 EcoR1 fragment 20 (Steinberg, N.,1987, J. Mol Biol. 194(3):469-79). Thus, the phage head serves as a molecular syringe that delivers the inactivating ribozyme(s) and/or toxic agent(s) to the pathogen.

In specific preferred embodiments of the invention, a toxic agent is encoded in a Transfer plasmid, and is used in connection with a P1 bacteriophage delivery system. Such Transfer plasmid preferably contains 1) an origin or replication 2) selectable marker 3) Pac ABC genes with a P1 PAC site 4) P1 lytic replicon and 5) nucleic acids encoding one or more toxic agents of the invention *(e.g.,* antisense molecule, ribozyme, or toxic protein, etc). The Transfer plasmid may be produced in a bacterium producing cell *(e.g.,* a P1 lysogen). In preferred embodiments of the invention, the bacteriophage P1 plasmid *(e.g.,* the P1 prophage) is engineered to be incapable of being packaged into a phage head. In this embodiment, only Transfer plasmids are packaged into virions. Such inhibition of P1 plasmid packaging is accomplished by introducing a mutation or deletion in the P1 plasmid that inhibits the P1 plasmid from being packaged into a virion or phage head. Mutation(s) or deletion(s) of the P1 plasmid which inhibit packaging include but are not limited to one or more mutations and/or deletions in the P1 plasmid PAC site. Any mutation(s) and/or deletion(s) of the P plasmid which inhibits packaging of the bacteriophage P1 plasmid is with in the scope of the invention. Such mutations or deletions are introduced by standard techniques known in the art. In several embodiments, the P1 lysogen has a temperature sensitive repressor mutation (*e·g.* C1.100). Preferably, induction of the P1 lysogen leads to the production of P1 phageheads containing only the packaged Transfer plasmid. Bacteriophage containing the packaged Transfer plasmid nucleic acids may then be used to infect a target cell such as a bacterial pathogen. The bacteriophage infects a bacterial pathogen by injecting its nucleic acids into the bacterium. The toxic agent encoded in the bacteriophage nucleic acids is thus delivered to the bacterium. Within the bacterium, the Transfer plasmid nucleic acids recircularize, and the toxic agent is expressed in the bacterium leading to toxicity and death. Similar mutation and/or deletion strategies may be used with the other viral delivery systems of the invention such that the deletion(s) and/or mutation(s) allow packaging of the nucleic acids encoding toxic agent or ribozyme of the invention, but prevent packaging of nucleic acids encoding one or more viral genes or plasmids. Such strategies allow for construction of viral delivery systems which have increased safety *(e.g.,* when used in connection with therapeutics of the invention).

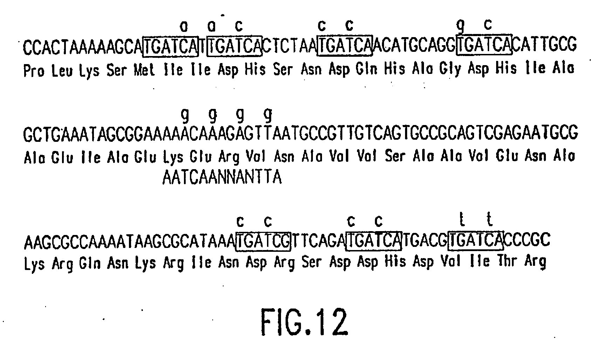

In a specific embodiment, the invention provides a bacteriophage able to package/deliver Transfer plasmid in P1 virions which will infect a pathogenic bacterial target. In another specific embodiment, bacteriophage P1 (PAC site) knockouts able to package/deliver Transfer plasmid DNA but unable to incorporate P1 DNA thus preventing horizontal transfer of undesirable products to non-pathogenic indigenous microflora. In another specific embodiment of the invention, the phage delivery system comprises a Transfer plasmid carrying the genes encoding the antimicrobial agents, a plasmid origin of replication, the P1 lytic origin of replication and a minimal PAC site *(e.g.,* such as the minimal P1 pac site as shown in Figure 12, SEQ ID NO:7). In this embodiment, the plasmid is maintained in a bacteriophage P1 lysogen unable to package its own DNA. The defective lysogen provides all the replication factors needed to activate the P 1 origin of replication on the transfer plasmid and all the structural components necessary to form mature virions. The lysogen also carries the c1.100 temperature-sensitive repressor mutation. C1 is responsible for the repression of functions leading to vegetative phage production. Induction of the lysogen by a temperature shift results in multiplication of DNA, packaging of the transfer plasmid into P1 phage heads and lysis of the production strain. Virions are harvested and used to deliver the Transfer plasmid to the pathogen. The phagehead contains multiple copies of Transfer plasmid DNA and is targeted to pathogenic bacteria by the bacteriophage's natural receptor mediated mechanisms. Upon delivery, plasmid DNA recircularizes and expression of the toxic agent under the control of environmental, virulence-regulated or species-specific promoters results in rapid cell death.

In specific embodiments, the invention provides novel Transfer plasmids encoding toxic agents which may be used in combination with a bacteriophage delivery system in order to treat a bacterial infection in a host.

### Baderiophage Lambda

Another example of a system using bactenophage virions to package DNA carrying ribozymes and/or toxic agents directed against *E. coli* is the bacteriophage lambda. Similar strategies are used to generate virions capable of delivering ribozymes and/or toxic agents directed against other microorganisms. The virions used to package the DNA can be species-specific, such as the virion derived from the bacteriophage lambda coat, or they can possess a broader host range, such as virion derived from bacteriophage P1, as described above. Broad host-range viruses facilitate production of the antimicrobial agents without the loss of species specificity because species-specific promoters are used to direct the transcription of the ribozymes which are directed against species-specific targeted RNA sequences. For example, a lambda bacteriophage entails the use of a plasmid carrying the ribozyme and/or toxic agent, a plasmid origin of replication, a selectable marker for plasmid maintenance, the minimal lambda origin of replication, and cos sites, which are required for packaging of DNA into lambda virions. This plasmid is maintained in a lambda lysogen that is defective in integration/excision and recombination functions. The defective lysogen provides all of the replication factors needed to activate the lambda origin of replication on the plasmid and all of the structural components needed to form mature virions; however, the lysogen is not able to replicate and package its own DNA into the virions. The lysogen may also carry a temperatnre-sensitive represser mutation (such as the cI857).

### Other Viral Vectors

Retroviral vectors are also commonly used to deliver genes to host cells both *in vivo* and *ex vivo.* Retroviral vectors are extremely efficient gene delivery vehicles that cause no detectable harm as they enter the cells. The retroviral nucleic acid may integrate into host chromosomal DNA allowing for long-term persistence and stable transmission to future progeny, such a vector would be useful for the delivery of a toxic agent and/or ribozyme(s) used to target a cellular gene product involved in a chronic or hereditary disorder or to target a viral gene or a microbial gene or a parasitic gene involved in a chronic or persistent infection. An example of an appropriate retroviral vector are, lentiviruses which have the advantage of infecting and transducing non-dividing cells. In such an embodiment, a lentiviral vector encoding a packagable RNA vector genome operably linked to a promoter in which all the functional retroviral auxiliary genes are absent, is used to transfer the DNA encoding the toxic agent and/or ribozyme of the present invention. Examples of such vectors are described in WO 98/17815, WO 98/17816 and WO 98/17817, each of which is incorporated herein by reference in their entirety.

In yet another embodiment, non-integrating viral vectors which infect and transduce non-dividing cells, such as adenoviral vectors may be used to deliver the toxic agent and/or ribozymes of the present invention. Adenoviral vectors have several advantages because the use of such vectors avoids risks associated with pennanently altering the host cell genome or of promoting insedional mutagenesis. Adenoviruses are one of the best developed non-integrating viral vectors and can be used to transfer expression cassettes of up to 75 kb. Recombinant adexiovirases can be produced at very high titers, is highly infectious and efficiently transfers genes to a wide variety of non-replicating and replicating cells and is ideal for *in vivo* mammalian gene transfer.

Adenovirus-based vectors are relatively safe and can be manipulated to encode the desired toxic agent and/or ribozymes and at the same time to be inactivated in terms of their ability to replicate in a normal lytic viral life cycle. Adenovirus has a natural tropism for airway epithelia. Therefore, adenovirus-based vectors are particularly preferred for respiratory gene therapy applications. In a particular embodiment, the adenovirus-based gene therapy vector comprises an adenovirus 2 serotype genome in which the Ela and the EIb regions of the genome, which are involved in early stages of viral replication have been deleted and replaced by nucleotide sequences of interest. In a further embodiment, the adenovirus-based gene therapy vector contains only the essential open reading frame (ORF3 or ORF6 of adenoviral early region 4 (E4) and is deleted of all other E4 open reading frames, or may additionally contain deletions in the E3 regions (*e.g*., *see* U.S. Patent No. 5,670,488, incorporated herein by reference in its entirety). In another embodiment, the adenovirus-based therapy vector used may be a pseudo-adenovirus (PAV), which contain no harmful viral genes and a theoretical capacity for foreign material of nearly 36 kb.

In another embodiment, adeno-associated virus (AAV) systems may be used to deliver the toxic agent and/or ribozymes of the present invention. AAV has a wide host range and AAV vectors have currently have been designed which do not require helper virus. Examples of such AAV vectors are described in WO 97/17458, incorporated herein by reference in its entirety.

Vaccinia viral vectors may be used in accordance with the present invention, as large fragments of DNA are easily cloned into its genome and recombinant attenuated vaccinia variants have been described (Meyer, et al., 1991, J. Gen. Virol. 72:1031-1038). Orthomyxoviruses, including influenza; Paramyxoviruses, including respiratory syncytial virus and Sendai virus; and Rhabdoviruses may be engineered to express mutations which result in attenuated phenotypes (see U.S. Patent Serial No. 5,578,473, issued November 26, 1996). These viral genomes may also be engineered to express foreign nucleotide sequences, such as the selected toxic agent and/or ribozymes of the present invention (see U.S. Patent Serial No. 5,166,057, issued November 24, 1992, incorporated herein by reference in its entirety). Reverse genetic techniques can be applied to manipulate negative and positive strand RNA viral genomes to introduce mutations which result in attenuated phenotypes, as demonstrated in influenza virus. Herpes Simplex virus, cytomegalovirus and Epstein-Barr virus, Sindbis virus and poliovirus (see Palese et al., 1996, Proc. Natl. Acad. Sci. USA 93:11354-11358). These techniques may also be utilized to introduce foreign DNA, *i.e*., the selected toxic agent and/or ribozyme, to create recombinant viral vectors to be used in accordance with the present invention. In addition, attenuated adenoviruses and retroviruses may be engineered to express the toxic agent and/or ribozymes. Therefore, a wide variety of viruses may be engineered to design the ribozymes delivery vehicles of the present invention.

The viral vectors of the present invention may be engineered to express the toxic agents and/or ribozymes in a tissue specific manner. For example, the promoter of the carcinoembryonic antigen (LEA) is expressed in a proportion of breast, lung and colorectal cancers, but rarely in healthy tissues. In order to target a hepatoma, the α-fetoprotein (AFP) promoter whose activity is restricted to malignant cells. Proliferating cells can be targeted with a flt-1 promoter, which has been shown to allow preferential targeting of proliferating endothelial cells. See Miller et al., 1997, Human Gene Therapy 8:803-815, incorporated herein by reference in its entirety.

### 5.3.2. ABIOLOGIC DELIVERY VEHICLES

Abiologic delivery of one or more toxic agents and/or ribozymes is accomplished by a variety of methods, including packaging plasmid DNA carrying the gene(s) that codes for the toxic agent(s) and/or ribozyme(s) into liposomes or by complexing the plasmid DNA carrying the gene(s) that codes for the toxic agent(s) and/or ribozyme(s) with lipids or liposomes to form DNA-lipid or DNA-liposome complexes. The liposome is composed of cationic and neutral lipids commonly used to transfect cells *in vitro.* The cationic lipids complex with the plasmid DNA and form liposomes.

A liposome is provided, comprising a nucleic acid comprising a pathogen specific promoter operably linked to a sequence encoding a trans-acting ribozyme comprising a) a 5' autocatalytically cleaving ribozyme sequence, b) a catalytic ribozyme comprising a target RNA-specific binding site and c) a 3' autocatalytically cleaving ribozyme sequence.

A liposome is provided, comprising a nucleic acid encoding a pathogen-specific promoter operably linked to a sequence encoding one or more toxic agents is provided.

The liposome of the invention, wherein the nucleic acid encodes more than one trans-acting ribozyme and/or more than one toxic agent is provided. The liposome can comprise any ribozyme-encoding nucleic acid, or any toxic agent encoding nucleic agent particularly those described herein. Such nucleic acids may be operably linked to a tissue-specific or pathogen-specific promoter.

The liposomal delivery systems of the invention can be used to deliver a nucleic acid comprising a tissue-specific promoter operably linked to a sequence encoding a multi-ribozyme comprising a) a 5' autocatalytically cleaving ribozyme sequence, b) a catalytic ribozyme comprising a target RNA specific binding site and c) a 3' autocatalytically cleaving ribozyme sequence.

The liposome delivery system of the invention can be used to deliver a nucleic acid comprising a tissue-specific promoter operably linked to a sequence encoding one or more toxic agents. The liposome delivery system of the invention can be used to deliver a nucleic acid comprising a pathogen-specific promoter operably linked to a sequence encoding one or more toxic agents.

Cationic and neutral liposomes are contemplated by this invention. Cationic liposomes can be complexed with a negatively-charged biologically active molecule *(e.g..* DNA) by mixing these components and allowing them to charge-associate. Cationic liposomes are particularly useful when the biologically active molecule is a nucleic acid because of the nucleic acids negative charge. Examples of cationic liposomes include lipofectin, lipofectamine, lipofectace and DOTAP (Hawley-Nelson et aL,1992, Focus 15(3):73-83; Felgner et aL, 1987, Proc. Natl. Acad. Sci. U.S.A. 84:7413; Stewart et al., 1992, Human Gene Therapy 3:267-275). Procedures for forming catiomc liposomes encasing substances are standard in the art (Nicolau et al., 1987, Methods Enzymol. 149:157) and can readily be utilized herein by one of ordinary skill in the art to encase the complex of this invention.

In yet another embodiment of the present invention, the plasmid DNA carrying the gene(s) that codes for the toxic agents and/or ribozymes of the invention are complexed with liposomes using an improved method to achieve increased systemic delivery and gene expression (Templeton et al., 1997, Nature Biotechnology 15: 647-652, incorporated herein by reference in its entirety). In accordance with the present invention, an improved formulation of cationic lipids which greatly increase the efficiency of DNA delivery to host cells, with extended half-life *in vivo* and procedures to target specific tissues *in vivo.* For example, but not by limitation, peptides and proteins may be engineered to the outer lipid bilayer, such as liver-specific proteins, leads to substantially enhanced delivery to the liver etc.

In one embodiment of the present invention, systemic delivery and *in vivo* and *ex vivo* gene expression is optimized using commercially available cationic lipids, *e.g.,* dimethyldioctadeclammonium bromide (DDAB); a biodegradable lipid 1, 2-bis(oleoyloxy)-3-(trimethylammonio) propane (DOTAP); these liposomes may be mixed with a neutral lipid, *e.g.,* L-α dioleoyl phosphatidylethanolamine (DOPE) or cholesterol (Chol), two commonly used neutral lipids for systemic delivery. DNA:liposome ratios may be optimized using the methods used by those of skill in the art *(e.g.,* see Templeton et al., 1997, Nature Biotechnology 15: 647-152, incorporated herein by reference in its entirety).

In yet another embodiment of the present invention, the plasmid DNA carrying the nucleic acids encoding the toxic agents and/or ribozymes of the invention may be delivered via polycations, molecules which carry multiple positive charges and are used to achieve gene transfer *in vivo* and *ex vivo:* Polycations, such as polyethilenimine, may be used to achieve successful gene transfer *in vivo* and *ex vivo (e.g.,* see Boletta et aL, 1996, J. Am. Soc. Nephrol. 7: 1728, incorporated herein by reference in this entirety.)

The liposomes may be incorporated into a topical ointment for application or delivered in other forms, such as a solution which can be injected into an abscess or delivered systemically, or delivered by an aerosol.

Plasmid DNA coding for the ribozymes or toxic agent is used rather than preformed ribozymes or toxic agent for the following reasons. Plasmid DNA allows the targeted cells to produce the toxic agent or ribozyme and, thus, results in a higher delivered dose to the cell than can be expected by delivery of nbozyme RNA or toxic agent via liposome. The DNA also provides specificity of action based on target sequence specificity. The liposomes deliver their DNA to any cell in the area of administration, including cells of the host. The promoter driving the transcription of the toxic agent or ribozyme is specific for the targeted microorganism and, thus, will be inactive in other cell types. Therefore, liposomal delivery of DNA coding for the toxic agent or ribozyme provides amplification and specificity. The present invention relates to promoter elements which are pathogen-specific or tissue-specific. Such promoter elements are used to achieve pathogen-specific or tissue-specific expression of the toxic agent(s) and/orribozyme(s) of the present invention. The invention also relates use of an origin of replication which modulates specificity of the . replication or copy number of a vector or plasmid in a cell or pathogen.

### 5.3.2.1. DELIVERY & EXPRESSION USING MULTI-RIBOZYMES

In another embodiment of the invention, expression of a toxic agent is directed by a tissue-specific, pathogen-specific, and/or target-specific ribozyme or ribozyme cassette. The invention provides ribozymes that have the unique characteristic of being both target RNA-specific in their catalytic action, and tissue-specific or pathogen-specific in their expression. A ribozyme can be tissue-specific in the case of treating tissue-specific disease, or it can be pathogen-specific in the case of treating a pathogen such as *E. coli.* Multi-ribozymes may have one or more target-specific ribozyme(s) *(e.g.,* a trans-acting catalytic ribozyme) as well as elements which control tissue-specific or pathogen-specific expression.

In one embodiment, the nucleic acids of the invention comprise a tissue-specific promoter operably linked to a sequence encoding a) a 5' autocatalytically cleaving ribozyme sequence, b) a catalytic ribozyme comprising a target RNA-specific binding site and c) a 3' autocatalytically cleaving ribozyme sequence.

The tissue-specific promoter in the nbozyme-producing construct results in tissue-specific expression of the ribozyme in tissne(s) that actively transcribe RNA from the selected promoter. Thus, only the target RNA in tissue that utilize the promoter will be cleaved by the ribozyme.

Further, in accordance with the present invention, the multi-ribozyme may consist of one or more ribozyme cassettes. Each cassette in turn may consist of a catalytic core and one or more flanking sequences. In one embodiment, the ribozyme cassette may consist of a 5' autocatalytically cleaving ribozyme sequence, a core catalytic ribozyme comprising a trans-acting ribozyme and a 3' autocatalytically cleaving ribozyme. In yet another embodiment, the catalytic core contains sequences encoding one or more toxic agent(s). In other embodiments, the multi-ribozymes comprise a cassette including, the enhanced 5' and 3' antocatatytically cleaving ribozyme sequence. In another embodiment, the multi-ribozymes contain one or more internal trans-acting ribozymes. In a preferred embodiment, the multi-ribozymes of the present invention include, but are no limited to triple ribozyme cassettes. In another embodiment, multi-ribozymes include but are not limited to one or more triple ribozyme cassettes linked together. In yet another embodiment, the multi-ribozyme comprises a ribozyme cassette containing one or more internal trans-acting ribozyme. In an additional embodiment, the multi-ribozyme comprises a series of one or more ribozyme cassettes containing one or more internal trans-acting ribozymes or any combination thereof In further embodiments, the multi-ribozyme cassettes or toxic agent(s) are expressed in a tissue-specific or pathogen-specific manner. In a preferred embodiment of the invention, pathogen-specific expression is coupled to a pathogen-specific promoter.

### 5.4. PROMOTER SELECTION

Promoter selection is accomplished using techniques that are available in the art. As used herein, regulatory elements include but are not limited to, inducible and non-inducible promoters, enhancers, operators and other elements known to those skilled in the art that drive and regulate expression. Specifically, the invention provides inducible promoters which have increased transcriptional control and high expression levels. The promoter can be a naturally occurring strong, intermediate or weak constitutively expressed or regulated promoter from the targeted microorganism, or an artificially contrived constitutively expressed or regulated promoter containing either a strong intermediate or weak consensus sequence that delivers desired levels of toxic agent or ribozyme expression in the targeted microbe.

Promoters specific for the target (*e.g*., a specific pathogen, genus, etc.) in question can be selected by screening genomic sequences for the ability to activate a promoterless reporter gene. The promoterless reporter gene is based on the strategy developed for use with plasmid pMC1871 (Casadaban et al., 1983, Meth. EnzymoL 100:293). For non-viral pathogens, plasmid capable of stable replication and maintenance in the microorganism understudy is modified by standard molecular biology techniques to carry the coding region of a reporter gene (Sambrook et al. latest edition). The reporter gene can be any of a number of standard reporter genes including but not limited to the lacZ gene of *E*. *coli,* which codes for β-galactosidase. Total genomic DNA is isolated from cells of the pathogen, cleaved with restriction endonucleases to yield fragments of a few hundred basepairs on average. These fragments are then ligated into a unique restriction endonuclease cleavage site at the 5' end of the reporter gene coding region, creating a library of plasmids. The library is then transformed into the pathogen by standard techniques and the resulting transformants are screened for expression of the reporter gene. In the case of lacZ, the transformants can be plated onto medium containing the chromogenic galactosidase substrate X-Gal (5-bromo-4-chloro-3-indolyl-D-galactosade). Transfonnants that contain a plasmid with an insert carrying a promoter will express β-galactosidase and will turn blue on X-Gal plates. The intensity of the blue color is relative to the level of expression; promoters of different strength can be selected based on the intensity of the blue color.

The above-described screening procedure can be modified to identity regulated promoters. For example, promoters that are regulated by carbon source availability can be screened on plates that contain different carbon sources. Other modifications are possible and will depend, in part, on the organism in question. To test for species-specificity, the identified promoters are transferred to promoterless reporter plasmids capable of replication and maintenance in a different organism. Truly species-specific or pathogen-specific promoters will not activate the expression of the reporter gene in any other species. Obvious modifications can be used to identify and test artificial promoters composed of synthetic oligonucleotides inserted into the promoterless reporter plasmid.

In one embodiment, the nucleic acids of the invention comprise a tissue-specific promoter operably linked to a sequence encoding a 5' autocatalytically cleaving ribozyme sequence, one or more catalytic target-specific trans-acting ribozymes or one or more toxic agents and a 3' autocatalytically cleaving ribozyme sequence.

The tissue-specific promoter in the ribozyme-producing construct results in tissue-specific expression of the ribozyme in tissue(s) that actively transcribe RNA from the selected promoter. Thus, only the target RNA in tissue that utilize the promoter will be cleaved by the ribozyme. The pathogen-specific promoter binding site in the ribozyme-producing construct results in pathogen-specific expression of the ribozyme in pathogens or microbes that actively transcribe RNA from the selected promoter. Thus, only the target RNA in pathogens that utilize the promoter will be cleaved by the ribozyme.

Tissue-specific promoters can be used in the present nucleic acid constructs. Examples of these promoters include the sequences for probasin-promoter, a promoter-specific for prostate epithelium prostate-specific antigen (prostate), keratin, k7 (epidermal sebaceous glands), albumin (liver), fatty acid binding protein (ileum), whey acidic protein (breast), lactalbumin, smooth muscle actin (smooth muscle), etc. In a specific embodiment, a mouse albumin promoter/enhancer is used which consists of nucleotides 338-668 from GenBank® accession # U04199, followed by the sequence gagtcgacggatccgg, followed by nucleotides 1762-1846 from accession # J04738, followed by the nucleotide sequence tgggggtgggggtgggg followed by nucleotides 1864-2063 of accession # J04738. In one embodiment, the mouse promoter/enhancer is active in hepatocyte(e.g., human hepatocytes, hepatocyte cultures, etc.) and is useful for tissue-specific expression in liver tissue. It will also be clear that target-specific promoters not yet identified can be used to target expression of the present ribozymes to the selected tissue(s). Once a target-specific promoter is identified its binding sequence can be determined by routine methods such as sequence analysis may be used. The promoter is defined by deletion analysis, mutagenesis, footprinting, gel shifts and transfection analyses (Sambrook et al., *Molecular Cloning: A Laboratory Manual,* 2nd Ed., Cold Spring Harbor Laboratory, Cold Spring Harbor, New York, 1989). Pathogen-specific promoters can be used in the present nucleic acid constructs.

### 5.4.1. BACTERIAL SPECIFIC PROMOTERS/EXPRESSION

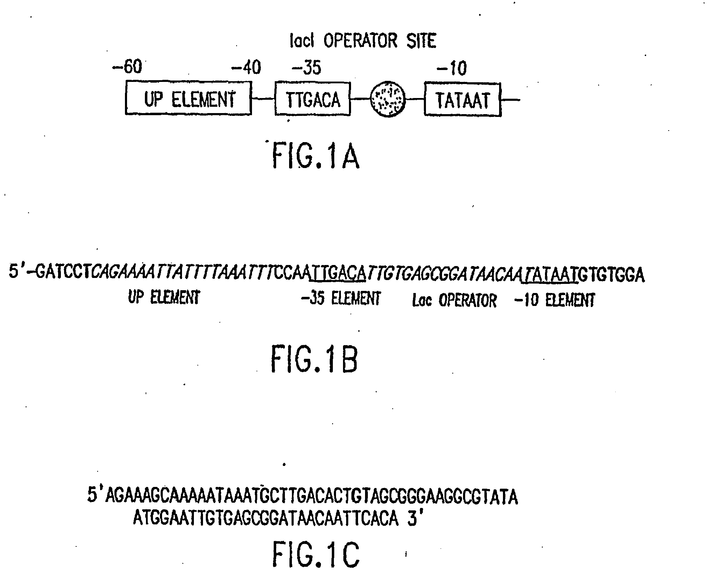

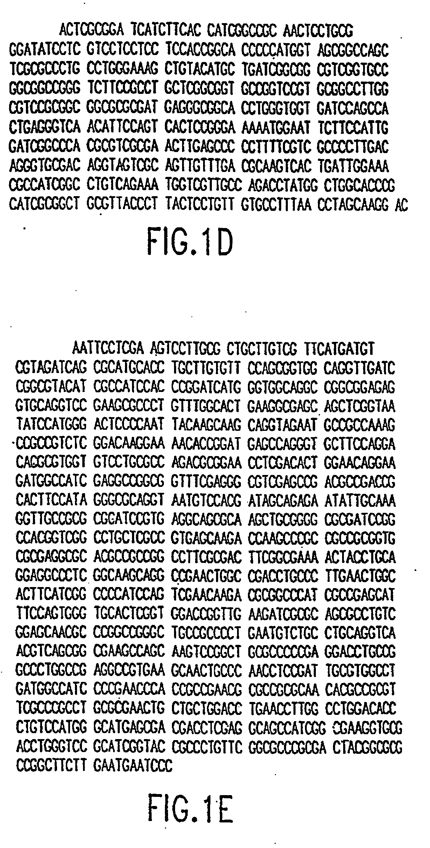

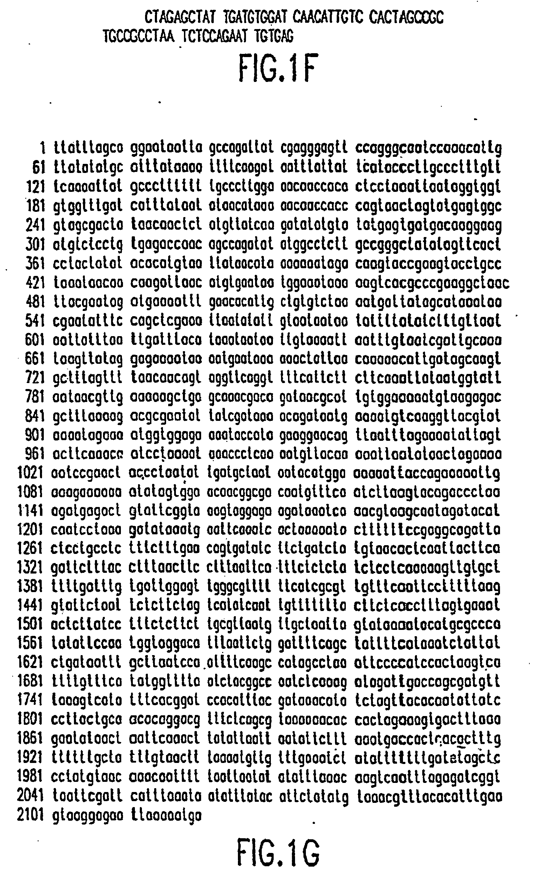

The present invention provides bacterial promoters that allow for tight regulation of transcription and enhanced expression. In one embodiment, a novel promoter called LEASED has been constructed from three elements (see Figure 1). The first element, termed RIP is a combination of two consensus sites at -10(TATAAT) and -35(TTGACA) located with respect to transcription initiation. The second element is based on the lacI repressor binding sequence (termed lac operator sequence) which is placed between the -10 and -35 consensus sites. This is in contrast to the conventional lac and tac promoters where the lac operator is found downstream of the -10 consensus element. Placement of the lac operator between the -10 and -35 sites, more effectively blocks RNA polymerase binding to the promoter, thus enhancing transcriptional control from the promoter. Thus, the levels of lacI repressor protein present, which binds to the operator sequence and hence determines the rate of transcription, are controlled in two ways; 1) by endogenously expressed IacI protein and 2) by a plasmid expressing the lacI gene. Under normal conditions, the 1acI repressor protein binds to the lac operator sequence and prevents transcription by blocking RNA polymerase binding. The promoter is 'switched on' following the addition of isopropyl B-D-thiogalacto pyranoside, which binds and subsequently titrates out the repressor protein. RNA polymerase can then bind to the promoter and transcription can proceed.

The third element of the LEASHI promoter is termed the UP element. The UP element is an adenine/thymine rich sequence which is placed immediately upstream of the -35 element. Addition of the UP element, further increases expression from this promoter. Accordingly, the invention provides the use of a LEASHI promoter to express the toxic agents of the invention.

In a specific embodiment of the invention, the promoter which is operably linked to a nucleic acid encoding a toxic agent or ribozyme is the LEASHI promoter.

In specific embodiment, a ribozymes of the invention is operably linked to a LEASHI promoter. In another specific embodiment of the invention, a toxic agent of the invention is operably linked to a LEASHI promoter.

In a specific embodiment, the invention encompasses expression of *DicF1* from a ribozyme cassette under the control of a regulatable promoter, such as the LEASHI promoter.

In another embodiment of the invention, the lacI operator sequence of the LEASHI promoter is placed 5' of the -35 consensus site. In another embodiment of the invention, the lacI operator sequence of the LEASHI promoter is placed 3' of the -10 consensus site. In other embodiment of the invention, one or more additional lacI operator sequences are added to the LEASHI promoter and are placed 5' to the -35 consensus site and/or 3' of the of the -10 consensus site.

In other specific embodiments, the invention provides for the use of an *anr*, *arc.* or *proC* promoter. Both are transcriptionally off in *E*. *coli* and on in *Pseudomonas aeruginosa.* These promoters provide the advantage of allowing controlled expression of the toxic agents in the pathogen *(Pseudomonas),* while allowing the packaging strain *(E. coli)* to be protected from the toxic actions of the toxic agent therapeutic. Such promoters are particularly useful to facilitate manufacturing of the delivery vehicle. Such promoters also enable bacterial specific targeting of the gene therapeutic in the patient In specific embodiments, an anr promoter is operably linked to a sequence encoding a toxic agent (such as *doc, gef, chpBK,* or *kicB,* etc), and may be used, for example, for the eradication of *Pseudomonas.*

In other specific embodiments, the invention provides for the use of the *TSST-1* promoter. TSST-1 is an environmentally regulated staphylococcus-specific promoter. TSST-1 is useful to express *doc* or other toxic agents. A staphylococcus specific phage capable of delivering the transfer plasmid into S. *aureus* strains is used to specifically target the Staphylococcal pathogen.

Other classical bacterial inducible promoters are renowned for their inability to tightly control transcription, and a significant level of background expression is characteristically observed. A significant advantage of the promoter of the present invention is that it will alleviate the high levels of background commonly observed in inducible promoters. A limiting factor leading to high background levels of transcription when a promoter of interest is on a high-copy number plasmid is due to the lack of repressor molecules available to bind to the promoters. The present invention overcomes this problem by using a lacI expression plasmid and secondly, by placement of the lac operator between the -35 and -10 consensus elements which more effectively blocks transcription during normal conditions. Furthermore, the UP element placed immediately upstream of the -35 region enhances transcription from the core promoter.

The invention also relates to the rmB promoter. In one embodiment of the invention, the promoter is the rmB promoter is modified such that one or more lacI operator sites are added to the promoter. An example of such a modified rmB promoter is shown in Figure 1B. In another embodiment of the invention, the lacI operator sequence of the rmB promoter is placed 3' of the -10 consensus site. In other embodiment of the invention, one or more additional lacI operator sequences are added to the rmB promoter and are placed 5' to the -35 consensus site and/or 3' of the of the -10 consensus site.

### 5.5. HOST CELLS

The present invention encompasses the expression of the toxic agents and/or ribozymes in primary cells, animal, insect, fungal, bacterial, and yeast cells for *in vitro* screening assay and *ex vivo* gene therapy. The present invention also encompasses the expression of the toxic agents and/or ribozymes in cell lines for *in vitro* screening assay and *ex vivo* gene therapy. In accordance with the present invention, a variety of primary or secondary cells or cell strains may be used including but not limited to cells isolated from skin, bone marrow, liver, pancreas, kidney, adrenal and neurological tissue to name a few. Other cell types that may be used in accordance with the present invention are immune cells (such as T-cells, B-cells, natural killer cells, etc.), macrophages/monocytes, adipoctyes, pericytes, fibroblasts, neuronal cells, reticular cells etc. In a further embodiment, secondary cell lines may be used as engineered responsive cells and tissues in accordance with the present invention, including, but not limited to hepatic cell lines, such as CWSV, NR, Chang liver cells, or other cell lines such as CHO, VERO, BHK, Hela, COS, MDCK, 293, 373, HUVEC, CaSki and W138 cell lines. A toxic agent or ribozyme of the invention may also be expressed in any cell line which is not sensitive to the effects of the toxic agent or ribozyme (*e*.*g*., a cell which is resistant to the particular toxic agent or ribozyme, or a cell which co-expresses a neutralizing agent or antidote).

For long term, high-yield production of recombinant proteins, stable expression is preferred. For example, cell lines which stably express the selected toxic agent and/or ribozyme may be engineered. When a toxic agent is to be stably expressed, expression may be controlled by an inducible promoter, or, the cell may be engineered to co-express an antidote to the toxic agent, in order to allow the cell to survive during production of a toxic agent. Rather than using expression vectors which contain viral origins of replication, host cells can be transformed with DNA controlled by appropriate expression control elements *(e.g.,* promoter sequences, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to grow to form foci which in turn can be cloned and expanded into cell lines. This method may advantageously be used to engineer cell lines. This method may advantageously be used to engineer cell lines which express the selected gene products. Such cell lines would be particularly useful in screening and evaluation of compounds that affect the endogenous activity of the selected gene product.

A number of selection systems may be used, including but not limited to the herpes simplex virus thymidine kinase (Wigler, et al.,1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al, 1980, Cell 22:817) genes can be employed in tk, hgprt or aprt cells, respectively Also, antimetabolite resistance can be used as the basis of selection for the following genes: dhfr, which confers resistance to methofirexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al.,1981, J. Mol. Biol. 150:1); and hygro, which confers resistance to hygromycin (Santerre, et al., 1984, Gene 30:147).

### 5.6. TARGETS

The present invention provides a toxic agent or a trans-acting ribozyme which targets any cellular, viral, bacterial, fungal, or other single cellular or multicellular organism from any known taxonomic family, genus, or species. Another embodiment of the invention provides a toxic agent which is lethal or toxic to a pathogen such as a bacteria, fungus, yeast, diseased cell. Such toxic agents may be delivered to the pathogen by the methods of the invention. The microorganisms may be any virus, nonvirus, bacterium, or lower eukaryotes such as fungi, yeast, parasites, protozoa, or other eukaryotes that maybe considered pathogens of humans, animals, fish, plants, or other forms of life. In several embodiments, the targets of the antimicrobial ribozyme therapeutics described herein are the RNAs of invading or normal flora microorganisms. In other embodiments, the targets of the antimicrobial toxic agent therapeutics described herein include RNAs, proteins, genes and other molecules of invading or normal flora microorganisms. Thus, the invention has important implications in human and veterinary medicine.

The toxic agents of the present invention may be engineered to target essential genes, gene products, or processes necessary for growth of parasites, bacteria, virus life cycles, etc., and expression can be driven with tissue-specific or pathogen-specific promoters. The toxic agents or trans-acting catalytic nbozymes of the present invention may be engineered to target a wide variety of cellular RNAs, tumor or cancer associated with RNAs, bacterial RNAs, parasitic RNA etc. For example, ribozyme targets sites are indicated in Tables 11, 13 and 13 herein. The toxic agent or trans-acting ribozyme can be targeted to noncellular RNAs necessary for growth of parasites, bacteria, virus life cycles, etc., and expression can be driven with tissue-specific or pathogen-specific promoters. 0 The virion construct used in this method can comprise any nucleic acid encoding a toxic agent or ribozyme, particularly those described herein targeted to essential genes of the pathogen or diseased cell. The virion can be a bacteriophage, or other virus selected for its ability to target a specific cell-type, microorganism or animal. The bacteriophage can be lambda, P1, Phi-11 or other phage. When P1 is the virion, the transfer plasmid can further comprise a PAC site and PAC ACB genes. This construct is preferred when using P1. Alternatively, the virion can be selected because it has a broad range of targets. Important examples which are specifically presented in the application are:
A) Use of the LEASHI promoter with a Bacterial target (such as *E*. *coli)* to direct expression of the toxic agent such as *doc, gef, chpBK kicB or DicFl;*
B) Use of the LEASHI promoter with a Bacterial target (such as *E*. *coli)* to direct expression of the toxic agent comprising *Sof* sense RNA;
C) Use of the *anr, arc,* or *proC* promoter with a *Pseudomonas* target (such as P. *aeruginosa)* to direct expression of a toxic agent such as *doc, gef, chpBK kicB or DicF1;*
D) Use of the *TSST-1, hla,* or *SrcB* promoter with a *Staphylococcus* target (such as S. *aureus)* to direct expression of the toxic agent such as *doc, gef, chpBK, kicB , pemK, hok, relF, sigB,* or *lysostaphin;*
E) Use of the albumin promoter with a Hepatitis B virus target (chosen to cleave the vital RNA pregenome, S protein, and polymezase/and x protein transcripts using the same ribozyme target site);
F) Use of the albumin promoter with Hepatitis B virus and Hepatitis C targets (using trans-acting nbozyme target sites on both Hepatitis B virus and Hepatitis C);
G) Use of generic promoters active in erythrocytes, using a ribozyme targeted to highly conserved regions of the EMP-1 protein family from *P. falciparum,* which are necessary for cytoadherence and antigenic variation in malaria;
   and
H) Use of the keratin 7 promoter, with trans-acting ribozymes targeted to a specific sites near the translational start site of the E6 protein, a site known to be critical for expression of both the E6 and E7 proteins which are intimately involved in cervical carcinogenesis, as well as a more 3' site in a highly conserved region of the B6 protein.

Examples of bacterial pathogens that can be targeted by a toxic agent or trans-acting ribozyme of the present invention include, but are not limited to, species of the following genera: *Salmonella, Shigella, Chlamydia, Helicobacter, Yersinia, Bordatella*, *Pseudomonas, Neisseria, Vibrio, Haemophilus, Mycoplasma, Streptomyces, Treponema. Coxiella, Ehrlichia, Brucella. Streptobacillus, Furospirocheta, Spirillum, Ureaplasma; Spirochaeta, Mycoplasma, Actinomycetes, Borrelia, Bacteroides, Trichomoras. Branhamella, Pasteurella, Clostridium, Corynebacterium, Listeria, Bacillus, Erysipelothrix, Rhodococcus, Escherichia, Klebsiella, Pseudomonas, Enterobacter, Serratia, Staphylococcus, Streptococcus, Legionella, Mycobacterium, Proteus, Campylobacter, Enterococcus, Acinetobacter, Morganella, Moraxella, Citrobacter, Rickettsia, Rochlimeae,* as well as bacterial species such as: P. *aeruginosa; E. coli, P. cepacia, S. epidermis, E. faecalis, S. pneumonias, S. xylosus, S. aureus, N. meningitidis, S. pyogenes, Pasteurella multocida, Treponema pallidum,* and P. *mirabilis.*

The pathogen of the present invention can also include, but is not limited to pathogenic fungi such as *Cryptococcus neoformans; Blastomyces dermatitidis; Aiellomyc*es *dermatitidis, Histoplasma capsulatum; Coccidioides immitis*; *Candida species, including* C. *albicans, C*. *tropicalis,* C. *parapsilosis, C*. *guilliermondii and* C *krusei, Aspergillus species, including A. fumigatus, A. flavus and A. niger, Rhizopus species; Rhizomucor species; Cunninghammella species; Apophysomyces species, including A. sakrenaea, A. mucor and A. absidia; Sporothrix schenckii, Paracoccidioides brasiliensis; Pseudallescheria boydii, Torulopsis glabrata; Trichophyton species, Microsporum species and Dermatophyres species,* as well as any other yeast or fungus now known or later identified to be pathogenic.

Furthermore, the pathogen of the present invention can be a parasite, including, but not limited to, members of the Apicomplexa phylum such as, for example, *Babesia, Toxoplasma, Plasmodium, Eimeria, Isospora, Atoxoplasma, Cystoisaspora, Hammondia, Besmotia, Sarcocystis, Frenkelia, Haemoproteus, Leucocytozoon, Theileria*, *Perkinsus and Gregarina spp.; Pneumocystis carinii;* members of the Microspora phylum such as, for example, *Nosema, Enterocytozoon, Encephalitozoon, Septata, Mrazekia, Amblyospora, Ameson, Glugea, Pleistophora and Microporidium spp.;* and members of the Ascetospora phylum such as, for example, *Haplosporidium spp.,* as well as species including *Plasmodium falciparum, P. vivax, P. ovale, P. malaria; Toxoplasma gondii; Leishmania mexicana, L. tropica, L. major, L. aethiopica, L. donovani, Trypanosoma cruzi, T. brucei, Schistasoma mansoni, S*. *haematobium, S. japonium; Trichinella spiralis; Wuchereria bancrofti; Brugia malayli; Entamoeba histolytica; Enterobius vermiculoarus; Taenia solium, T. saginuta, Trichomonas vaginatis, T. hominis, T. tenax; Giardia lamblia; Cryptosporidium parvum; Pnemocytis carinii, Babesia bovis, B. divergens, B. microti, Isospora belli, L hominis; Dientamoeba fragilis; Onchocerca volvulus; Ascaris lumbricoides; Necator americanis; Ancylostoma duodenale; Strongyloides stercoralis; Capillaria philippinensis; Angiostrongylus cantonensia; Hymeicolepis nana; Diphyllobothrium latum; Echinococcus graraulosus, E. multilocularis; Paragonimus westermani. P. caliensis; Chlonorchis sinensis; Opisthorchis felineas, G*. *Viverini, Fasciola hepatica, Sarcoptes scabiei, Pediculus humanus; Phthirius pubis; and Dermatobia hominis,* as well as any other parasite now known or later identified to be pathogenic.

Examples of viral pathogens include, but are not limited to, retroviruses (human immunodeficiency viruses), herpes viruses (herpes simplex virus; Epstein Barr virus; varicella zoster virus), orthomyxoviruses (influenza), paramyxoviruses (measles virus; mumps virus; respiratory syncytial virus), picoma viruses (Coxsackie viruses; rhinoviruses), hepatitis viruses (hepatitis C), bunyaviruses (hantavirus; Rift Valley fever virus), arenaviruses (Lassa fever virus), flaviviruses (dengue fever virus; yellow fever virus; chikungunya virus), adenoviruses, birnaviruses, phleboviruses, caliciviruses, hepadnaviruses, orbiviruses, papovaviruses, poxviruses, reoviruses, rotaviruses, rhabdovirues, parvoviruses, alphaviruses, pestiviruses, rubiviruses, filiviruses, coronaviruses, as well as any virus of the family of picornaviridae; caliciviridae; togaviridae; flaviviridae; coronaviridae; rhabdoviridae; filoviridae; paramyxoviridae; orthomyxoviridae; bunyaviridae; arenaviridae; reoviridae; retroviridae, hepadnaviridae; parvoviridae; papovaviridae; adenoviridae; herpesviridae; and poxyviridae, and any other virus now known or later identified to be pathogenic.

### 5.7. TARGET SELECTION

One critical component in the development of the therapeutics of the invention is the selection of appropriate targets.

### Toxic Agent Targets

The inventions toxic agents are selected based on their ability to inhibit the growth of a pathogen or selected cell or cause lethality in a pathogen or selected cell, or render a pathogen or selected cell less fit. Several specific examples of toxic agents are described herein which serve to illustrate the selection of a toxic agent of the invention.

For example, a toxic agent may be an addiction system toxin (such as *doc). Doc* encodes a toxin which is translationally coupled to a protein called *phd. Phd* is an antidote to *doc,* and acts to neutralize the toxic effects of *doc.* The two proteins, *phd* and *doc* form an operon on the P1 plasmid in which *phd* precedes *doc*. Further, the *phd* gene contains a ribosome entry site and is translated efficiently The native *doc* gene however, lacks a recognizable ribosome entry site and is translated poorly. Thus, *doc* was selected because of its potential toxicity when expressed in a cell or pathogen lacking the corresponding antidote, *phd.* In this embodiment, *doc* has been engineered to be uncoupled from *phd.* For example, *doc* is engineered into a separate plasmid from *phd.* The plasmid containing *doc* has also been engineered such that a ribosome entry site has been constructed upstream of the nucleic acids encoding *doc* in order to increase the levels of translation of *doc.* This plasmid is containing the toxic agent and/or ribozyme of the invention is called the Transfer plasmid. In one specific embodiment of the invention, the Transfer plasmid encodes the toxic agent *doc.*

A packaging strain (*e.g*., bacteria cell) is then used to package the Transfer plasmid containing *doc* into a bacteriophage phage head. The packaging strain cells contain the P1 plasmid as well as the Transfer plasmid with the uncoupled *doc* and ribosome entry site. The packaging strain may also include a third plasmid, if necessary, which encodes additional phd protein which can act to protect the packaging strain against the toxicity of *doc (e.g.,* if the promoter of the Transfer plasmid is leaky and leads to production of *doc* in the packaging cell).

Thus, the packaging strain acts to package the transfer plasmid containing the toxic agent (such as *doc*) into phage heads or virions. Phage lysates of the packaging strain contain the infectious bacteriophage virions.

The phage lysates are then used to infect a selected pathogen (*e.g., E. coli, P. aeruginosa, etc.).* Further, the phage lysate may be used to prepare a therapeutic of the invention, such as a pharmaceutical preparation. Phage maybe delivered to a bacteria or pathogen or a host with a pathogenic infection by methods described herein, or by any method known in the art. For example, the phage lysates may be lyophilized and delivered to a host in need of treatment for a bacterial infection, fungal infection, etc.

The above targeting method, wherein the virion is a bacteriophage is provided. The bacteriophage can be lambda, P1 or other phage. The targeting method, wherein the Transfer plasmid further comprises a PAC site and PAC ABC genes is also provided. The bacteriophage P1 which is engineered to be packaging deficient is also provided. This construct is preferred when using P1.

### Antisense Targets

A toxic agent of the invention may be an antisense molecule selected to target an antidote of a toxic protein, or selected to target an essential RNA critical to the survival of a pathogen or selected cell The proposed target of the toxic antisense molecule of the invention may also be the RNA of any gene which plays a critical role in the survival of the pathogen, or which is essential to the pathogen's life cycle. The present invention also encompasses modifications to naturally occurring antisense molecules which modulate the expression of an essential gene product of a pathogen. For example, as described below, one proposed target of an antisense of the invention is the *ftsZ* gene whose gene product plays a critical role in the initiation of cell division of *E. coli.* For example, the toxic agent may be an antisense molecule which is constructed to be modified and enhanced such that is it more homologous to its target RNA. Thus, as in the case *of DicF,* the antisense sequence has been modified and enhanced to engineer the *DicF1* antisense toxic agent, which has increased complementarity to its target RNA. Further, the *DicF1* or *DicFl-like* antisense molecule has enhanced properties in that it may be expressed and delivered by the methods of the invention, thus providing the target cell with increased levels of the toxic antisense RNA.

Third, a toxic agent may be selected to target an essential antisense molecule. Thus, a toxic agent may be a sense molecule which is designed to be complementary to an essential antisense RNA. An example of an essential antisense molecule is *Sof. Sof* is an antisense antidote for the chromosomally encoded toxin called *gef* (Poulsen, L, et al., 1991, Mol. Microbiology 5:1639-48). *Sof* normally acts to regulate the levels ofgefin the bacterium. The inventors of the present invention have designed sense molecules which are complementary to *Sof.* The sense molecules against *Sof* act to inhibit the ability *of Sof to* regulate *gef,* and thus cause toxicity in the pathogen by allowing the endogenous *gef* levels to become toxic.

### Ribozyme Targets

For ribozymes to be effective anti-microbial therapy, it is preferable to target the RNA of, for example, several key proteins, tRNA, rRNA or any other RNA molecule essential for cell viability or fitness, in order to insure complete inactivation and prevent escape of the invading microorganism.

The complexity of human RNA is about 100 fold lower than that for human DNA, and specificity can be achieved with as few as 12-15 base pairs. The stability of the RNA-RNA duplex is effected by several factors, such as GC content, temperature, pH, ionic concentration, and structure. The nearest neighbor rules can provide a useful estimate of the stability of the duplex (Castanotto et al. "Antisense Catalytic RNAs as Therapeutic Agents", *Advances in Pharmacol.* 25:289-317,1994).

The catalytic ribozyme of the invention also includes a catalytic sequence, which cleaves the target RNA near the middle of the site to which the target RNA-specific sequences bind. In the hammerhead-type of ribozyme, the catalytic sequence is generally highly conserved. The conserved catalytic core residues are 5' CUGANGA 3' and 5' GAAA 3' linked by an evolutionarily conserved stem-loop structure.

The most conserved and probably most efficiently cleaved sequence on the target RNA is 5' GUC 3'. However, NUX (wherein X = A, U or C) can also be cleaved efficiently. Such cleavage sites are ubiquitous in most RNAs allowing essentially all RNA's to be targeted (Whitton, J. Lindsay "Antisense Treatment of Viral Infection" *Adv. in Virus Res.* Vol. 44,1994).

With regard to the selection of the appropriate sites on target RNA, it is known that target site secondary structure can have an effect on cleavage *in vitro* (Whitton, 1994 supra). A number of procedures are available to select accessible sites in RNA targets. In a preferred procedure, a library screen may be employed to select appropriate sites on the target RNA. Accessibility of the selected site may then be confirmed using techniques known to those skilled in the art. Thus, the selected target molecules sequence can be routinely screened for potential secondary structure, using the program RNAFOLD (from the PCGENE group of programs or available on the Internet). Thus, reasonable predictions of target accessibility can be made. Computer assisted RNA folding (Castanotto et aL, 1994), along with computational analysis for 3-dimensional modeling of RNA (Major et al.; *Science* 253:I255-1260,1991 and Castanotto et al.,1994) is certainly effective in guiding the choice of cleavage sites. The nucleic acid, wherein at least one trans-acting nbozyme is targeted to a *ccdA, kis, pemI, parD, phd, higA, chpAI, chpBI, kicA, soc, SOS, srnC, flmB, pndB, sof, korA, korB, korC, korD, korE, or korF* transcript of the pathogen is provided. The nucleic acid, wherein at least one trans-acting nbozyme is targeted to the *rpoA* transcript of the pathogen is provided. The nucleic acid, wherein at least one trans-acting ribozyme is targeted to the *secA* transcript of the pathogen is provided. The nucleic acid, wherein at least one trans-acting ribozyme is directed to the dnaG transcript of the pathogen is provided. The nucleic acid, wherein at least one trans-acting ribozyme is directed to the *ftsZ* transcript of the pathogen is provided. A nucleic acid encoding a multi-ribozyme can encode all or some of the above trans-acting ribozymes. The ribozymes can all be under the control of a single promoter.

For example, several bacterial genes, essential for viability and unrelated in activity, have been selected and are described herein to highlight how the selection of appropriate mRNA targets is carried out for the preferred construction of the antimicrobial agent against prokaryotic targets. Cross-genera RNA targets can be used to design an antimicrobial that can have broad application, modified by the specificity of the promoter. In addition, several toxic agents are described herein to highlight how the selection of appropriate toxic agents is carried out for the preferred construction of the antimicrobial agent against prokaryotic targets.

In one embodiment of the invention, the first ribozyme targets an essential transcription factor, the second ribozyme targets an essential general secretory component, the third ribozyme targets an essential component of the primosome required for DNA biosynthesis and the fourth ribozyme targets an enzyme required for cell division. Consequently, the ribozymes are redundant in the fact that they inhibit growth by specifically targeting a fundamental process required for bacterial growth. Thus, this can minimize the development of resistance to the antimicrobial therapeutic.

For example, one target is the essential protein, *rpoA* or the alpha subunit of RNA core polymerase. *rpoA* was selected rather than the other components of the RNA polymerase holoenzyme, because it is thought to facilitate the assembly of an active RNA polymerase enzyme complex. Inactivation of the *rpoA* transcript results in a decrease in the intracellular concentration of the holoenzyme RNA polymerase rendering the cell less able to respond to changes demanded of it once it has invaded a new host The nucleotide sequence of *rpoA* is known for a large number of microorganisms (>20 genera) and they are readily available from GenBank®.

A second example of a ribozyme target can be the mRNA of the *secA* gene from bacteria. The product of this gene is the essential and rate-limiting component of the general secretory pathway in bacteria (Bassford et al.,1994, Nucleic Acids Reseaarch Apr. 11, 22(7):1326; Nucleic Acid Research. 22(3):293-300). *SecA* has been found in every prokaryotic cell investigated to date. Additionally, its biosynthesis is translationally coupled to the upstream gene, X (Schmidt et al., 1991, J. Bactesiol.173(20):6605-11), presenting a convenient target for a ribozyme. Inhibition or decreased synthesis of *secA* is also sufficient to confer a reduction in viability to the cell (Schmidt et al.,1987, J. BacterioL 171(2):643-9). Furthermore, as a pathogen responds to changes required of the infectious process a change in the availability of a key protein such as *secA* will disadvantage the pathogen enabling the host to counteract it. Finally, control over the secretion-responsive expression of *secA* is at the level of translation (Christoffersen et al., 1995, J. Med. Chem. 38(12):2023-37), and the regulatory sequences within its polycistronic message have been localized to a region comprised of the end of the upstream gene, X, and the beginning of *secA.* Consequently, inactivation of the transcript by the catalytic cleavage of a ribozyme has profound consequences for the viability of the invading microorganism.

The third ribozyme can target essential factor for DNA biosynthesis, such as DnaG. Every 1 to 2 seconds, at least 1,000 times for each replication fork within *E. coli,* priming of an Okazaki fragment is repeated as a result of an interaction between the cellular primase dnaG (Bouche et al., 1975, J. Biol. Chem. 250:5995-6001) and *dnaB* (Marians, KJ. 1996, Replication Fork Propagation, p. 749-763. In F.C. Neidhardt (ed.), Escherichia coli and Salmonella: Cellular and Molecular Biology, 2nd ed, vol.1. American Society for Microbiology, Washington, DC.). As would be expected of a protein required every 1 to 2 seconds during replication, a lesion within DnaG or an alteration in its concentration results in an immediate stop phenotype (Marians, KJ.1996, Replication Fork Propagation, p. 749-763. In F.C. Neidhardt (ed.), Escherichia coli and Salmonella: Cellular and Molecular Biology, 2nd ed, vol.1. American Society for Microbiology, Washington, DC.); Weschler et al,1971, Mol. Gen. Genet. 113:273-284). Therefore, inactivation of the DnaG message by a ribozyme should have profound cellular consequences in that general priming of the lagging strand is reduced if not eliminated. DnaG is a component of the primosome, a multi-protein complex responsible for priming replication. Any of the components of the primosome, either individually or in any combination, can serve as a target for inactivation of the primosome and, thus, kill the cell. The other components of the primosome are *DnaB, DnaC,* DnaT, PriA, PriB, and PriC. Thus, the primosome is also sufficiently complex to provide numerous other targets (DnaB, DnaC, DnaT, PriA, PriB and PriC) for inactivation by the trans-acting ribozyme.

A fourth target can be *ftsZ.* This gene also encodes an essential protein,*ftsZ*, that is required for cell division in that it is responsible for the initiation of separation. *ftsZ* was selected because cleavage of the *ftsZ* RNA leads to inhibition of cell division and a reduction in viability. Any toxic agent or ribozyme which targets *ftsZ* (such as *DicF1*) may be used to inhibit division of a cell requiring the *ftsZ* gene product. Also, for example, upon cleavage of the *ftsZ* message by a ribozyme, such ribozyme can attack additional copies of the *ftsZ* message inhibiting the division of the cell. The nucleotide sequence of *ftsZ* like the other targets selected, is commonly available from GenBamk®.

It should be clear that any other essential protein of a pathogen can have its message targeted in the present invention, and that determining which proteins are essential can be routinely determined according to standard protocols in the art. In fact, there are over 52,000 viral, 41,000 bacterial and 12,300 fungal sequences deposited in the public section of the Entrez Database at the National Center for Biotechnology Information. Any of these can be used to design the catalytic trans-acting ribozyme of the invention.

In addition to targeting mRNA of essential proteins, ribozymes may be targeted against other RNA species within the cell. Specifically, appropriate targets in bacteria, fungi and other lower eukarytoes include ribosomal RNA such as Small Subunit RNAs (SSU) or Large Subunit (LSU) and tRNA molecules required for protein synthesis. With respect to pathogenic *Staphylococci,* the RNA III moiety in a relatively low abundance transcript which is not translated and should be accessible for cleavage. As long as the RNA targeted contains a canonical ribozyme cleavage domain, the ribozyme therapeutic can hybridize and cleave the complementary RNA, thus impacting the fitness of the microbial cell. Additionally, over 3000 rRNA species have been sequenced and aligned. This information is available from the Ribosomal Database Project and should facilitate rapid design and adaptation of ribozyme(s) against such targets. For example the 16S rRNA molecule of bacteria is especially attractive in that there are over 4000 copies of the 16S rRNA per cell. Consequently, a reduction in number slows the process of protein synthesis in so far as the 16S rRNA molecule is involved in the process of translational initiation. Thus, a toxic agent or ribozyme directed against mRNA and rRNA impacts the fitness of the offending microorganism.

### 5.8. PROTECTION OF TOXIC AGENT AND/OR RIBOZYME PRODUCING CELLS

The nucleic acids coding for the toxic agents or ribozymes can be toxic to the cells that are needed to produce the toxic agent or ribozyme-carrying virions. When using a broad host-range virus like P1, the organism used to produce the virion can be different from the target organism. In this way, the producing strain is resistant to the toxic effects of the toxic agents or ribozymes because they are not efficiently expressed in the producing strain, due to species-specific promoter elements, and the ribozymes will not have any target RNA molecules to attack, due to the species-specific sequences that target the ribozymes. When using a species-specific virus that must be expressed and assembled within a strain of the targeted microorganism, this toxicity becomes a significant concern. The assembly of a virion consisting of anti-*E*. coli ribozyme or toxic agent genes packaged in lambda will illustrate the approach used to circumvent the toxicity. For example, the ribozymes directed against RNA species of *E. coli* is expressed from an artificial promoter containing consensus promoter elements. This promoter provides high level transcription of the ribozyme immediately upon infection of targeted cells, In order to prevent the unwanted death of the producing strain of *E. coli,* transcription is repressed in the producing strain by a mechanism not available to the wild type strains that are targeted for killing. Sequences constituting the DNA binding sites for a heterologous transcription factor are interspersed between the essential activating elements of the ribozyme promoter. Expression of the heterologous transcription factor in the producing strain results in the occlusion of the activating promoter elements and preventing the binding of RNA polymerase. As an example, the gene for the *Saccharomyces cerevisiae* transcription factor Ste12p may be expressed in *E*. *coli* and bind to its binding sites, the pheromone response element, located within the ribozyme promoter. Ste12p will not be found in wild strains of *E. coli;* therefore, the ribozyme promoter will be accessible to RNA polymerase following delivery of the plasmid to the targeted cells.

An alternative strategy that can protect the producing strain from the toxicity of the ribozymes employs ribozyme-resistant versions of the targeted RNA molecules. This strategy can be used when the target RNA molecule codes for a protein. The ribozyme target site within the mRNA molecule is mutated by site-directed mutagenesis such that the amino acid sequence of the translated protein does not change but the mRNA sequence no longer serves as a substrate for the ribozyme. For example, hammerhead ribozymes require an NUX sequence within the target mRNA for cleavage to occur. By changing this sequence to something else, the ribozyme will not cleave the mRNA. This type of ribozyme resistant version of the target RNA can be expressed from a plasmid or integrated into the chromosome of the producing strain and thus render this strain resistant to the toxic effects of the ribozyme.

Another strategy that can protect the producing cell from the toxicity of a toxic agent employs co-expression of a neutralizing agent or antidote. Such co-expression of an antidote or neutralization agent protects the packaging cell from the toxic effects of the encoded toxic agent Such a strategy is particularly useful is the promoter used to express the toxic agent is leaky, and leads to expression of the toxic agent in the producing cell. For example, a packaging strain (*e.g*., bacteria cell) may used to package the a viral vector containing a toxic agent into a bacteriophage phage head. Survival of the packaging cell or optimization of the quantities of vector or phage made by the producing cell may require co-expression of an antidote or neutralization agent in the producing cell. A neutralization agent is any molecule (such as protein, antisense, sense, or other molecule (such as a drug, chemical, etc.)) which counteracts the toxic effects of a toxic agent. By way of illustration, in a specific example, the packaging strain cells contain a bacteriophage P1 plasmid as well as the Transfer plasmid comprising the toxic agent *doc* and a ribosome entry site. In the case that the Transfer plasmid is determined to be toxic to the packaging strain, a third plasmid may be introduced, which encodes an antidote to *doc,* such as the *phd* protein. The additional plasmid with the antidote acts to protect the packaging strain against the toxicity of *doc*.

The improvement in the present invention is that a non-replicative delivery system has an advantage in that once the phage coat has injected the nucleic acid into the targeted bacterium, the expression of the toxic agent or ribozyme will destroy the microbe, as opposed to a lytic infection cycle typical of an intact bacteriophage. Consequently, amplification of the phage coat will not be an issue and it is less likely that the non-replicative phage delivery system will generate an immune response such that subsequent use of the delivery system would be jeopardized. Moreover, if the patient has been exposed to a resistant pathogenic microbe and the therapeutic of the invention is effective and neutralizes the invading microbe, then it is expected that the microbial antigens liberated as a result of the action of the therapeutic, will illicit sufficient humoral immunity and cell-mediated immunity to confer protection against subsequent attacks.

### 5.9. THERAPEUTICS AND PHARMACEUTICAL PREPARATIONS/FORMULATIONS AND METHODS FOR ADMINISTRATION

The present invention further encompasses the use of a toxic agent and/or ribozymes of the present invention for the treatment of disease, viral infection, parasitic infection and microbial infection. The present invention further provides a method of treating a subject having a proliferative disease of a specific tissue by inhibiting cell proliferation in the tissue, comprising administering to the subject a toxic agent and/or ribozyme operably linked to a tissue-specific promoter sequence, which promoter is specific for the diseased tissue, and whereby the ribozyme and/or toxic agent encoded by the nucleic acid is expressed, cell proliferation is inhibited, and the proliferative disease is treated.

The present invention further provides a method of treating a subject having a pathogenic infection or disease, by inhibiting replication of the pathogen, comprising administering to the subject a toxic agent and/or ribozyme operably linked to a pathogen-specific promoter, whereby the ribozyme and/or toxic agent encoded by the nucleic acid is expressed, the pathogen is inhibited from replicating or is killed or rendered less fit, and the infection or disease is treated. The present invention encompasses the toxic agent(s) and/or ribozyme(s) of the present invention in pharmaceutical formulations.

In several embodiments of the invention, toxic agents or ribozymes of the invention are particularly suited as antimicrobial therapeutics. For example, upon nucleic acid hybridization with the target RNA transcript, a ribozyme-RNA complex achieves a catalytic form that acts as a nuclease to cleave the targeted RNAs. Thus, cleavage deprives the invading microorganism of essential cellular processes which then kills or renders it less fit. Additionally, a toxic agent of the invention may also be used as an antimicrobial therapeutic. A toxic agent may be used alone, or in combination with one or more other toxic agents. Thus, delivery of a toxic agent to an invading microorganism, kills or render it less fit. A toxic agent may also be used in combination with one or more ribozymes. Further, a combination of ribozymes and toxic agents may be used as an antimicrobial therapeutic.

The invention provides use of one or more ribozymes and/or toxic agents directed towards essential, housekeeping, or virulence genes of one or a series of candidate microorganisms. Inactivation of essential proteins and virulence determinants render the invading microbes inactive or slow their growth, while at the same time, the essential processes of the host are not significantly affected

A method of delivering a toxic agent or ribozyme to a target (*e.g*., a pathogen) in a subject is provided, comprising a) generating a liposome comprising a promoter and a sequence encoding a toxic agent or ribozyme; and b) delivering the liposome to the subject, whereby the target-specific promoter directs transcription of the toxic agent or ribozyme in the cells of the target. The target can be a pathogen, for example, a baderia, fungus, yeast, parasite, virus or non-viral pathogen.

A method of targeted delivery of a toxic agent or ribozyme to a pathogen in a subject is provided, comprising a) generating a virion comprising non-viral DNA of the invention; b) combining it with a liposome; and b) delivering the liposome containing the virion to the subject, whereby liposome enters the eukaryotic cell and releases the virion, which delivers the DNA to the pathogen, whereby the pathogen-specific promoter directs transcription of the toxic agent or ribozyme in the cells of the pathogen.

A method of treating an infection in a subject is provided, comprising administering to the subject the liposome comprising DNA comprising a target-specific promoter and a sequence encoding a toxic agent or ribozyme, whereby the toxic agent or ribozyme encoded by the DNA is expressed and the infectious agent is killed or weakened. The liposome used in this method can comprise any ribozyme-encoding nucleic acid, or any toxic agent-encoding nucleic acid, particularly those described herein targeted to genes of the pathogen. The infection can be bacterial, fungal, yeast, parasitic, viral or non-viral

Direct *in vivo* gene transfer may be carried out with formulations of DNA trapped in liposomes (Ledley *et al*., 1987), or in proteoliposomes that contain viral envelope receptor proteins (Nicolau *et al*., 1983), and with DNA coupled to a polylysine-glycoprotein carrier complex. In addition, "gene guns" have been used for gene delivery into cells (Australian Patent No. 9068389). Lastly. naked DNA, or DNA associated with liposomes, can be formulated in liquid carrier solutions for injection into interstitial spaces for transfer of DNA into cells (WO90/11092) Asialofetuin-labeled liposomes are known to selectively target hepatocytes via the asialoglycoprotein receptor (Wu *et al*, 1998, Hepatology 27/3:772-8; Hara *et al*, 1996, Biochim, Biophys. Acta 1278/1:51-8; Hara *et al.*, 1995, Gene Therapy 2/10:784-8; Hara *et al*., 1995, Gene 159/2:167-74; each of which is hereby incorporated by reference in its entirety). Asialoglycoprotein receptor-mediated endocytosis has been used as a means to effect gene transfer or transfection using asialofetuin-labeled liposomes charged with a nucleic acid of interest. Other modifications of liposomes, such as those employing sugars or asialoglycans are known.

Accordingly, in one embodiment of the instant invention, asialofetuin-labeled liposomes are used as non-viral vectors for the delivery of the DNAzymes, antisense oligonucleotides or ribozymes of the instant invention. Targeted delivery of the DNAzymes, antisense oligonucleotides or ribozymes of the instant invention to animal liver cells either *in vivo* or *in vitro* is thereby achieved.

*Ex* vivo gene therapy, wherein target cells are removed from the body, transfected or infected with vectors carrying designed nucleic acids of the invention, and re-implanted into the body is also provided. Techniques currently used to transfer DNA *in vitro* into cells include calcium phosphate-DNA precipitation, DEAE-Dextran transfection, electroporation, liposome- mediated DNA transfer, cationic lipid mediated transfection, such as, for example, Lipofectamine™ or transduction with recombinant viral vectors (*see generally* Ausubel *et al.,* 2001, Current Protocols in Molecular Biology, John Wiley & Sons, Inc., which is incorporated by reference in its entirety). These transfection protocols have been used to transfer DNA into a variety of different cell types including epithelial cells (U.S. Pat. No. 4,868,116; Morgan *et al*., 1987), endothelial cells (WO89/05345), hepatocytes (Ledley *et al*., 1987; Wilson *et al.,* 1990) fibroblasts (Rosenberg *et al.,* 1988; U.S. Pat. No. 4,963,489), lymphocytes (U.S. Pat. No. 5,399,346; Blaese *et al*., 1995) and hematopoietic stem cells (Lim *et al.*, 1989*;* U.S. Pat. No. 5,399,346).

Viral vectors are often the most efficient gene therapy delivery system, and a number of recombinant, replication-defective viral vectors are well known in the art to transduce (*i*.*e*, infect) cells both *ex vivo* and *in vivo.* Such vectors include retrovirus, adenovirus, adeno-associated virus, baculovirus and herpesvirus vectors.

Parenteral administration, if used, is generally characterized by injection (intravenous, intradermal, subcutaneous and intramuscular). Injectables can be prepared in conventional forms, either as liquid solutions or suspensions, solid forms suitable for solution of or suspension in liquid prior to injection, or as emulsions. A more recently revised approach for parenteral administration involves use of a slow release or sustained release system such that a constant level of dosage is maintained. See, *e.g.,* U.S. Patent No. 3,610,795, which is incorporated by reference herein. In certain preferred embodiments of the invention administration is parenteral.

The present invention relates to prophylactic administration. For example, many hospital patients or immunocompromised hosts are particularly susceptible to pathogenic infections. Further, many hospital strains of pathogens are resistant to traditional antibiotic treatment, such as Penicillin. The therapeutics of the invention are particularly useful for preventing pathogenic infection or treating infections caused by resistant strains of pathogens.

Suitable carriers for parenteral administration of the substance in a sterile solution or suspension can include sterile saline that can contain additives, such as ethyl oleate or isopropyl myristate, and can be injected, for example, intravenously, as well as into subcutaneous or intramuscular tissues.

Topical administration can be by creams, gels, suppositories, aerosols, sprays, and the like. *Ex vivo* (extracorporeal) delivery can be as typically used in other contexts. In a preferred therapeutic use, the DNAzymes, antisense oligonucleotides, and/or ribozymes of the invention are administered to a subject with one or more papillomavirus infections such as warts of the hands, warts of the feet, warts of the larynx, condylomata acuminata, epidermodysplasia verruciformis, flat cervical warts, cervical intraepithelial neoplasia, or any other infection involving a papillomavirus. It is generally preferred to apply the therapeutic agent in accordance with this invention topically or interlesionally. Other forms of administration, such as transdermal or intramuscular administration may also be useful. Inclusion in ointments, salves, gels, creams, lotions, sprays, inhalants or suppositories is presently believed to be highly useful. The DNAzymes, antisense oligonucleotides, and/or ribozymes of the invention may also be used in prophylaxis. Such may be accomplished, for example, by providing the medicament as a coating in condoms or in a spermicidal formulation for use alone or in conjunction with condoms, diaphragms, and the like. In preferred embodiments of the invention, administration is as a topical treatment. In one embodiment of the invention; treatment of infections associated with bums or open wounds, topical administration may be preferred.

Oral administration is also provided. Suitable carriers for oral administration include one or more substances which can also act as flavoring agents, lubricants, suspending agents, or as protectants. Suitable solid carriers include calcium phosphate, calcium carbonate, magnesium stearate, sugars, starch, gelatin, cellulose, cuboxypolymethylene, or cyclodextrans. Suitable liquid carriers can be water, pyrogen free saline, pharmaceutically accepted oils, or a mixture of any of these. The liquid can also contain other suitable pharmaceutical additions such as buffers, preservatives, flavoring agents, viscosity or osmo-regulators, stabilizers or suspending agents. Examples of suitable liquid carriers include water with or without various additives, including carboxypolymethylene as a pH-regulated geL

The therapeutics of the invention can be administered to a subject in amounts sufficient to produce an antibiotic effect or to inhibit or reduce the activity of the target pathogen. Optimal dosages used will vary according to the individual, on the basis of age, size, weight, condition, etc., as well as the particular modulating effect being induced. One skilled in the art will realize that dosages are best optimized by the practicing physician and methods determining dosage are described, for example, in Remington's Pharmaceutical Sciences (Martin, E.W., ed., Remington's Pharmaceutical Sciences, latest edition, Mack Publishing Co., Easton, Pennsylvania). Treatment can be at intervals and can be continued for an indefinite period of time, as indicated by monitoring of the signs, symptoms and clinical parameters associated with a particular infection. The parameters associated with infection are well known for many pathogens and can be routinely assessed during the course treatment.

In one preferred embodiment, the present invention provides compositions containing one or more nucleic acids of the invention that may be used to treat viral infections. While individual needs vary, a determination of optimal ranges of effective amounts of each component in the composition is within the purview of the skilled artisan. Typical dosages comprise 0.001 to 100 mg/kgbody weight. The preferred dosages comprise 0.1 to 10 mg/kg body weight The most preferred dosages comprise 0.1 to 1 mg/kg body weight. Preferred topical applications will be in a volume sufficient to cover the papilloma, or sufficient to cover any cervical lesions identified by culposcopy. Topically-applied DNAzmes or antisense oligonucleotides are contemplated to be in the range of 1 µg to 250 µg, or preferably in the range of 50 µg to 100 µg, per application. For example, a typical HPV topical application is 50 µg in 50 µl. For HBV reagents of the invention, total dosage is contemplated to be in the range of 500 µg to 1000 µg. A determination of optimum dosage ranges will include consideration of toxicity studies and is well within the purview of the skilled artisan.

In addition to the pharmacologically active agent, the compositions of the invention may contain pharmaceutically-acceptable carriers comprising excipients and auxiliaries which facilitate processing of the active compounds into preparations which can be used for delivery to the site of action.

Suitable formulations for parenteral administration include aqueous solutions of the active agents in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers. Liposomes can also be used to encapsulate an agent for delivery into the cell.

As described above, pharmaceutical compositions for systemic administration according to the invention may be formulated for enteral, parenteral or topical administration. Indeed, all three types of formulations may be used simultaneously to achieve systemic administration of the active mgtedient.

Suitable formulations for oral administration include hard or soft gelatin capsules, pills, tablets, including coated tablets, elixirs, suspensions, syrups or inhalations and controlled release forms thereof.

### 6. EXAMPLE: CONSTRUCTION & CHARACTERIZATION OF PROMOTERS

Classical bacterial inducible promoters are renowned for their inability to tightly control transcription, and a significant level of background expression is characteristically observed.

### The Leashi Promoter

The present invention provides bacterial promoters that allow for tight regulation of transcription and enhanced expression. A novel promoter called LEASHI has been constructed from three elements (see Figure 1A). The first element, termed RIP was a combination of two consensus sites at -10(TATAAT) and -35(TTGACA) located with respect to transcription initiation. The second element was based on the 1acI repressor binding sequence (termed lac operator sequence) which was placed between the -10 and -35 consensus sites. Placement of the lac operator between the -10 and -35 sites, more effectively blocked RNA polymerase binding to the promoter, thus enhancing transcriptional control from the promoter. The promoter was designed such that it was 'switched on' following the addition of isopropyl B-D-thiogalacto pyranoside, which binds and subsequently titrates out the repressor protein. RNA polymerase can then bind to the promoter and transcription can proceed.

The third element of the LEASHI promoter was the UP element. The UP element was an adenine/thymine rich sequence which was placed immediately upstream of the -35 element Addition of the UP element, further increased expression from this promoter.
The LEASHI promoter sequence: (SEQ ID NO:1)

5' GATCCTCAGAAAATTATTTTAAATTTCCAATTGACATT
GTGAGCGGATAACAATATAATGTGTGGA3'

A significant advantage of the LEASHI promoter of the present invention is that it alleviates the high levels of background commonly observed in inducible promoters.
A limiting factor leading to high background levels of transcription when a promoter of interest is located on a high-copy number plasmid, is due to the lack of repressor molecules available to bind to the promoters. The present invention overcomes this problem by using a lacI expression plasmid and secondly, by placement of the lac operator between the -35 and -10 consensus elements which more effectively blocks transcription during normal conditions. Furthermore, the UP element placed immediately upstream of the -35 region enhanced transcription from the promoter.

The LEASH1 promoter (Figure 1B) was designed as a lacI-regulated promoter which a broad spectrum promoter activity in a wide variety of bacteria. The IPTG inducible LEASH1 functions in *Escherichia coli* and is tightly regulated. It is active in both Gram-negative and Gram-positive bacteria.

As described herein, ribozymes of the invention have been operably linked to the LEASHI promoter. In another specific embodiment of the invention, a toxic agent of the invention was operably linked to a LEASHI promoter.

### The Modified rrnB Promoter

A novel promoter called the modified nnB has been constructed (see Figure 1C). Modified rmB promoter sequence: (SEQ ID NO:2)

5'AGAAAGCAAAAATAAATGCTTGACACTGTAGCGGGAAGGCGTATA
ATGGAATTGTGAGCGGATAACAATTCACA 3'

### The Anr, Arc, and Proc Promoters

The *Anr* (Figure 1D),*proC* (Figure 1E) and *Arc* (Figure 1F) promoters are species-specific. Both anr and proC are transcriptionally off *in E. coli* and on in *Pseudomonas aeruginosa.* These promoters provide the advantage of allowing controlled expression of the toxic agents in the pathogen (Pseudomonas), while allowing the packaging strain *(E. coli*)to be protected from the toxic actions of the therapeutic. Such promoters are particularly useful to facilitate manufacturing of the delivery vehicle. Such promoters also enable bacterial specific targeting of the gene therapeutic in the patient.
*Pseudomonas aeruginosa* 'specific' promoters (5' to 3')

### ANR promoter (SEQ ID NO:3)

5' ACTCGCGGATCATCTTCACCATCGGCCGCAACTCCTGCGGGATATCCTCGTC

CTCCTCCTCCACCGGCACCCCCATGGTAGCGGCCAGCTCGCGCCCTGCCTGGGA
AAGCTGTACATGCTGATCGGCGGCGTCGGTGCCGGCGGCCGGGTCTTCCGCCTG
CTCGGCGGTGCCGGTCCGTGCGGCCTTGGCGTCCGCGGCGGCGCGCGATGAGGG
CGGCACCTGGGTGGTGATCCAGCCACTGAGGGTCAACATTCCAGTCACTCCGGG
AAAAATGGAATTCTTCCATTGGATCGGCCCACGCGTCGCGAACTTGAGCCCCCT
TTTCGTCGCCCCTTGACAGGGTGCGACAGGTAGTCGCAGTTGTTTGACGCAAGT
CACTGATTGGAAACGCCATCGGCCTGTCAGAAATGGTCGTTGCCAGACCTATGG
CTGGCACCCGCATCGCGGCTGCGTTACCCTTACTCCTGTTGTGCCTTTAACCTAG
CAAGGAC

### ProCpromoter (SEQ ID NO:4)

5' AATTCCTCGAAGTCCTTGCGCTGCTTGTCGTTCATGATGTCGTAGATCAGCGC
ATGCACCTGCTTGTGTTCCAGCGGTGGCAGGTTGATCCGGCGTACATCGCCATC
CACCCGGATCATGGGTGGCAGGCCGGCGGAGAGGTGCAGGTCCGAAGCGCCCT
GTTTGGCACTGAAGGCGAGCAGCTCGGTAATATCCATGGGACTCCCCAATTACA
AGCAAGCAGGTAGAATGCCGCCAAAGCCGCCGTCTCGGACAAGGAAAACACCG
GATGAGCCAGGGTGCTTCCAGGACACGCGTGGTGTCCTGCGCCAGACGCGGAA
CCTCGACACTGGAACAGGAAGATGGCCATCGAGGCCGGCGGTTTCGAGGGCGT
CGAGCCGACGCCGACCGCACTTCCATAGGGCGCAGGTAATGTCCACGATAGCA
GAGAATATTGCAAAGGTTGCCGCGCGCATCCGTGAGGCAGCGCAAGCTGCGGG
GCGCGATCCGGCCACGGTCGGCCTGCTCGCCGTGAGCAAGACCAAGCCCGCCG
CCGCGGTGCGCGAGGCGCACGCCGCCGGCCTTCGCGACTTCGGCGAAAACTACC
TGCAGGAGGCCCTCGGCAAGCAGGCCGAACTGGCCGACCTGCCCTTGAACTGG
CACTTCATCGGCCCCATCCAGTCGAACAAGACGCGGCCCATCGCCGAGCATTTC
CAGTGGGTGCACTCGGTGGACCGGTTGAAGATCGCGCAGCGCCTGTCGGAGCA
ACGCCCGGCCGGGCTGCCGCCCCTGAATGTCTGCCTGCAGGTCAACGTCAGCGG
CGAAGCCAGCAAGTCCGGCTGCGCCCCCGAGGACCTGCCGGCCCTGGCCGAGG
CCGTGAAGCAACTGCCCAACCTCCGATTGCGTGGCCTGATGGCCATCCCCGAAC
CCACCGCCGAACGCGCCGCGCAACACGCCGCGTTCGCCCGCCTGCGCGAACTG
CTGCTGGACCTGAACCTTGGCCTGGACACCCTGTCCATGGGCATGAGCGACGAC
CTCGAGGCAGCCATCGGCGAAGGTGCGACCTGGGTCCGCATCGGTACCGCCCTG
TTCGGCGCCCGCGACTACGGCGCGCCGGCTTCTTGAATGAATCCC

### ARC promoter (SEQ 1D NO:5)

5' CTA GAG CTA TTG ATG TGG ATC AAC ATT GTC CAC TAG CCG CTG
CCG CCT AAT CTC CAG AAT TGT GAG

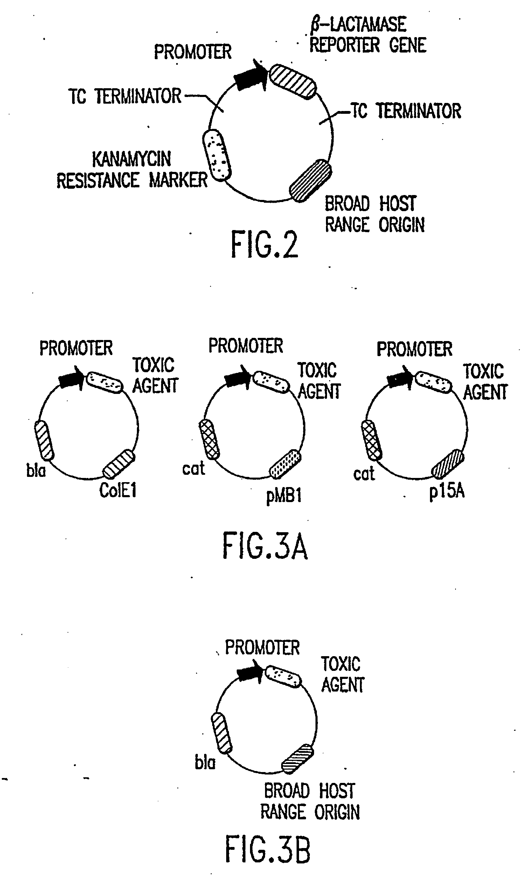

*Anr, Arc,* and *proC* promoters, which were expressed preferentially in *P*. *aeruginosa,* have been isolated and shown to express a toxic agent specifically in this pathogenic bacterium (See Tables 1 and 2 and Figure 2). Specifically, as shown in Table 1, promoters were cloned upstream of the β-lactamase reporter gene in a cassette flanked by multiple transcription terminators. Constructs were transformed into *E*. *coli* or P. *aeruginosa* and plated onto agar containing different amounts of carbenicillin. Three repeat evaluations gave the same result.

**Table 1. Evaluation of Promoters Utilizing β-Lactamase as a Reporter Gene**

| | E. coli | | P. aeruginosa |
|---|---|---|---|
| Construct | 25 µg/ml carbenicillin | 50 µg/ml Kanamycin | 5 mg/ml carbenicillin |
| emptyvector (no promoter) | - | + | - |
| UPRIP β-lactamase | + | + | + |
| *proC* **β**-lactamase | - | + | + |
| anr β-lactamase | - | + | + |

As shown in Table 2, the *chpBK* gene was cloned in both orientations under the control of P. *aeruginosa* promoters *proC* and *anr.* Equal quantities of DNA (500 ng) were transformed into *E. coli* and P. *aeruginosa* and plated on agar. Mock transformations were also performed with 'no DNA'. + indicates greater than 100 colonies, - indicates no detectable colonies. Parentheses indicate orientation of the *chpBK* gene in relation to the promoter. Experiments were repeated at least two times with the same result. Importantly, plasmids using *proC* and *anr* to regulate *chpBK* expression did not induce cell death in *E. coli* indicating lack of transcriptional activation function.

**Table 2. Evaluation of Promoters that are Expressed Preferentially in P. aeruginosa**

| Construct | *E. coli* | *P. aeruginosa* |
|---|---|---|
| patent vector | + | + |
| anr *chpBK* (positive) | + | - |
| anr *chpBK* (negative) | + | + |
| proC *chpBK* (positive) | + | - |
| proC *chpBK* (negative) | + | + |

The development of species-specific promoters is particularly important in aspects of the invention in which it is desired to allow indigenous commensal bacteria to be protected from the toxic agents of the invention while targeting the pathogenic *P. aeruginosa.*

### TSST-1 Promoter

The environmentally regulated staphylococcus-specific promoter TSST-1 has been obtained and a transfer plasmid utilizing this promoter is used to express *doc* or other toxic agents. A staphylococcus specific phage capable of delivering the transfer plasmid into S. *aureus* strains is used to specifically target the Staphylococcal pathogen.
TSST-I promoter (SEQ ID NO:6) (GenBank® accession number U9368 8, see also Lindsay,J.A., et al.,1998, "The gene for toxic shock toxin is carried by a family of mobile pathogenicity islands in Staphylococcus aureus" MoL Microbiol. 29 (2), 527-543):

1 ttatttagca ggaataatta gccagattat cgagggagtt ccagggcaatccaaacattg
61 ttatatatgc atttataaaa ttttcaagat aatttattat tcataccccttgcccttttgtt
121 tcaaaattat gcccttttttt tgcccttgga aacaaccaca ctcctaaattaataggtggt
181 gtggtttgat catttataat ataacataaa aacaaccacc cagtaactagtatgagtggc
241 gtagcgacta taacaactct atgttatcaa gatatatgta tatgagtgatgacaaggaag
301 atgtctcctg tgagaccaac agccagatat atggcctctt gccgggctatatagttcact
361 cctactatat acacatgtaa ttataacata aaaaaataga caagtaccgaagtacctgcc
421 taaataacaa caagattaac atgtgaataa tggaaataaa aagtcagcccgaaggctaac
481 ttacgaatag atgaaaattt gaacacattg ctgtgtctaa aatgattatagcataaataa

541 cgaatatttc cagctcgaaa ttaatatatt gtaataataa tattttatatcttgttaat
601 aattatttaa ttgatttaca taaataataa ttgtaaaatt aatttgtaatcgattgcaaa
661 taagttatag gagaaaataa aatgaataaa aaactattaa caaaaacattgatagcaagt
721 gctttagttt taacaacagt aggttcaggt tttcattctt cttcaaattataatggtatt
781 aataacgttg aaaaagctga gcaaacgaca gataacgcat tgtggaaaaatgtaagagac
841 gctttaaaag acgcgaatat tatcgataaa acagataatg aaaatgtcaaggttacgtat
901 aaaatagaaa atggtggaga aaataccata gaaggaacag ttaatttagaaaatattagt
961 acttcaaaca atcctaaaat aaaccctcaa aatgttacaa aaattaatataactagaaaa
1021 aatccgaact accctaatat tgatgctaat aatacatgga aaaaattaccagaaaaattg
1081 aaagaaaaaa atatagtgga acaacggcga caatgtttca atcttaagtacagaccctaa
1141 agatgagact gtattcggta aagtaggaga agataaatca aacgtaagcaatagatacat
1201 caatcctaaa gatataaatg aattcaaatc actaaaaata ctttttccgaggcagatta
1261 ctcctgcctc tttctttgaa cagtgatatc ttctgatcta tgtaacactcaattacttca
1321 gattctttac ctttaacttc ctttaattca tttctctcta tctcctcaaaaagttgtgct
1381 ttttgatttg tgattggagt tgggcgtttt ttcatcgcgt tgtttcaattcctttttaag
1441 gtattctaat tctcttctag tcatatcaat tgttttttta cttctcacctttagtgaaat
1501 actcttatcc tttctcttct tgcgttaatg ttgctaatta gtataaaatacatgcgccca
1561 tatattccaa tggtaggaca tttaattctg gattttcagc tattttcataaatctattat
1621 ctgataattt gcttaatcca attttcaagc catagcctaa attccccatccactaagtca
1681 ttttgtttca tatggtttta atctacggcc aatctcaaag atagattgaccagcgatgtt
1741 taaagtcata tttcacggat ccacatttac gataaacata tctagttacacaatattatc
1801 ccttactgca acacaggacg tttctcagcg taaaaaacac cactagaaagtgactttaaa
1861 gaatataact aattcaaact tatattaatt aatattcttt aaatgaccactcacactttg
1921 tttttgcta tttgtaactt taaaatgttg tttgaaatct atatttttttgatatagctc
1981 cctatgtaac aaacaatttt taattaatat atatttaaac aagtcaatttagagatcggt
2041 taattcgatt catttaaata atatttatac attctatatg taaacgtttacacatttgaa
2101 gtaaggagaa ttaaaaatga

### 7. EXAMPLE: EFFECTS OF TOXIC AGENTS ON BACTERIAL GROWTH

In order to demonstrate the methods of the invention, the inventors have expressed and targeted several toxic agents to bacterial pathogens. Toxic agents were selected based on their ability to inhibit the growth of a pathogen or diseased cell or cause lethality in a pathogen or diseased cell. The examples hereinbelow illustrate toxic agents of several naturally occurring phage, plasmid and chromosomally encoded toxic proteins and demonstrated their effectiveness as antimicrobial therapeutic agents.

Specifically, several naturally occurring phage, plasmid and chromosomally encoded toxic proteins have been identified and have demonstrated effective as antimicrobial therapeutic agents, including but not limited to *SecA, 16S RNA, dicF, sof, dicF* antisense, 16S antisense, toxic proteins of the toxin/antidote pairs *doc*/*Phd, gef*/*Sof, chpBK*/*ChpB1,* or *kicB*/*KicA.*

To illustrate that a toxic agent may be a toxic gene product of an addiction system toxin, a toxic gene product of a chromosomally-encoded toxin, or antisense molecule, nucleic acids encoding *doc*, *gef, chpBK kicB or DicF1* were engineered into Transfer plasmids for use in the P1 bacteriophage delivery system. Plasmid construction was performed by standard methods known in the art.

As shown in Figure 3, expression vectors for the cloning of toxic agents were engineered. Specifically, genes encoding the toxic proteins *chpBK, kicB, doc* and *gef* under the control of the lacI-regulated promoter, were cloned into an *E. coli* vector containing replication origin ColE1 (300-500 copies per cell), pNB1 (15-20 copies per cell) or p15A (10-12 copies per cell) and the selectable marker CAT (chloramphenicol acetyltransferase). However, any selectable marker known in the art may be used (*e.g.*, bla, ampicillin resistance). Toxic agents were cloned in an *E. coli* strain that overexpressed the lacI repressor protein from a lacI expression plasmid. Genes encoding the toxic proteins *chpBK*, *kicB, doc* and *gef* under the control of lacI- regulated promoter, were obtained by PCR and cloned into an *E. coli* shuttle vector. Lethal agents were cloned in an *E.* coli strain that overexpressed the lacI repressor protein form a lacI expression plasmid.

Plasmids containing the toxic agents *doc or gef* were also been engineered such that a ribosome entry site has been constructed upstream of the nucleic acids encoding the toxic agent in order to increase the levels of translation of *doc or gef.* Plasmids harboring a toxic agent was called a Transfer plasmid. The Transfer plasmid was constructed such that it contained 1) an origin of replication 2) selectable marker 3) P1 PAC site, and PAC ABC genes 4) P1 lytic replicon 5) nucleic acids encoding the toxic agent (*e.g., doc, gef,* or *DicF1*)*.*

Specifically, Transfer plasmids were constructed based on pBluescript (ColB1 origin) and pBBR 122 (broad host range origin) parent vectors. The nucleic acids encoding the toxic agents *doc* or *gef* were cloned into the broad host range transfer plasmid. The nucleic acid encoding *dicF* was cloned into the Co1E1 transfer plasmid. The structure of each vector is available. Both *doc* and *gef* were placed under *lacI* regulated promoter. The Transfer plasmids were designed to undergo rolling circle replication during the phage lytic cycle.

A packaging strain *(e.g.,* bacteria cell) was then used to package the Transfer plasmid containing the nucleic acid encoding the toxic agent into a bacteriophage phage head. The packaging strain for each of the three toxic agents contained the P1 bacteriophage prophage as well as the Transfer plasmid containing the nucleic acids encoding the toxic agent. In some cases, the packaging strain also contained a third plasmid, if necessary, which encoded additional antidote protein which acted to protect the packaging strain against the toxicity of the toxic agent or the third plasmid encoded additional repressor protein to switch off the promoter of the Transfer plasmid.

Thus, the packaging strain (P1 lysogen) was used to package the transfer plasmid containing the toxic agent *(e.g., doc, gef*, or *DicF1*) into phage heads or virions. Phage lysates of the packaging strain contained the infectious bacteriophage virions, and were used to infect bacterial targets in the following manner.

The P1 lysogen (P1cm C1.100) carrying the transfer plasmid with the toxic agent *(doc* or *gef* or *DicF1)* was grown at 30°C in LB, 10 mM MgSO₄, 5 mM CaCl₂, 12.5 µg/ml chloramphenicol until A₄₅₀ reached 0.8 at which time the culture was shifted to a 42°C water bath and aerated vigorously for 1 h. Chloroform was added and incubation continued for an additional 20 min at 37 °C. The phage stock was clarified by centrifugation at 4,000 g for 20 min. DNase (1 µg/ml and RNase (10 µg/ml) were added and after incubation at 37°C 30 min the phage were centrifuged at 4,000 g 20 min. Phage particles were precipitated from the phage stocks by adding NaCl to 1 M and polyethylene glycol 6000 to 10% (w/v). After incubation on ice for 2 h the phage were pelleted by centrifugation at 11,000 g for 15 min. The pellet was carefully dissolved in 50 mM Tris.CI pH 7.5,10 mM MgSO₄, 5 mM CaCl₂, 0.01% Gelatin. Polyethylene glycol was removed by extraction's with chloroform.

The phage lysates were then used to infect a selected pathogen (*e.g.*, *E*. *coli*)*.* Target cells (10⁵ CFU/ml, treated with 10 mM MgSO₄, 5 mM CaCl₂) were infected at various M.O.I s (0.1,1,10,100) with each of the above phage lysate. Following 30 min infection at 30°C. Cell death was assessed by scoring the plates for the total number of colony forming units.

Both types of Transfer plasmids (ColE1 and broad host range based) were transferred by the P1 delivery system to various *E*. *coli* strains *in vitro.* The P1 system was also used to deliver the broad host range transfer plasmid to *P*. *aeruginosa in vitro.* The CoIE1 transfer plasmid was successfully transferred to *E*. *coli in vivo* and the broad host range transfer plasmid has been delivered in *vivo* to both P. *aeruginosa* and *E*. *coli.*

Results indicated that the infection of the bacterial cells with the phage lysates comprising the infectious virions containing a toxic agent was capable of killing the infected bacterial cells. Further, bacterial cell death was seen to be dose dependent such that higher M.O.I lead to increased cell death. Thus, the methods and compositions of the invention are useful as antimicrobial agents for treating pathogenic infections.

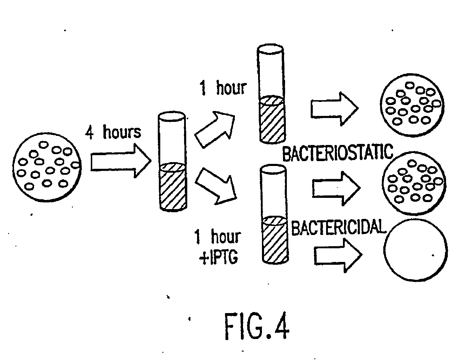

Lethality testing of the toxic agents and has revealed that *doc, gef, chpBK* and *kicB* are all bactericidal to *E. coli.* (See Figure 4). Specifically, colonies were grown in liquid culture under conditions where the expression of the toxic proteins was repressed. following expression of the protein by induction with IPTG for 1 hour, cultures were plated out overnight onto agar lacking IPTG. The absence of colonies indicates the protein is lethal (see also Table 4 for Results). Constructs were transformed into *E*. *coli* and plated onto agar with or without 1mM IPTG. Equal quantities of DNA (500 ng) were also transformed into *P. aeruginosa, S. aureus* and *E. faecalis.* Mock transformations were also performed with 'no DNA.' + indicates greater than 100 colonies, - indicates no detectable colonies. Experiments were regeated at least two times with the same result. All agents were lethal to *E.* coli but only *doc* was toxic in all four.

| **Table 4. Assessment of the Toxic Proteins in *E. coli, P. aeruginosa, S. aureus*and *E*. *faecalis* using the Broad Host Range Plasmid.** | | | | | |
|---|---|---|---|---|---|
| Construct | *E. coli* | *E. coli* +IPTG | *P. aeruginosa* | *S. aureus* | *E. faecalis* |
| Parent vector | + | + | + | + | + |
| *doc* | + | - | - | - | - |
| *gef* | + | - | + | + | + |
| *chpBK* | + | - | - | + | + |
| *kicB* | + | - | + | + | + |

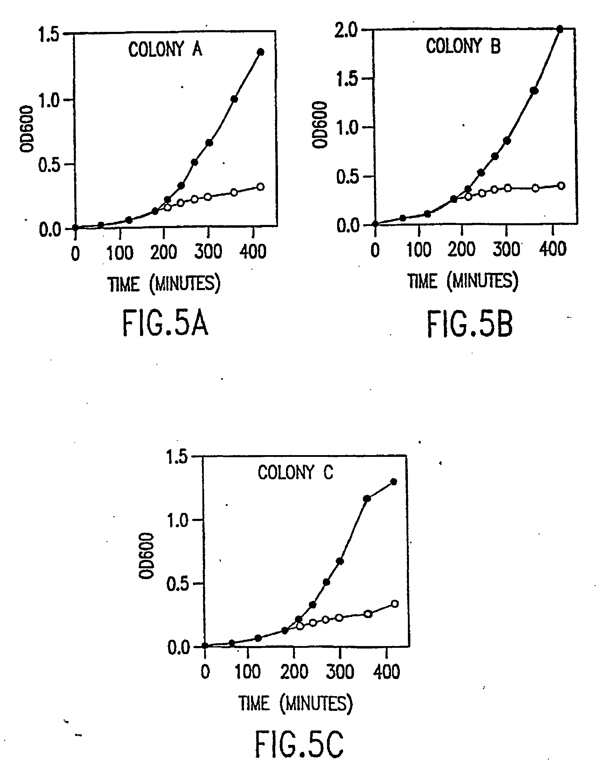

As shown in Figure 5, the growth ofE. *coli* harboring a *doc* expression plasmid was demonstrated to be inhibited when the expression of *doc* was induced by IPTG. Specifically, cells were grown overnight in LB at 32°C, diluted 1:100 into fresh IB medium and incubated at 32°C for 180 min. The culture was then divided equally and incubated at 32° in the absence (O) or presence of 2 mM IPTG (O) which results in the expression of the lethal agent *doc*. Growth was calculated by spectrophotometric measurements with 1 ml samples at OD600.

Unlike traditional antibiotics which merely slow the growth of the bacteria, lethality testing of *doc* has shown that 99% cell death was achieved when cells were induced with IPTG for 20 min. A significant reduction (92% cell death) was also demonstrated when the cells were under no selective pressure to maintain the *doc* expression plasmid. Thus, the rapid killing of bacteria reduces the potential for selective pressure to give rise to resistant strains, which is important in eradicating multidrug resistant bacteria.

### LOW RESISTANCE TO DOC

In order to examine the frequency of resistance to *doc*, resistant mutants were isolated. The rationale was to select for spontaneous mutations and no mutagens were used. Following prolonged exposure to sublethal concentrations of 40 *doc* resistant *E*. *coli* clones were isolated. DNA isolated from these clones were tested by transformation to a *doc-*sensitive cell, however the presence of 1PTG did not induce cell killing. This indicates that resistance is due to mutations or recombination events in the *doc* expression plasmid, suggesting that a chromosomal mutation of the *doc* target occurs at a very low frequency.

### TOXICITY TO P. AERUGINOSA

*Doc* and *chpBK* have been demonstrated to be toxic to *P. aeruginosa.* Of particular note is that *doc* had a broad-spectrum activity in both Gram-negative (*E. coli* and *P. aeruginosa*) and Gram-positive (S. *aureus* and *E.faecalis*) bacteria (see Table 4, above). As seen in Table 4, the toxic agent *doc* killed all species of bacteria tested. *Doc, gef, chpBK* and *kicB* were all able to kill *E. coli. chpBK* killed *E. coli* and P. *aeruginosa.*

### DEVELOPMENT OF A BACTERIOPHAGE TOXIC AGENT DELIVERY SYSTEM

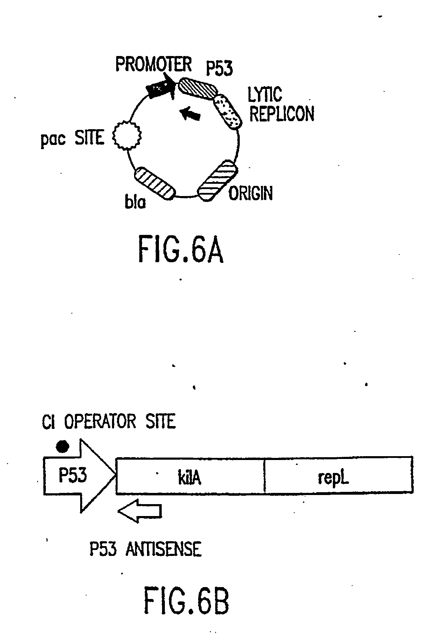

A toxic agent delivery system has been achieved for the use of a bacteriophage P1 system to package and deliver a Transfer plasmid (See Figure 6A and 6B) to *E. coli* and *P*. *aeruginosa.* Figure 6A depicts the Transfer plasmid containing the essential signals for packaging (a *pac* site and a lytic replicon under the control of the P1 P53 promoter), a selectable marker for detection (bla, ampicillin) and ColE1 origin of replication in *E. Coli.* Figure 6B depicts the lytic replicon which comprises the C1 repressor-controlled P53 promoter antisense and genes *kilA* and *rep*L*.* The *kil*A gene contains a 52% in frame deletion. P53 antisense is implicated in the stability of the P1 replicon. The methods of the invention are exemplified herein by two transfer plasmids capable of being efficiently packaged in P1 virions for delivery to pathogenic Gram-negative bacteria. Importantly, the delivery system is not under the constraints of superinfection exclusion (Figure 7). In order to demonstrate delivery efficiency of the Transfer plasmid by the P1 Delivery System to B. *coli,* the following assay was performed. The *E*. *coli* P1 Cm clts 100 lysogen canying the transfer plasmid was induced by thermal induction to produce phage particles. Phage lysates were created with DNase and RNase and precipitated particles were resuspended in 50 mM Tris.Cl pH 7.5, 10mM MgSO₄, 5 mM CaCl₂, 0.11% gelatin, E. *coli* C600 and *E. coli* P1 C600 target cells (10⁵ CFU/ml, treated with 10mM MgSO₄, 5mM CaCl₂) were infected with each of the phage lysates. Following 30 min incubation at 30°C, infections were plated onto selection plates and antibiotic resistant colonies were scored. Values indicate number of antibiotic resistant colonies ± standard error, n=6.

Further, the phage-based delivery system is not blocked by a resident phage, such as P1 and lambda, or by compatible plasmids. This is important because analyses of environmental samples suggests that up to 40% of P. *aeruginosa* strains in the natural ecosystems (lake water, sediment, soil and sewage) contain DNA sequences homologous to phage genomes. The bacteriophage based system is useful to transfer genetic information *in* vivo by delivery of a transfer plasmid expressing an antibiotic marker to *E. coli* and P. *aeruginosa* in a mouse peritonitis model of infection. Plasmid transfer was confirmed by restriction analysis and sequencing of the plasmid DNA re-isolated from bacteria recovered from the intraperitoneal space. Demonstration of transfer in *vivo* has also been obtained.

### DEVELOP BACTERIOPHAGE P1 KNOCKOUTS ABLE TO PACKAGE TRANSFER PLASMID DNA BUT UNABLE TO INCORPORATE P1 DNA

One consideration of using unmodified phage as a delivery vehicle is the potential risk of lysogenic conversion. In order to develop a bacteriophage delivery vector which is capable of delivering a Transfer plasmid, to a target bacteria, but which is unable to deliver its own DNA to a target bacteria, a modified P1 phage was developed.

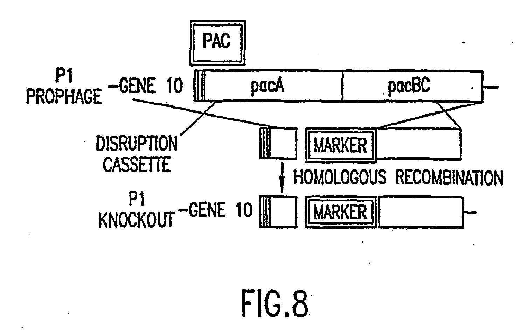

As shown in Figure 8, the P1 prophage DNA has been modified to generate a pac site knockout. The disruption cassette contain a nutritional or antibiotic marker flanked by sequences homologous to the P1 prophage. the linear fragment was protected from exonuclease attach by the incorporation of phosphorothioate groups. A double crossover event between the in vitro-altered sequence and the P1 prophage resulted in deletion of the pac site and acquisition of the selectable marker. The function of this knockout serves to inhibit the ability of the p1 bacteriophage to package or transfer its own DNA to a target bacteria.

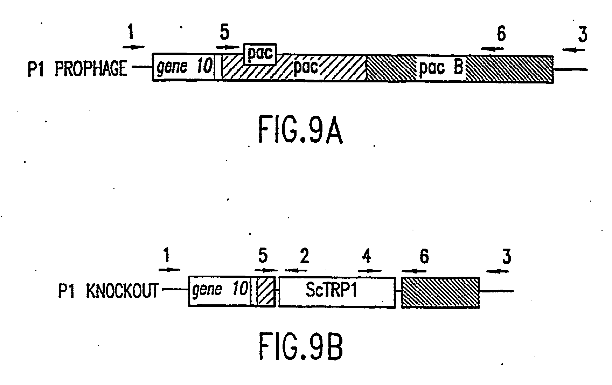

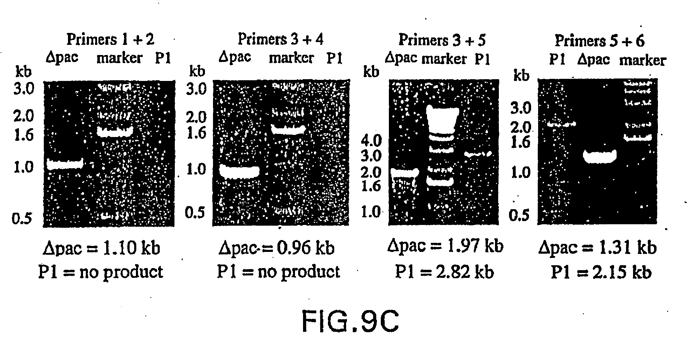

As shown in Figure 9, the modified P1 was unable to transfer the chloramphenicol marker associated with its genome, suggesting that phage particles produced from the pac mutants lack phage DNA. The top panel of Figure 9 shows the physical map of the P1 prophage and predicted P1 knockout following integration of the disruption cassette at the pac site. Arrows indicate location of the PCR primers used to verify the replacement of the P1 pac site with the S. cerevisiae TRP1 gene. The gels shown the products of the PCRs using P1 specific primers (1, 3, 5 and 6) and disruption cassette specific primers (2 and 4) to detect either the wild type P1 prophage r the P1 knockout. Primers 1 and 3 do not bind within the P1 sequences in the disruption cassette therefore PCR with primer 1+2 and 3+4 only detect a specific integration event which results in replacement of the pac site with the *S. cerevisiae* TRP1 gene.

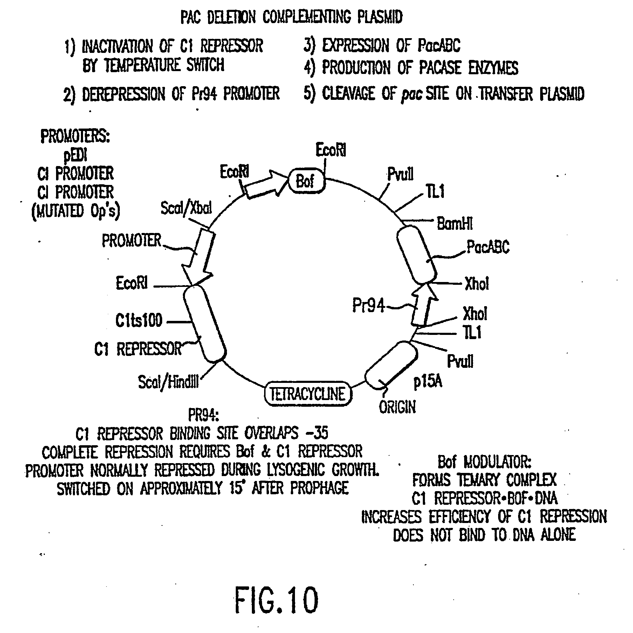

As a consequence of the *pac* site lying within the *pacABC* operon, the modified phage needed to be complemented in *trans* with the pacase enzyme. Construction of the *pacABC* complementing plasmid is shown in (Figure 10 and Table 5).

**Table 5 Construction of the pacABC complementing plasmid.**

| |
|---|
| c1-pBSK |
| clts100-pBSK |
| Bof-pACYC184 |
| pBDI-clts100-pBSI |
| C1pro-clts100-pBSK |
| clmut-clts100-pBSK |
| Bof-pEDI clts100-pACYC184 |
| Bof-C1mut clts100-pACTC184 |
| Bof-C1pro clts100-pACYC184 |
| TpacABCT-Bof-pEDI clts100-pACYC184 |
| TpacABCT-Bof-Clpro clts100-pACYC184 |
| Tpr94pacABCT Bof pEDI clts100-pACYC184 |
| cl, cl repressor, clts100, C1 repressor with temperature sensitive cl. 100 mutation; Bof, modulator of C1 repressor-, pEDI, promoter, elpro, promoter with C1 operator sites Op99a and Op99b; c1mut, promoter Clpro with mutated operator sites; pacABC, genes encoding pacase enzyme; T, transcirption terminatore; Pr94, promoter with operator site Op94; pBSK, E. coli vector pBluescript, pSACYC184, *E. coli* cloning vector. |

P1 *pac*ABC were expressed from an early promoter Pr94. Two phage encoded proteins, C1 repressor and Bof modulator, were used to regulate expression from the Pr94 promoter. Although Bof alone does not bind to DNA, together with C1 it increased the efficiency of the repressor-operator interaction. The c1 repressor has the c1ts 100 mutation and was therefore be temperature sensitive. This allowed the coordinated expression during the phage lytic cycle to the *pacABC* genes.

The complementation plasmid allowed P1 *pac* mutant to package the Transfer plasmid but not its own viral DNA. Complementation with the pacase enzymes did allow the P1 *pac* mutants to package the transfer plasmid, however a portion of the phage particles produced from the *pac* mutants contained P1 viral DNA. Analysis of the chloramphenicol resistant transductants indicated that the majority were unable to produce a second round of multiplication, suggesting that they were defective lysogens. The *pac* mutants appeared to have acquired a *pac* site, by recombination with the complementing plasmid, thereby enabling the mutants to package and deliver its own viral DNA.

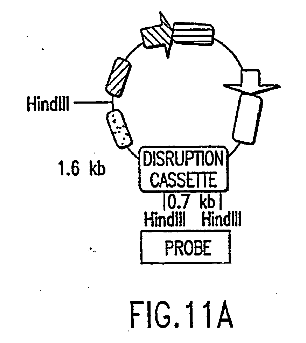

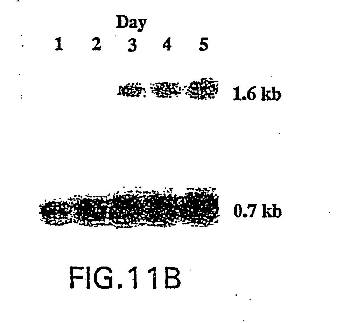

Southern blot analysis verified that the *pacABC* genes on the complementing plasmid had been replaced with the *ScTRP1* disrupted copy (Figure 11). Specifically, the P1 mutant lysogens harboring the Transfer plasmid and pac ABC complementing plasmid were growth at 32C and diluted 1:100 into fresh medium every 16 hours. DAN was extracted on day 1, 2, 3, 4, and 5, digested with HindIII and probed with a ScTRP 1 EcoR1-BamH1 fragment under high stringency conditions.

In order to prevent reconstruction of functional (pacABC pac enzyme by recombination events between the Transfer plasmid and the modified P1 phage genome, silent mutations were introduced into the complementing plasmid as shown in Figure 12. Silent mutations in the complementing plasmid *pac* sits lead to a defedive *pac* site even if recombination occurred, and ensured that a defective pac site was be introduced into the P1 pac knockout (Figure 12). The 162 bp pac site is sufficient to promote pac cleavage and P1 packaging. The positions of the hexanucleotide elements with the HEX4 and HBX3 domains are shown by open boxes. the IHF binding site, consensus sequence 5'-AATCAANNANTTA, is indicated. Regulation of pac cleavage involves adenine methylation at 5' - GATC sites. Silent mutations introduced into the pac site are indicated by lower case letters.

### IN VIVO DELIVERY OF THERAPEUTIC AGENTS BY P1 VIRIONS

All five animal models listed in Table 6 are exemplified herein. LD50's have been established in the peritonitis models for *E*. *coli* and *P*. *aeruginosa,* and the doses required are high (10⁷-10⁸ bacterial cells/animal). Further, a cystic fibrosis model of pseudomonas infection in mice is used to demonstrate efficacy of the toxic agents and methods of the invention for treatment of opportunistic hmg infection characteristic of this disease.

| **Table 6. Animal Models for Prokaryotic Gene Therapy** | |
|---|---|
| **Animal Model** | **LD50** |
| *E. coli* peritonitis | 3x10⁸ cfu |
| Pseudomonas peritonitis | 1x10⁷ cfu |
| Pseudomonas-bum in mice | < 10 cfu |
| Pseudomonas-burn in rats | 1 x 10⁸ cfu |
| Pseudomonas in neutopenic mice | 2 x 10³ cfu |

### PERITONITIS MODELS

A Transfer plasmid of the invention has been delivered with the P1 delivery system to *E.* coli and *Pseudomonas in vivo* in the mouse peritonitis model. Transfer was confirmed by re-isolation of the plasmid from bacteria recovered from the intraperitoneal space, and by restriction analysis of the recovered plasmid. Results demonstrate that the delivery vehicles of the invention are capable of delivering the toxic agents of the invention to a bacterial target without toxicity to the infected subject.

The immune response to the phage and phage clearance kinetics *in vivo* has also been examined. Results indicate that single infections of 2 x 10⁹ lysogen forming units (1fu) of P1 phage per mouse resulted in the production of anti-phage antibodies in 8-14 days.

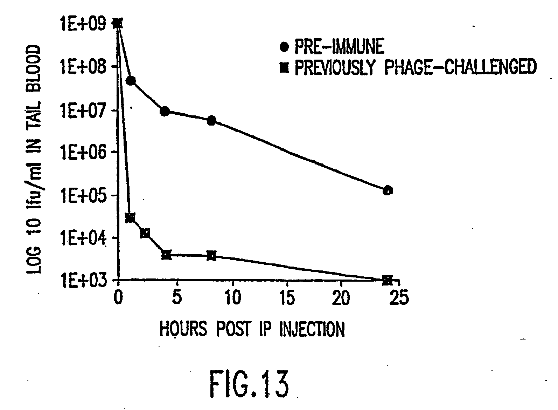

Two groups of 4 mice were injected intraperitonially (IP) with 2 x 10⁹ 1fu of long circulating P 1 phage. Peripheral blood was sampled by tail clip at 1, 4, 8 and 24 hours post injection and titered with *E. coli* C600 target cells. The previously phage-challenged group had been injected IP with an equivalent dose of the same phage preparation 18 days prior to this experiment. The pre-immune group had no prior treatments. This resulted in rapid clearing of the phage *in vivo* (Figure 13). However, for human therapeutic considerations, many infected subjects (especially for pseudomonas) will be immunocompromised and incapable of generating strong immune responses. Accordingly, the Therapeutics and compositions of the invention maybe particularly beneficial for such human subjects.

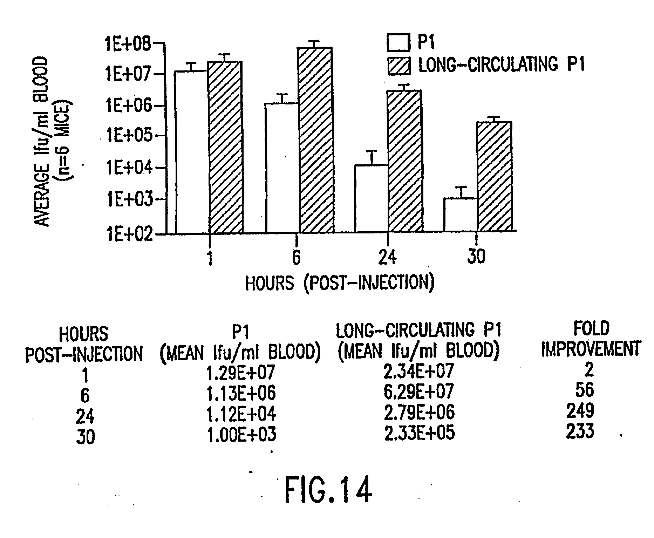

In addition, a long-circulating variant of P1 was selected by passage through mice that results in greater than 200 times more phage remaining in circulation at 24 and 30 hours after injection (Figure 14). Groups of 6 mice were injected IP with either 5x10⁸ Ifu P1 phage or 5x10⁸ Ifu long-circulating P1 phage. Peripheral blood was sampled by tail clip at 1,6,24, and 30 hours post injection and titered with *E. coli* C600 target cells. The number of viable phage remaining per ml of blood at each time-point is indicated in Figure 14. The fold improvement in persistence in the circulation is given in the last column of the table (1fu long-circulating P1 phage/lfu original P1 phage). Accordingly, use of the long-circulating P1 is within the scope of the invention. Such variant may be particularly preferable when increased concentrations of phage are desired in the circulation of an infected subject. For example, it may be desired in the case that the subject has a pathogen or bacterial infection in the blood.

### EMBRYONATED HEN EGG MODEL

In order to demonstrate efficacy of the toxic agent delivered by the P1 delivery vehicle, an embryonated hen egg model of infection has been modified from published protocols. Superficially, the hen egg model of Hartl, A, et aL, (1997, "Pseudomonas aeruginosa infection in embryonated hen's eggs" Arzneim.-Forsch. 47(II): 1061-1064), was modified in the manner in which the eggs were incubated and the shells opened and administration was Performed. Briefly, eggs were incubated in the vertical position, wide pole up, with automatic turning in a 90 degree are every 4 h. Shells were opened on the wide pole end, by reinforcing the shell with adhesive tape and cutting a round hole with a scissors through the tape and shell (opening diameter approx. 1cm). The underlying shell membrane was moistened with sterile water, then partially removed by tearing off a 1 cm² portion with a sterile forceps, which exposes the transparent chorioallantoic membrane (CAM). The shell was sealed against moisture loss with more adhesive tape and incubation continued for 18-24h. Viability was assessed at that time by candling (observing the embryo by holding the egg in front of a bright light source). Observation of spontaneous movement was evidence of viability. Viable eggs were inoculated by pipetting bacterial suspensions onto the CAM. Therapeutic agents were pipetted onto the CAM or injected through the shell at other locations by syringe. Openings in the shell were resealed with tape, incubation continued, and viability was scored at intervals by candling as above. Bacteria and phage were introduced into the egg trough an opening made in the shell, which was then sealed and gestation continued.

An embryonated hen egg model was established, as above, which harbors a variety of advantages as an *in vivo* system. Specifically, the egg model required very low LD50 (<10 cfu/egg for P. aeruginosa and >50 cfu/egg for virulent strains of *E*. *coli*), the egg model is also rapid, self contained and provides for an immature immune system. Human clinical isolates of *E. coli* and *P. aeruginosa* (PA01) consistently produce lethal infections in this model at very low doses of bacteria (100-1000 cells) allowing demonstration of the therapeutic agents. These tests show efficacy for the toxic agent such as *doe in vivo.*

In order to demonstrate the ability of the delivery vehicles of the invention to deliver a Transfer plasmid in vivo, a Transfer plasmid carrying a kanamycin resistance gene was delivered to *E*. *coli* and *P*. *aeruginosa in vivo,* in mice and in embryonated hen eggs. Results indicated that delivery of toxic agent by the P1 system is successful in vivo.

### P. aeraginosa Hen Egg Model

*In vivo* plasmid transfer in chicken embryos: The Transfer plasmid pBHR was delivered to bacterial cells by P1 phage *in vivo* in using embryonated hen eggs, Specifically, groups of six embryonated hen eggs were inoculated via the chorioallantoic membrane on the tenth day of gestation with the bacteria and phage indicated. P1 lysogen which harbors *pDoc,* a transfer plasmid which encodes the *doc* gene. This phage preparation was a mixture of particles containing either p1 DNA or *pDoc.* Phage lysates were approximately a ratio of 99:1 P1 containing phage particles to *pDoc* containing particles.

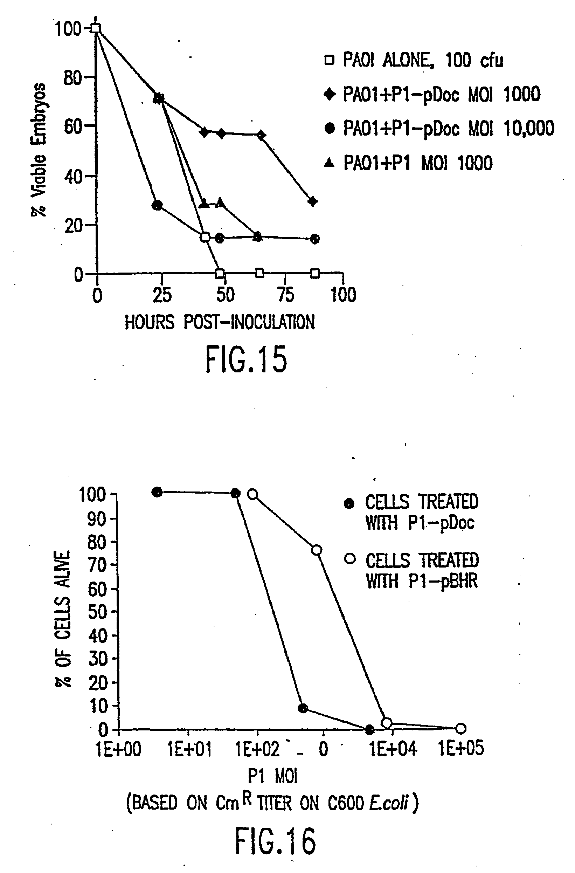

Results demonstrated increased survival of eggs when PI or P1-*pDoc* lysates are added immediately after inoculation with human clinical *P. aeruginosa* PA01 (Figure 15).

### E. coli Hen Egg Model:

A human clinical *E*. *coli* isolate which is refractory to transduction with P1 DNA has been found to produce a lethal infection in embryonated hen eggs. This isolate was designated EC-4, and is important for two reasons. First, since this strain cannot form a stable P1 lysogen, killing of EC-4 cells by *doc-*carrying phage preparations demonstrated the lethal activity of the toxic agent *doc.*

Specifically, P1-*pDoc* lysates killed *EG-4 E. coli in vitro* more efficiently than P1-pBHR phage alone (see Figure 16). Specifically, BC-4 cells (500 cfu) were treated with phage containing toxic agent *doc* (P1-*pDoc*) or control transfer plasmid pBHR (P1-pBHR) at the multiplicities of infection (MOI) shown in Figure 16, plated on non-selected media and counted as a percent of live cells treated with buffer alone. Results indicate that the toxic agent *doc* was able to render *E. coli* BC-4 less fit and increase killing of the pathogenic bacteria. Additionally, E coli. killing was confirmed *in vitro:* at a P1 MOI of 500-700, *doc* was able to kill the E. coli at a MOI 5-7, i.e. 1% of the total P1 particles (Figure 16).

Second, existence of this strain in a random sample of clinical isolates from local hospital demonstrated that there were pathogenic bacterial strains in the human population which were resistant to lytic phage therapy but susceptible to Toxic agent phage delivery system. Specifically, as shown in Table 7, below three clinical *E*. *coli* isolates were compared for their ability to be transduced with P1 DNA (as indicated by acquisition of chloramphenicol resistance) and transduced with transfer plasmid DNA (as indicated by acquisition of kanamycin resistance) relative to laboratory *E*. *coli* strain C600. All three clinical isolates were transduced with the Transfer plasmid, but only two became lysogenic with P1. These results indicate that a phage resistant mechanism was preventing transduction of P1 viral DNA was unable to prevent delivery of the transfer plasmid to EC-4 cells.

**Table 7, Clinical Isolates - susceptibility to P1 Transduction.**

| *E. coli* | Transduction with P1 DNA | Transduction with Transfer Plasmid DNA | |
|---|---|---|---|
| C600 (lab strain) | 1.9 x 10⁷lfu | 7.7 x 10⁵ lfu | C600 in transduced withP1 DNA and the transfer plasmid |
| EC-1 (Human) | 2.0x10⁷ lfu | 8.5 x 10⁶ lfn | BC-1 in transduced with P1 DNA and the transfer plasmid |
| EC-2 (Human) | 9 x 10⁶ lfu | 7.5 x 10⁶ lfu | EC-2 in transduced withP1 DNA and the transfer plasmid |
| EC-4 (Human) | 0 | 2.8 x 10⁶ lfu | EC-4 in transduced with P1 DNA and the transfer plasmid |

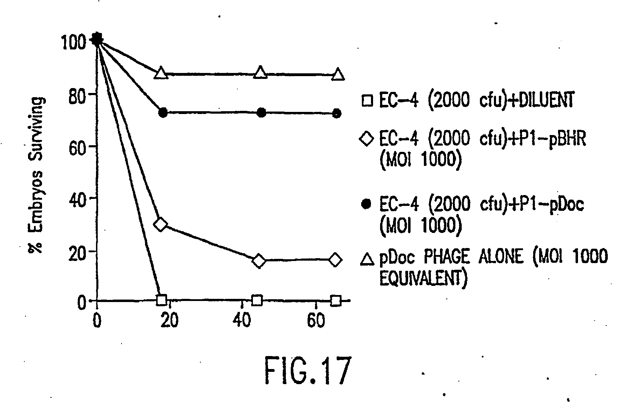

Further, an infection with 2x10³ EC-4 cells was cured in eggs by a P1-*pDoc* lysate treatment given immediately after inoculation with the bacteria at a P1 MOI of 700-800 *(doc* containing virions were 1% of total phage particles) (Figure 17). Specifically, groups of seven embryonated hen eggs were inoculated via the chorioallantoic membrane on the tenth day of gestation with the bacteria and phage indicated. P1*-pDoc* phage was produced from a P1 lysogen which harbors *pDoc,* a transfer plasmid encodes the *doc* gene or control transfer plasmid pBHR. This phage preparation was a mixture of virions containing either P1 DNA *or pDoc.* Phage lysates were approximately a ratio of 99:1, P1 containing phage particles to *pDoc* containing particles. These results indicate that a pathogenic infection may be eradicated by a therapeutic of the invention, such as *doc* via a P1 delivery vehicle.

### MAMMALIAN ANIMAL MODELS

Three mouse and rat models are used to demonstrate the efficacy of the Toxic agents of the invention. Each models uses an immunocompromised animal, which is then followed by a bacterial challenge. The models differ in the route of bacterial challenge and the means of producing the immune impairment.

In two models, immune impairment is produced in a burn model.

Specifically, a burn of 10-20% total body surface area in humans or other animals results in a period of immune impairment, involving nearly all branches of the immune system, which lasts from 10-14 days. Two bum models, well documented in the literature (see, e.g., J.P. Waymack, et al, 1988, "An evaluation of cyclophosphamide as an immunomodulator in multiple septic animal models" J. Bums and Clinical Rehabilitation 9(3):271-274; see also Stieritz, D. D. and Holder, I. A.,1975, "Experimental studies of the pathogenesis of infections due to Pseudomonas aeruginosa: Description of a burned mouse model" J. Infect. Dis. 131(6): 688-691) for experimental infections with *Pseudomonas aeruginosa,* are used to demonstrate the effectiveness of a toxic agent therapeutic against the types of infections which occur with this type of wound.

The third model utilizes the biological modulator cyclophosphamide to produce an immunocompromised state (leukopenia), in which endogenous microflora of the intestinal tract can invade the body cavity and cause sepsis. This type of sepsis has been documented in human patients with immunodeficiency (see, Furuya, et al ,1993, "Mortality rates amongst mice with endogenous septicemia caused by *Pseudomonas aeruginosa* isolates from various sources." J. Medical Microbiology 39:141-146; Woods, et al, 1997, "Correlation of *Pseudomonas aeruginosa* virulence factors from clinical and environmental isolates with pathogenicity in the neutropenic mouse" Can. J. Microbiol. 43: 541-551).

### Model 1: Adult mice, dorsal burn, wound surface bacterial challenge

The first model of use is that of Stieritz, D. D. and Holder, L A.(1975, J. Infect. Dis. 131(6): 688-691); also see Neely, A.N. and Holder, I. A., 1996, "A murine model with aspects of clinical relevance for the study of antibiotic-induced endotoxin release in septic hosts. J. Endotoxin Research 3: 229-235.). Young adult female mice, 22-25g, ICR strain (or possibly Balb/c, CD1, C3HEB/FeJ, C3H/HeJ, C57BL/6, DBA/2, A/J, CBA, C3H/HeN) are anesthetized with pentobarbitol and shaved of dorsal hair. A heat resistant plastic card with a 1 x 1.5 inch opening is placed on the shaved back, 0.5 ml ethanol pipetted onto the exposed skin and ignited for a 10 second burn. The flame is extinguished, and the mouse given 1-2 ml saline via intraperitonial (IP) injection as fluid replacement. This procedure produces a non-lethal, partial thickness bum covering 12-15% of the body surface area of a 22-25g mouse (Neely and Holder,1996, supra). One hour after the burn, and after mice have received analgesia (buprenorphine 2 mg/kg, IM), a small inocula of bacteria (100 cfu P. aeruginosa) in 0.1 ml saline is injected subcutaneously into the wound. Toxic agent treatment agent or placebo is administered either simultaneously to the same site (also 0.1 ml in saline) or by IP injection (up to 0.5 ml in saline) 1 hour after or shortly before challenge. Animals are observed for sepsis and medicated for pain (buprenorphine 2 mg/kg, 1M) at intervals not exceeding 12h. Normal diet and water is provided ad libitum. Mortality is expected in untreated burned groups within approximately 48h. Blood samples (10-25 ul) may be taken at 12-24h intervals by tail bleed to monitor bacterial load. Blood and organs are collected at time of death or euthanasia, to monitor bacterial load and confirm death from *P. aeruginosa* sepsis or clearance of infection in treated animals.

### Model 2: Adult rat, dorsal burn, IP or wound surface bacterial challenge

The second model is that of Waymack et al. (J.P. Waymack, G.D. Warden, J.W. Alexander, P.M. and S. Gonce.,1988, "An evaluation of cyclophosphamide as an immunomodulator in multiple septic animal models". J. Bums and Clinical Rehabilitation 9(3):271-274). Young male Lewis rats (100-125g) are anesthetized by IP pentobarbitol injection (~ 40 mg/kg) and shaved of dorsal hair. The animal is pressed against a heat resistant template that exposes the shaved area (20% of the total body surface area). This template is immersed in a 95°C waterbath for 10 seconds. After removal from the waterbath, the animals receive 5-10 ml Ringer's Lactate solution by IP injection for fluid maintenance therapy (approximately one half blood volume is recommended) and buprenorphine for analgesia (0.1-0.5 mg/kg, every 12h). This injury is reported to be a full-thickness burn resulting in zero mortality in the absence of further injury. A 50% lethal dose bacterial challenge (1x10⁸ cfu P. *aeruginosa* in 0.5 ml saline) is introduced by IP injection on day 4 post-bum or by painting the bacterial suspension on the wound on day 1 post-bum. The IP infection route is reported to produce sepsis within 24h (i.e. day 5 post-bum) with all deaths occurring by day 12 post-burn. Therefore, an IP injection demonstration may be terminated on day 12 post-bum. Painting of *Pseudomonas* on the bum is reported to result in sepsis 7-8 days after inoculation (day 8-9 post-burn), and survival rates are stable by day 20. Euthanasia of animals subjected to this regimen will be on day 21 post-burn. Normal diet and water will be provided ad libitum. Some animals are treatment with the therapeutic of the invention (P1 phage comprising a Transfer plasmid encoding a toxic agent) which is administered topically to the bum region or by IP injection. Blood samples (50 100 ul) may be taken by retro-orbital bleed of pentobarbitol anesthetized rats at intervals of 12-24h to monitor bacterial load. Blood and organs are collected at time of death or euthanasia, also to monitor bacterial load and confirm death from *P. aeruginosa* sepsis.

### Model3: Adult mouse, antibiotic and cyclophosphamide injections, oral bacterial challenge

This is the model of endogenous septicemia of Furuya et al.(Furuya, N., Hirakata, Y., Tomono, K., Matsumoto, T., Tateda, K., Kaku, M., and Yamaguchi, K. ,1993, "Mortality rates amongst mice with endogenous septicemia caused by *Pseudomonas aeruginosa* isolates from various sources." J. Medical Microbiology 39:141-146). Mice weighing 20-25g are housed in a sterile environment (*e.g*., in an isolator) and given sterile diet and water. IP injections of sodium ampicillin (200mg/kg) are given on days 1 and 2 to disturb normal intestinal flora and aid colonization by *P. aeruginosa.* Cyclophosphamide is injected IP (250 mg/kg) on days 6 and 9. This dose induces leukopenia without lethality in the absence of infection. The bacteria are administered to the mice in their drinking water on days 2-4. Treatment with therapeutic of the invention (P1 phage comprising a Transfer plasmid encoding a toxic agent) is started on day 9, and is administered by IP injection. Fecal pellets are be collected before bacterial challenge and at intervals throughout the infection to monitor for the absence and presence of *P. aeruginosa.* The onset of sepsis is expected 24-48 h after the second dose of cyclophosphamide (day 11), and approximately 80% mortality is expected by day 14. Signs of distress in the animals are treated with buprenorphine (2 mg/kg, twice daily or as needed). Blood samples obtained by tail bleed may also be taken at 12-24 h intervals after day 4. Alternatively, the ampicillin injections can be avoided by introducing the bacteria by IP injection the day after the final cyclophosphamide injection (Woods, D.E., Lam, J.S., Paranchych, D.P., Speert, D.P., Campbell, M., and Godfrey, A.J. ,1997, "Correlation of *Pseudomonas aeruginosa* virulence factors from clinical and environmental isolates with pathogenicity in the neutropenic mouse. Can. J. Microbiol. 43:541-551).

**Table 8: Therapeutic formulations in the following format used in the mouse models:**

| Group | Challenge | Phagemid treatment | Approximate # of survivors |
|---|---|---|---|
| 1 | Burn or cyclophosphamide (cyc) | 1000 x moi agent 1 | 6/6 |
| 2 | Burn or cyc | 1000 x moi agent 2 | 6/6 |
| 3 - 8 | Buca or cyc | 1000 x moi agent (n) | 6/6 |
| 9 | Burn or cyc +LD₁₀₀ pseudomonas | None | 0/6 |
| 10 | Burn or cyc +LD₁₀₀ pseudomonas | 1000 x moi agent 1 | 0/6 6/6 |
| 11 | Burn or cyc +LD₁₀₀ pseudomonas | 1000 x moi agent 2 | 0/6 6/6 |
| 12-17 | Burn or cyc +LD₁₀₀ pseudomonas | 1000 x moi agent 3 | 0/6 6/6 |
| Total: up to 112 mice/ model X 2 models=224 mice | | | |

**Table 9: A dose response demonstration is performed as follows:**

| Group | Challenge | Phagemid treatment | Approximate # of survivors |
|---|---|---|---|
| 1 | Burn or cyclophosphamide (cyc) | 1000 x moi | 6/6 |
| 2 | Burn or cyc +LD₁₀₀ pseudomonas | None | 0/6 |
| 3 | Burn or cyc +LD₁₀₀ pseudomonas | 1000 x moi | 0/6 6/6 |
| 4 | Burn or cyc +ID₁₀₀ pseudomonas | 100 x moi | 0/6 6/6 |
| 5 | Burn or cyc +LD₁₀₀ pseudomonas | 10 x moi | 0/6 6/6 |
| 6 | Burn or cyc +LD₁₀₀ pseudomonas | 1 x moi | 0/6 6/6 |
| Total: up to 36 mice/ model/agent x 8 agents x 2 models = 576 mice | | | |

**Table-10: Therapeutic formulations in the following format are used in the rat models:**

| Group | Challenge | Phagemid treatment | Approximate # of survivors |
|---|---|---|---|
| 1 | Burn | 1000 x moi | 6/6 |
| 2 | Burn+LD₁₀₀Pseudomonas | None | 0/6 |
| 3 | Burn+LD₁₀₀ pseudomonas | 1000 x moi | 0/6 6/6 |
| 4 | Burn +LD₁₀₀ pseudomonas | 100 x moi | 0/6 6/6 |
| 5 | Burn+LD₁₀₀pseudomonas | 10 x moi | 0/6 6/6 |
| 6 | Burn +LD₁₀₀ pseudomonas | 1 x moi | 0/6 6/6 |
| Total: up to 36 rats/agent x up to 8 agents =278 rats | | | |

Results of animal demonstrations indicate that the phage therapeutics comprising a toxic agent of the invention, is suited to treat bacterial infections of a subject. Other animal models known in the art are within the scope of the invention, including but not limited to models using calves, pigs,, lambs, guinea pig, rabbits, etc. In a preferred aspect of the invention, the subject in need of a therapeutic of the invention is a mammal with a burn injury.

### TREATMENT OF OPPORTUNISTIC INFECTIONS IN A MURINE MODEL OF CYSTIC FIBROSIS

The toxic agents of the invention are useful for the treatment of pathogenic infection such as infections associated with cystic fibrosis. As demonstration of the unility of the invention, a mouse model of pseudomonas respiratory infection is used which mimics the type of infection seen in human cystic fibrosis (CF) patients. This model uses adult (6-8 week old) mice which carry the DF508 mutation in the cftr gene (C57BL/6 DF508 mice ) and their wild type counterparts (C57BL/6 mice), or BALB/c adult mice without cftr mutations. The DF508 mutation is one of the most common mutations found in human CF patients, and the C57BL/6 DF508 mice have many symptoms similar to humans with this disease. After weaning, DF508 cftr homozygous mutants must be maintained on a liquid diet of Peptamin (Clintec Nutrition Co., Deerfield, MI) and water containing golytely (Braintree Laboratories, Braintree, MA) in order to prevent fatal bowel obstructions which are common in these mice due to their cftr mutation (see Zaidi, T. S., et al, 1999 "Cystic fibrosis transmembrane conductance regulator-mediated corneal epithelial cell ingestion of *Pseudomonas aeruginasa* is a key component in the pathogenesis of experimental murine keratitis" Infection and Immunity 67(3): 1481-1492). BALB/c mice can also be used if C57BL/6 DF508 mice are not available.

The experimental procedure is as follows (see e.g., Pier, G.B. et al.,1996, "Role of mutant CFTR in hypersusceptibility of cystic fibrosis patients to lung infections" Science 271: 64-67). Adult mice are anesthetized by intraperitoneal injection of a freshly prepared mixture of ketamine hydrochloride (65 mg/kg) and xylazine (13 mg/kg). Then with mice are held in an upright position, and 10 ul of a bacterial suspension is placed in each nostril (20 ul total). Mice are allowed to regain consciousness and then either observed for survival for up to 72 hours, or, sacrificed by CO₂ overdose at various time periods up to 24 hours after infection for determination of bacterial loads in various tissues, especially the lungs. Anesthesia is a necessary part of the infection procedure. Unanesthetized mice fail to aspirate the inoculum efficiently and do not become infected. Therapeutic phage comprising one or more toxic agents of the invention are administered, for example, intranasally, intravenously, or intraperitoneally. Mice administered the Therapeutic of the invention survive longer than the untreated control mice. Accordingly, the toxic agents of the invention may be delivered to a subject harboring a pathogenic (e.g., bacterial) infection for the purpose of ameliorating or eradicating the infection.

### 8. EXAMPLE: CONSTRUCTION & CHARACTERIZATION OF SOF SENSE RNA AS A TOXIC AGENT

In order to demonstrate that a toxic agent may be delivered and expressed using a ribozyme cassette, the inventors have engineered a toxic agent directed against an essential molecule called *Sof,* and delivered the toxic agent in a ribozyme cassette to bacterial cells to cause the death of the bacterial cells.

As described herein above, a toxic agent may be a molecule which is designed to target an essential molecule of a pathogen or selected cell. An example of an essential antisense molecule for bacteria is *Sof*. *Sof* is an antisense antidote for a chromosomally encoded toxin called *gef. Sof* normally acts to regulate the levels *of gef in* the pathogen, and thus allows the cell to survive in the presence of *gef.* The inventors of the present invention have designed sense molecules which are complementary to *Sof.* The sense molecules against *Sof* acted to inhibit the ability of *Sof* to regulate *gef,* and thus caused toxicity in the pathogen by allowing the endogenous *gef* levels to become toxic to the bacteria.

Specifically, *Sol* sense was constructed into a triple ribozyme cassette (with 5' and 3' *cis*-acting ribozymes). The ribozyme cassette containing the *Sof* sence toxic agent was linked to the LEASHI promoter. The nucleic acids encoding the ribozyme cassette were then used to transfom *E*. *coli.* Bacterial cells were plated onto LB Amp + IPTG. Plates were incubated overnight at 37°C: Plates were then scored for the presence of transformants, size of colonies, growth rate, and morphological differences.

Results of these studies indicated that expression of the *Sof* sense molecules from the ribozyme cassette lead to toxic effects in the targeted bacteria.

### 9. EXAMPLE: RIBOZYMES AND RIBOZYME CASSETTES

The ribozyme cassettes which are particularly useful in the methods of the invention include but are not limited to the following:

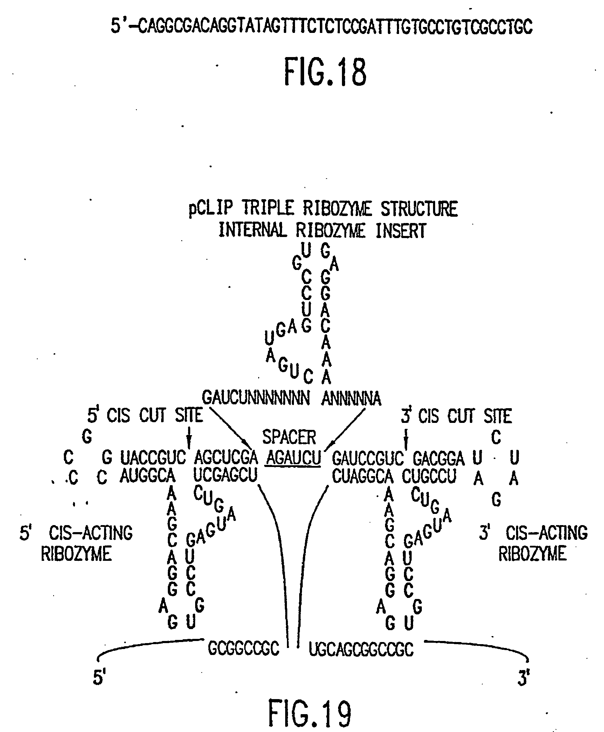

pClip (the genetic element described in Figure 19) is a modification of pBluescript, wherein the cassette shown is dropped into the *Not I* site in pBluescript. The toxic agent or trans-acting ribozyme is constructed into the *Bgl II* site (TGCTCT). Liberation of internal ribozymes or toxic agents from pClip results in a distribution of the toxic agent or ribozyme(s) to approximately 20% nuclear and 80% cytoplasmic, when delivered to a eukaryotic cell. pClip is also used to target prokaryotic cells.

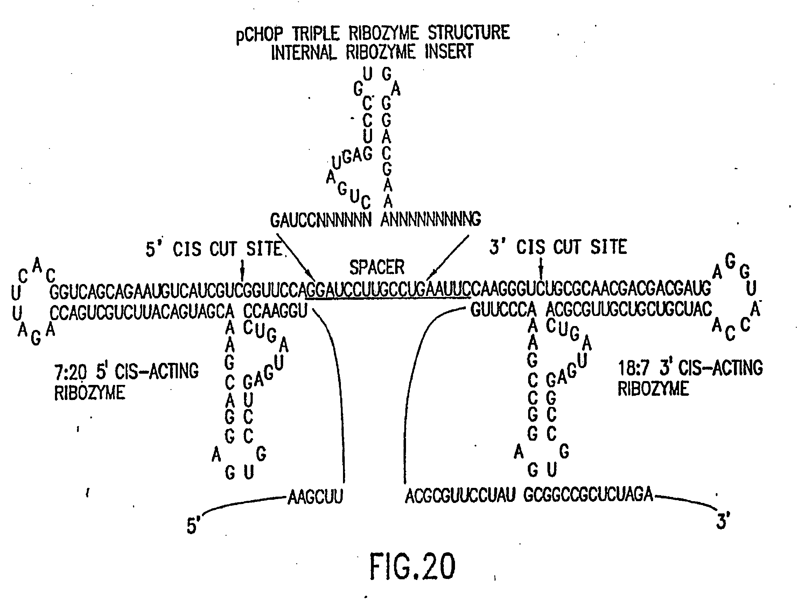

A second ribozyme cassette/vector that is useful in connection with the methods of the invention is pChop. pChop is modified from pClip to convey a more efficient and effective liberation of the internal trans-acting ribozymes or toxic agents. The pChop ribozyme cassette is diagramed in Figure 20. Liberation of internal catalytic core ribozymes from pChop increases localization to the nucleus when delivered to a eukaryotic cell.

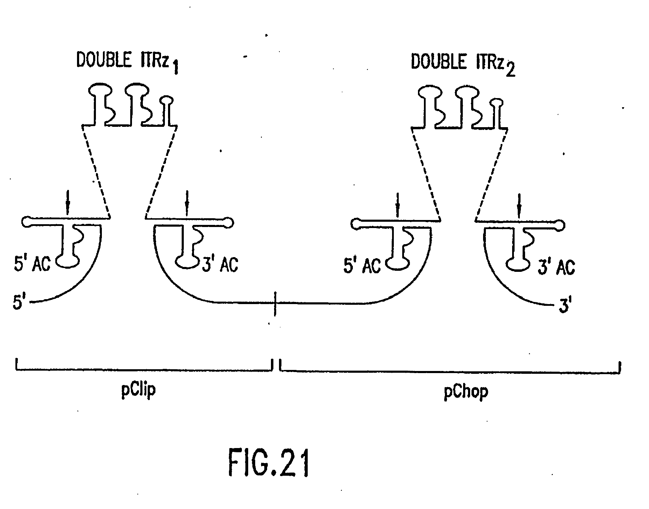

A third ribozyme cassette that was useful in connection with the methods of the invention is pSnip. The pSnip multi-ribozyme is constructed by engineering the pClip cassette 5' to pChop. In addition, the pSnip multi-ribozyme contains catalytic core sequences with two trans-acting ribozymes or toxic agents in each cassette. Each pair of trans-acting ribozymes or toxic agents is linked by a short spacer and stabilized by a hairpin loop located 3' to the pair. Figure 21 diagrams the schematic of the pSnip cassette.

A trans-acting ribozyme, or antisense toxic agent is synthesized as reverse complementary overlapping oligodeoxynucleotides, which are designed in such a way that when annealed they form single stranded ends identical to those produced by digestion with the restriction endonuclease contained with the region between the two cis-acting ribozymes. In this particular example the restriction endonuclease recognition site is that recognized by *Bgl II.* Essentially any RNA can be targeted: specificity is conferred by selecting sequences for the ribozyme that are reverse and complementary to sequences flanking the chosen cleavage site in the targeted RNA molecule. The toxic agent(s) or trans-acting ribozymes are then cloned into the cloning region(polylinker) within the double ribozyme cassette to produce the targeted toxic agent or ribozyme. Trans-acting ribozymes targeted to prokaryotic sequences have been constructed including, but not limited to, *Escherichia coli: secA (EcosecA,* AE000119 U00096), gene X (EcosecA, AE000119 U00096)*ftsZ*(AE000119;U00096), dnaG (AE000388 U00096), *rpoA*(AE000407 U00096) and tRNA-asp (X14007), *Streptomyces lividins secA* (Z50195) *, Enterococcus faecalis, ftsZ* (U94707) *Pseudornonas purida,* dnaG (U85774), *Streptomyces coelicolor rpoA* (X92107), *Staphylococcus warneri* tRNA-Asp (X66089 S42075), *Staphloccocus* RNA III.

The utility of the design using eukaryotic sequences has also been evaluated; a) repetitive B2 transcripts (B2); b) RNA polymerase I (Poll); c) Hepatitis B virus (HBV); d) Sonic Hedgehog (SH); e) Human Papillomavirus E6/E7 protein (HPV); f) RNA polymerase II (polII); g) Insulin-like Growth Factor 1 (IGF1); h) retinoblastoma protein (RB); i) and j) Multicatalytic Proteinase alpha-subunits C3 and C9 (C3 and C9, respectively); k) telomerase (tel);1) Transforming growth factor beta (TGFβ); m) catalase (CAT); n) Peroxisome proliferation associated receptor (PpaRα); and o) Cytochrome P₄₅₀ 1B1 (p4501E1); KiSS-1, NudC, Androgen Receptor, and SF-1 transcription factor. Target RNAs (with locus names and accession numbers) as well as the selected target sites are presented (Table 11).

**TABLE 11. Summary of Targeted RNAs and Target Sites**

| Target RNA | EMBL Locus | Accession | Target Site | Functional Testing | |
|---|---|---|---|---|---|
| | | | | *in vitro* | *in vivo* |
| pol II | HSRNAP14K | Z27113 | GTC₈₃ | ND | ND |
| HBV | XXHEPAV | X02496 | GTC₄₃₈ | IP | + |
| HBV | | X04615 | GTT₁₉₄₄ | + | + |
| HBV | | X02496 | GTT₁₉₄₆ | + | + |
| HCV | | M62321.1 | GTC₃₂₅ | + | + |
| RB | MUSP105RB | M26391 | GTC₂₆₄ | + | + |
| IGF1 | HUMIGF1B | M37484 | GTC₁₈₅ | ND | ND |
| SH | MMEVX1 | X54239 | GTC₅₅₈ | IP | IP |
| PolI | MUSRPA40 | D31966 | GTC₄₅₈ | + | + |
| HPV | PPH16 | K02718 | GTT₁₀₈ | IP | + |
| C3 | RATC3AA | J02897 | GTT₂₂ | + | + |
| C9 | RNPTSC9 | X533304 | GTC₁₀₁ | + | + |
| B2 | B2-Consensus | ## | GTT₂₄ | + | + |
| Tel | MMU33831 | U33831 | CTA₆₃ | ND | ND |

**Table 12:Sites Identified on HPV E6/E7 Target RNAs by Library Selection**

| HPV strains | GenBank® Accession Number | Nucleotide Triplet and Position |
|---|---|---|
| HPV11 | M14119 | CTC121 |
| HPV16 | K02718, | TTC110 |
| HPV18 | X05015 | ATC123 |
| HPV11 | M14119 | ATA443 |
| HPV16 | K02718 | GTC437 |
| HPV18 | X05015 | ATC444 |
| HPV11 | M14119 | GTC507 |
| HPV16 | K02718 | GTC506 |
| HPV18 | X05015 | ATA507 |

**Table 13: Sites on HBV RNA Identified by Library Selection Nucleotide Triplet and Site**

| Sites on HBV RNA | GenBank® Accession Number |
|---|---|
| GTC1473 | X02496 |
| CTC1534 | X02496 |
| CTC1532 | X04615,+* |
| ATC1842 | X02496 |
| ATC1840 | X04615,+* |
| GTT1946 | X02496 |
| GTT1944 | X04615,+* |
| CTC1950 | X02496 |
| CTC1948 | X04615,+# |
| TTC1948 | X75663, X75658 |

| | |
|---|---|
| * Includes but is not limited to X75664, X75657, X75665, X75663 and X75658. # Includes but is not limited to X75664, X75657, X75656, and X75665. | |

Ribozymes directed to specified target sites in HBV, Pol I and PTEN mRNA were constructed and are listed in Tables X and Y. Regarding Table X, the number following the "Rz-" prefix indicates the cutting site in the relevant mRNA. Table Y identifies the flanking sequences that correspond to the RNA sequences in the ribozyme that bind to the target site. These flanking sequences are found 5' and 3' to the catalytic core, which has the sequence 5'-UUUCGUCCUCACGGACUCAUCAG-'3 (SEQ ID NO). The cis elements of the triple ribozymes of the invention are found in the relevant disclosed vectors. In a preferred embodiment, two ribozymes (the same or different) may be combined with a short(3-15nt) linker between them.

The catalytic activities of sRz and mRz were determined using single turnover conditions. A trace amount of [³²P]-labeled target-RNA was incubated with 40 or 200 nM Rz in 5 mM MgCl₂,20 mM Tris-HCl (pH 7.4) at 37 C for 30 minutes, and the cleavage products were separated by denaturing PAGE *(see* Figures 23 and 24 for in vitro cleavage results for HBV-targeted Rzs). Three of the sRz showed "high" activity during a 30 minute cleavage reaction, cleaving between 39-44% of the target RNA using 40 nM Rz and 48-71% of the target RNA using 200 nM Rz. One of the sRz and one mRz showed "intermediate" activity; they cleaved 8-10% of target-RNA at 40 nM Rz or 10-15% at 200 nM. Another mRz was inactive. In additional experiments, when the Rz concentration was reduced to 1.6 nM, cleavage products with the 3 highly active sRz were still visible after PAGE (data not shown). Overall, 92% of sRz showed efficient activity levels, with 54% being highly active and 38% intermediately active. In contrast, none of the mRz were highly active; 50% showed intermediate or low activities, and 50% were inactive (Table X).

**Table X: Summary of Rz relative catalytic activities**

| Target | Rz, NUH | Activity (high, ###; intermediate, ##; low, #; inactive -) | | | |
|---|---|---|---|---|---|
| HBV | sRz-885, CUC | ### | | | |
| | sRz469, CUC | | ## | | |
| | sRz-408, GUC | ### | | | |
| | sRz.-777, AUC | ### | | | |
| | m1Rz-247, GUC | | ## | | |
| | m1Rz-355, GUC | | | | |
| PolI | sRz-458, CUA | ### | | | |
| | sRz-353, AUC | ### | | | |
| | sRz-595, AUC | | ## | | |
| | sRz-70, GUC | ### | | | |
| PTEN | sRz-281, AUC | | ## | | |
| | sRz-681, CUC | | ## | | |
| | sRz425, AUC. | ### | | | |
| | sRz-499, GUC | | ## | | |
| | sRz-774, CUC | | | # | |
| | m1Rz-127,CUU | | | | - |
| | mlb-151, AUU | | ## | | |
| | m1Rz-439,UUA | | | | - |
| | mlRz-760, AUC | | | | - |
| | m2Rz-227, AUU | | ## | | |
| | m2Rz-304,AUC | | | # | |
| | m2Rz-414, AUA | | | # | |
| | m2Rz-961, CUA | | | | - |
| | | (###) | (##) | *(#)* | (-) |
| | No. of sRz | 7 | 5 | 1 | |
| | % | 53.8 | 385 | 7.7 | |
| | No. of m1Rz | | 2 | | 4 |
| | % | | 333 | | 66.7 |
| | No. of m2Rz | | 1 | 2 | 1 |
| | % | | 25 | 50 | 25 |

For kinetic analyses, 40 nM Rz and 1 to 100 nM of target RNA were incubated for various periods (ranging from 20 seconds to 120 minutes), to obtain kinetic data for both single and multiple turnover conditions. Results for the HBV-targeted sRz (Figure 10 of U.S. Provisional Application No. 60/251,810, incorporated by reference herein) showed a Kₘ of 26 nM, with a K_{cat}/Kₘ of 1 x 10⁶(M⁻¹ min⁻¹). Similar analyses for the other sRz showed K_{cat}/Kₘ values of 0.6 x 10⁶ (M⁻¹ min⁻¹). In comparison, K_{cat}/Kₘ values obtained for mRz to these and other targets (Benedict et al.,1998, Carcinogenesis 19:1223-1230, Ren et al., 1999, Gene Ther. MoL Biol. 3:257-269, and Crone et al.,1999, Hepatology 29:1114-1123) are typically an order of magnitude lower.

To test the effectiveness of the sRz in cells, HepG2 cells (a human hepatoblastoma cell line), which can support HBV replication and secretion after transfection with HBV DNA (they cannot be directly infected with virus), were used. HepG2 cells were co-transfected with an HBV DNA construct and HBV-targeted sRz in the CLIP Triple ribozyme cassette (Benedict et al., 1998, Carcinogenesis 19:1223-1230, Ren et al., 1999, Gene Ther. Mol. Biol. 3:257-269, and Crone et al., 1999, Hepatology 29:1114-1123). The CLIP cassette encodes 2 cis-acting Rz flanking an internal, transacting Rz targeted to HBV. The 2 cis-acting Rz function to release themselves from the primary transcript, liberating the trans-acting internal hammerhead Rz with minimal non-specific flanking sequences, a process which affords significant advantages.

The HBV construct and the Trz constructs were co-transfected into HepG2 cells, and cultures were analyzed for the effects of sRz on HBV replication. At 4 and 5 days after transfection with the CLIP constructs containing sRz777 or sRz885, a dramatic inhibition of secretion of HBV was observed (Figure 11, Panel C, of U.S. Provisional Application No.60/251,810, incorporated by reference herein), and this was accompanied by inhibition of HbsAg secretion (Figure 11, Panel D, ofU.S. Provisional Application No.60/251,810, incorporated by reference herein) and by major reductions in HBV RNA target transcripts (Figure 11, Panels A and B, respectively, of U.S. Provisional Application No.60/251,810, incorporated by reference herein). The target sites for sRz777 and sRz885 are located in positions such that all 3 major HBV transcripts are targeted. For comparison, an mRz408 CLIP construct was also employed, which contained nucleotide substitutions in the 5' flanking sequence; this Rz showed "intermediate activity" cleaving HBV target at approximately 20% of the rate at which sRz408 did (not shown), an activity which was equivalent to that of mRz247. The mRz408CLIP construct was not effective in blocking HBV replication (Figure 11, Panels A-D, of U.S. Provisional Application No.60/251,810, incorporated by reference herein). In addition, a CLIP construct target to an mFold-selected site showed no activity against HBV in this system (data not shown).

Two additional repeat experiments with the 777 and 885 CLIP constructs also demonstrated marked reductions in secretion of HBV (see Figure 11, Panel E, of U.S. Provisional Application No.60/251,810, incorporated by reference herein), although the reductions in HbsAg secretion and HBV RNA transcripts were more variable. In related experiments, another HBV ribozyme (HBV Rz881, which when liberated from cis elements of the pCHOP vector has the sequence 5'-GGU UCC AGG AUC CAA GAG AGU CUG AUG AGU CCG UGA GGA CGA AAC UCC ACA GUG AAU UCC AAG GGU C-3' (SEQ ID NO) was shown to reduce HBV secretion from, and replication in, from mouse liver cells when complex in liposomes (Templeton et al. 1997) and injected intraperitonealy.

In summary, the selected Rz targeted to HBV have also been shown to be efficacious in a cell culture model for HBV replication.

**Table Y: Ribozyme Flanking Sequences**

| | | | |
|---|---|---|---|
| | | 9N | 6N |
| HBV | sRz-408 | 5'―TTCTCGGGG―GCTTGG―3' | |
| | sRz-469 | 5'―GGGCGCACC―TCTTTA―3' | |
| | sRz-777 | 5'―TCTGCCTAA―ATCTCT―3' | |
| | sRz-885 | 5'―TGGAGTTAC―TCGTTT―3' | |
| | m1Rz-247 | 5'―CGCAGCAGG―TGGAGC―3' | |
| | mlRz-355 | 5'―CGCGGGACG―CTTTGT―3' | |
| PolI | sRz-70 | 5'―TCGCAATGT―CATACT―3' | |
| | sRz-353 | 5'-TCATGCTGA-CCCGTC-3' | |
| | sRz-458 | 5'―CCATGCTGC―AAAGAT―3' | |
| | sRz-595 | 5'―ATATCCTCA―GCTCAG―3' | |
| PTEN | sRz-281 | 5'―TGAAGACCA―ACCCAC-3' | |
| | sRz-425 | 5'―TTATTGCA―GGGGCA―3' | |
| | sRz-499 | 5'―AAAAGGGAG―ACAATTT―3' | |
| | sRz-681 | 5'―ATATATTCC―CAATTC―3' | |
| | sRz-774 | 5'―GTAGAGTTC―CCACA―3' | |
| | m1Rz-127 | 5'―CAGAAAGAC-GAAGGT―3' | |
| | m1Rz-151 | 5'―GGAACAATA―GATGAT―3' | |
| | m1Rz-439 | 5'―GCAAATTTT―AAGGCA―3' | |
| | m1Rz-760 | 5'―GTGGTGATA―AAAGTA―3' | |
| | m2Rz-227 | 5'―TGAGAGACA―ATAACA―3' | |
| | m2Rz-304 | 5'-TAGAACTTA-AAACCC-3' | |
| | m2Rz-414 | 5'―ATTTGTGCA―TTTATT―3' | |
| | m2Rz-961 | 5'―TACTCACCC―ACAAAA―3' | |

### MATERIALS & METHODS

In Vitro Cleavage Tests. Rz targeted to the individual sites were transcribed from double-stranded DNA oligonucleotides using T7 (HBV and Pol I) or Sp6 (PTEN) polymerase as described for generation of the guide-RNA library. For standard screening of Rz activity, incubations contained trace amounts of [³²P]-labeled target RNA, 40 nM Rz RNA, and were for 30 minutes (or 2 hours) at 37 C in 20 mM Tris-HCl (pH 7.4), 5 mM MgCl₂. After incubations, samples were separated in a urea-polyacrylamide gel; the gels were then dried and radioactivity was analyzed using a Phosphor-Imager.

For kinetic analyses, a trace amount of [³²P]-labeled target-RNA was mixed with unlabeled target-RNA (to yield final concentrations of 1,10 or 100 nM target RNA) and Rz-RNA (40 nM final concentration) and incubations were performed using the same conditions as for the in vitro library selection described above, except that incubation times were varied (for 20 seconds, 40 seconds, 1 minute, 3 minutes,10 minutes, 30 minutes and 2 hours). The samples were then separated in a urea-polyacrylamide gel, and then dried and analyzed using a Phosphor-Imager.

Effects of Rz on HBV Replication in Cell Culture. To test the effectiveness of sRZ in cell culture, HepG2 cells were maintained in minimal essential medium supplemented with 10% heat-inactivated fetal bovine serum, in a humidified incubator at 30 C with 5% CO₂. These cells were co-transfected with pBB4.5HBV1.3 (a 1.3X unit length HBV DNA plasmid construct; see Delaney & Isom, 1998, Hepatology 28:1134-2246) and either pLSCLIP, pLSCLIPmRz408, pLSCLIPsRz777, or pLSCLIPsRz885 (pLSCLIP denotes the CLIP cassette in the LacSwitch vector, from Stratagene). pLSCLIPsRz777 and pLSCLIPsRz885 were constructed by annealing reverse complementary oligonucleotides (CLAW437/CLAW438 and CLAW397/CLAW398, respectively) and then inserting them into the Bgl II site of pLSCLIP. pLSCLIPmRz408 was constructed the same way with oligonucleotides CLAW435/CLAW436. However, these oligonucleotides were inadvertently synthesized so that the 5' flanking region contained mismatches; subsequent testing in vitro showed that this Rz had approximately 20% of the catalytic activity of the sRz408, which was equivalent to the activity of mRz247, and it was therefore included in the experiments as an "intermediate" comparison.

HepG2 cells were transfected using FuGENE6 transfection reagent (Boehringer Mannheim). A total of 5 µg of DNA (0.5 µg pBB4.5HBV1.3 and 2.7 µg of the PLSCLIP constructs), 24 µl of enhancer, and 30 µl of Effectene transfection reagent. The cells were incubated in the DNA/reagent mixture in serum-containing medium for 6 hours.

For Northern blot analyses, total RNA was isolated from transfected HepG2 cells four and five days post-transfection (Chomczynski & Sacchi, 1987, Anal Biochem 162:156-159), and Northern Blot analysis was performed using 10 µg of total RNA as described (Davis et al., 1986, Preparation and analysis of RNA from eukaryotic cells. In: Basic Methods in Molecular Biology, New York: Elsevier Science Publishing Co., Inc., 129-156). Hybridization was performed using a [³²P]-radiolabeled HBV probe generated by random priming (with Boehringer Mannheim Random Prime DNA Labeling ktis). The blots were probed simultaneously for HBV and GAPDH transcripts. Following hybridizations, the blots were rinsed under high-stringency conditions and exposed for audoradiography.

For analysis of secreted extracellular HBV DNA, medium was collected on day 4 and day 5 post-transfection, and centrifuged at 6,000 x g for 5 minutes to remove cellular debirs. Triplicate samples were pooled and HBV particles were precipitated and analyzed as described (Wei et al., 1996 J. ViroL 70:6455-6458). Viral pellets were resuspended in PBS and digested with Proteinase K, then extracted with phenol/chloroform. DNA was precipitated with 0.1 volume of 3 M sodium acetate and 1 volume of isopropanol. Ten micrograms oftRNA was added as a carrier during precipitation. Pellets were resuspended in TB and digested with 0.5 mg/ml RNase for 1 hour. DNA was then analyzed by clectrophoresis and Southern blotting, followed by autoradiography.

For analysis of secreted HBV Surface Antigen (HbsAg), detection was performed by radioimmunoassay using a Sorin Diagnostics kit. Medium from transfected cells was collected and centrifuged at 6,000 x g to remove cellular debris. Total counts were compared for analysis.

### 10. EXAMPLE: PREFERRED TARGET SEQUENCES AND SEVERAL PREFERRED DNAZYMES

The human papilloma virus (HPV) and hepatitis B virus (HBV) target sites identified in the instant disclosure are useful as sites for the hybridization of antisense oligonucleotides, DNAzymes or ribozymes targeted against these sites. Preferred targets are HPV E6/E7 mRNA or the core, pre-core or polymerase-encoding sequences of hepatitis viruses.

A preferred embodiment of a DNAzyme specific for HPV16, denoted HPV16-Dz57 is the following: 5'-TGTGGTAAGGCTAGCTACAACGATTTCTGGG-3'. The catalytic core is underlined and the flanking 5' and 3' sequences hybridize under physiological conditions to the target sequence identified as SEQ ID NO:AA.

In general, the DNAzymes of the present invention are made by adding 5' and 3' flanking sequences to a catalytic core sequence. The 5' and 3' flanking sequences are designed so as to be complementary to a target sequence of interest. For example, the target sequences identified in Table 14 (SEQ ID NOs:AA-BD) may be used for the design ofDNAzymes specific for each of the disclosed target sites. Table 14 shows DNA sequences corresponding to the sense orientation of target mRNAs. The DNAzymes in Table 15 have flanking sequences that are the reverse complement of the Table 14 sequences. Underlined in each target sequence (SEQ ID NOs:AA-BD) is the cleavage site where the DNAzyme cuts the RNA corresponding to the target site. A DNAzyme of the present invention is designed by identifying the nucleic acids flanking but not including the underlined cleavage site of interest in a specific target sequence and designing complementary nucleic acid sequences that will bind to the target sequence flanking the cleavage site. The complementary sequences may be synthesized chemically using techniques well known in the art and joined to the 5' and 3' ends of the catalytic core of the DNAzyme. The complementary nucleic acid sequences that are attached to the 5' and 3' ends of the catalytic core of the DNAzyme provide specificity for the target site of interest because the 5'and 3' flanking sequences specifically hybridize under physiological conditions to the target site of interest. The 5' and 3' flanking sequences that are attached to the DNAzyme catalytic core may be from 6-15 nucleotides in length, more preferably, they may be from 7-10 nucleotides in length, even more preferably, they may be from 8-9 nucleotides in length. In specific embodiments of the instant invention, the DNAzymes set forth in Table 15 (SEQ ID NOs:BE-BQ) are designed to be specific for the HPV target sequences disclosed as SEQ ID NOs:AA-AM, respectively. As will be readily apparent to a person of ordinary skill in the art, the design of DNAzymes specific for the target sequences of the instant specification may be accomplished using the strategy described in detail above. The specific exemplifications herein are in no way intended to limit the scope of the invention.

**Table 14: Target Sequences**

| Target | Accession No. | Cutting Site | Target Sequence | SEQ IDNO |
|---|---|---|---|---|
| HPV16 | K02718 | 136 | CCCAGAAAGTTACCACA | AA |
| | | 145 | TTACCACAGTTATGCAC | AB |
| | | 227 | GACGTGAGGTATATGAC | AC |
| | | 264 | CATAGTATATAGAGATG | AD |
| | | 328 | ATTAGTGAGTATAGACA | AE |
| | | 352 | TATAGTTTGTATGGAAC | AF |
| | | 492 | TATAAGGGGTCGGTGGA | AG |
| | | 504 | GTGGACCGGTCGATGTA | AH |
| | | 3915 | GTGCTTTTGTGTGTCTG | AI |
| | | 4015 | GCCTCTGCGTTTAGGTG | AJ |
| HPV11 | M14119 | 128 | GCCTCCACGTCTGCAAC | AK |
| | | 436 | GCCGTTGTGTGAAATAG | AL |
| | | 505 | GTGGAAGGGTCGTTGCT | AM |
| HBV | V01460 | 1473 | TICTCGGGGTCGCITGG | AN |
| | | 1842 | TCTGCCTAATCATCTCT | AO |
| | | 1950 | TGGAGTTACTCTCGTIT | AP |
| HPV16 | K02718 | 110 | TGCAATGTTTCAGGACC | AQ |
| | | 437 | GCCACTGTGTCCTGAAG | AR |
| | | 506 | GTGGACCGGTCGATGTA | AS |
| HPV11 | M14119 | 121 | AAAGATGCCTCCACGTC | AT |
| | | 443 | TGTGTGAAATAGAAAAA | AU |
| | | 507 | GTGGAAGGGTCGTTGCT | AV |
| HPV18 | X05015 | 123 | CTTTGAGGATCCAACAC | AW |
| | | 444 | ACCGTTGAATCCAGCAG | AX |
| | | 507 | TGGGCACTATAGAGGCC | AY |
| HPV16 | K02718 | 138 | CCCAGAAAGTTACCACA | AZ |
| | | 147 | TTACCACAGTTATGCAC | BA |
| | | 266 | CATAGTATATAGAGATG | BB |
| | | 330 | ATTAGTGAGTATAGACA | BC |
| | | 354 | TATAGTTTGTATGGAAC | BD |

**Table 15. DNAzymes specific for HPV target sequences. The bold underlined portion of each sequence represents the catalytic core of the DNAzyme; the 5' and 3' flanking sequences hybridize to the target sequence.**

| Target No. | DNAzyme | SEQ ID NO |
|---|---|---|
| HPV16-1 | 5'-TGTGGTAA**GGCTAGCTACAACGA**TTTCTGGG-3' | BE |
| HPV16-2 | 5'-GTGCATAA**GGCTAGCTACAACGA**TGTGGTAA-3' | BF |
| HPV16-3 | 5'-GTCATATA**GGCTAGCTACAACGA**CTCACGTC-3' | BG |
| HPV16-4 | 5'-CATCTCTA**GGCTAGCTACAACGA**ATACTATG-3' | BH |
| HPV16-5 | 5'-TGTCTATA**GGCTAGCTACAACGA**TCACTAAT 3' | BI |
| HPV16-6 | 5'-GTTCCATA**GGCTAGCTACAACGA**AAACTATA-3' | BJ |
| HPV16-7 | 5'-TCCACCGA**GGCTAGCTACAACGA**CCCTTATA-3' | BK |
| BPV16-8 | 5'-TACATCGA**GGCTAGCTACAACGA**CGGTCCAC-3' | BL |
| HPV16-9 | 5'-CAGACACA**GGCTACCTACAACGA**AAAAGCAC-3' | BM |
| BPV16-10 | 5'-CACCTAAA**GGCTAGCTACAACGA**GCAGAGGC-3' | BN |
| HPV11-1 | 5'-GTTGCAGA**GGCTAGCTACAACGA**GTGGAGGC-3' | BO |
| HPV11-2 | 5'-CTATTTCA**GGCTAGCTACAACGA**ACAACGGG-3' | BP |
| HPV11-3 | 5'-AGCAACGA**GGCTAGCTACAACGA**CCTTCCAC-3' | BQ |

### 11. EXAMPLE: EFFECTS OF ANTISENSE OLIGONUCLEOTIDES TARGETED TO AN HBV SITE IN TRANSGENIC MICE

As shown in the data presented in the following Tables 16 to 21, a DNAzyme (Dz879, 5'-GAG AGT AAG GCT AGC TAC AAC GAT CCA CAG T-3', SEQ ID NO: ) or its catalytically inactive counterpart (i.e., an antisense oligonucleotide, mDz879 5'-GAG AGT AAG CCT AGC TAC TAC GAT CCA CAG T-3'), was effective in reducing HBV replication and secretion in a transgenic mouse that expresses human HBV, i.e., in vivo.

### Table 16: Effects of DNAzyme (Dz879) or the catalytically inactive counterpart (m879) on serum HBV genome equivalents in HBV Transgenic Mice. 50

ug of Dz879 or m879 were administered in asialofetuin-coated liposomes twice per week for 2 weeks, and sacrificed 48 h after the final treatment.

As is evident, both Dz879 and m879 were effective in reducing HBV secredon in vivo after 2 weeks of treatment. However, the effect was diminished after 5 weeks, presumably because of an immune response to the asialofetuin.

| Serum HBV Genome Equivalents/ml (x10⁻³) | | | | |
|---|---|---|---|---|
| Group 1 | Group 3 | Group 5 | Group 7 | Group 9 |
| Dz, 2 weeks Control | Dz. 5 weeks | mDz, 2 weeks | mDz, 5 week | |
| 0.4±38 | 3.29±3.35 | 0.74±0.85 | 4.10±3.19 | 6.57±3.31 |
| P values, Student's t-test | | | | |
| 0.0025 | 0.072 | 0.003 | 0.180 | |

### Table 17:Effects of Dz879 and m879 on HBV Core Ag in liver of HBV Transgenic Mice.

Dz879 and m879 were administered in asialofetuin-coated liposomes as described. Liver tissue was obtained, fixed, processed, and immunohistochemistry was performed for HBV Core antigen around central veins. As is evident, there is a dramatic reduction in staining for HBV Core antigen after 2 weeks. In addition, the intensity of staining was also greatly reduced, indicating an even more marked effect than is shown by the cytoplasmic staining numbers.
*Animals:* Female Transgenic mice (founder 13.32)
*Treatment schedule:* twice per week, (Tue, Friday) X 2 or 5 weeks
*Virus:* Human hepatitis B virus
*Treatment route:* i.p.
*Drug:* Prepared at Penn State sent to USU
*Experiment duration:* 2 or 5 weeks

| | Mean HbcAg-stained cytoplasms/total cells^{a} ± standard devation (n^{b}) | |
|---|---|---|
| Treatment | Day 14^{e} | Day 35 |
| CL-ASF-DNAzyme | 0.04±0.05 (10)*** | 0.14±0.12(10)*** |
| CL-ASF-DNAzyme mutant | 0.15±0.13(10)*** | 0.30±0.15(9)* |
| No Treatment | 0.47±0.18 (10) | |

| | | |
|---|---|---|
| "Number of stained cytoplasms per total number of cells around humen of central veins. Average of 5 veins counted. ^{b}Number of animals in each treatment group. ^{c}Days after initial treatment *P<0.05, compared to no treatment. ***P<0.001, compared to no treatment. | | |

### Table 18. Effects of Dz879 and m879 on HBV RNA Transcripts in HBV Transgenic Mice.

Dz879 and m879 were administered as described, and liver tissue was extracted for RNA. RNA was analyzed by Northern blot analysis followed by densitometry; results showed major reductions in HBV RNA transcript levels (all transcripts, as was the case with cell culture results).
*Animals:* Female Transgenic mice (founder 1.332)
*Treatment schedule:* twice per week, (Tue, Friday) 2 weeks
*Virus:* Human hepatitis B virus
*Treatment route:* i.p.
*Drug*: Prepared at Penn State sent to USU
*Experiment duration:* 2 or 5 weeks

| Treatment | Relative Liver HBV RNA^{a} ± SD (n^{b}) |
|---|---|
| CL-ASF-DNAzyme | 7.0±3.5 (10) * |
| CL-ASF-DNAzyme mutant | 9.4±5.7(10)* |
| No Treatment | 16.0±5.6(10) |

| | |
|---|---|
| ^{a}Mean signal of HBV RNA divided by signal of GAPDH housekeeping gene using Northern blot analysis ± standard deviation. ^{b}Number of animals in each treatment group. *P<0.001, compared to no treatment group. Note: The RNA samples from 5 week-treatment were ofpoor integrity and difficult to measure. | |

### Table 19: Effects of Dz879 and m879 on HBV liver DNA in Transgenic Mice.

Dz879 and m879 were administered in asialofetuin-coated liposomes. Liver tissue was extracted for DNA, and HBV genomic DNA was quantitated by cross-over PCR. Administration of Dz879 and m879 resulted in a dramatic reduction in HBV liver DNA.
*Animals:* Female Transgenic mice (founder 1.3.32)
*Treatment schedule:* twice per week, (Tue, Friday) X 2 or 5 weeks
*Virus:* Human hepatitis B virus
*Treatment route:* i.p.
*Drug:* Prepared at Penn State sent to USU
*Experiment duration:* 2 or 5 weeks

| | Liver HBV DNA Mean log₁₀ fg/ug cel DNA ± sd (n^{a}) | |
|---|---|---|
| Treatment | Day 14^{b} | Day 35 |
| CL-ASF-DNAzyme | 1.9±0.22 (10)* | NT^{c} |
| CL-ASF-DNAzyme mutant | 1.75±0.21(10)* | NT^{d} |
| No Treatment | 4.11 ± 0.34 (8) | |

| | | |
|---|---|---|
| ^{a}Number of animals in each treatment group. ^{b}Days after initial treatment. ^{c}Controls from another experiment (ISA-2, control) ^{d}Faulty preparation of samples, invalid results. ^{*}P<0.01, compared to no treatment. | | |

### Table 20:

Effects of Dz879 or a control DNAzyme on HBV liver DNA in Transgenic Mice. This represents a repeat experiment showing marked effects of the DNAzyme on liver HBV DNA. Further experiments are being performed to determine if the catalytic core of the DNAzyme, or its rapid breakdown from oligonucleotides to monophosphate nucleotides may be responsible for the observed effects in the placebo group. Gene Expression analysis has shown increased expression of deoxycytidine kinase, for example, while indicating that cytokines are not responsible for the effects.
*Animals:* Female Transgenic mice (founder 1.3.32)
*Treatment schedule:* see below
*Virus:* Human hepatitis B virus
*Treatment route:* i.p.
*Drug:* Prepared at Penn State sent to USU
*Experiment duration:* 3 weeks

| Therapeutic | Dosage | Treatment Schedule | Liver HBV DNA mean log₁₀ fg/ug cell DNA±SD(n^{a}) |
|---|---|---|---|
| Group 13, DNAzyme | 50ug/ injectio n | Treat day 0,2,6,9, necropsy day 12 | 1.87 ±.059*** |
| Group 15, CL-ASF-placebo | | Treat day 0,2,6,9, necropsy day 12 | 1.79 ± 0.21 *** |
| Group 17, untreated | | - | 4.11±034 |

| | | | |
|---|---|---|---|
| ^{a}Number of animals in each treatment group. | | | |

### Table 21: Effects of Dz879 or a control DNAzyme on HBV Core Antigen Staining in Transgenic Mice.

*Animals:* Female Transgenic mice (founder 1.3.32)
*Treatment schedule:* see below
*Virus:* Human hepatitis B virus
*Treatment route:* i.p.
*Drug:* Prepared at Penn State sent to
*Experiment duration:* 3 weeks

| Therapeutic | Dosage | Treatment Schedule | Mean HbcAg-stained^{a} |
|---|---|---|---|
| Cytoplasm/Total cells | | | ± standard deviation (n^{b}) |
| Group 13, DNAzyme | 50ug/injecti on | Treat day 0,2,6,9, necropsy day 12 | 0.08±0.09(7)* |
| Group 15, CL-ASF-placebo | | Treat day 0,2,6,9, necropsy day 12 | 0.09±0.09(8)* |
| Group 17, untreated | | - | 0.34 ± 0.25 (6) |

| | | | |
|---|---|---|---|
| ^{a}Number of animals in each treatment group. ^{b}Days after initial treatment. *P<0.05, compared to group 17. | | | |

### 12. EXAMPLE: USE OF MULTIPLE DNAZYMES TO INHIBIT PAPILLOMA GROWTH IN COTTONTAIL RABBITS

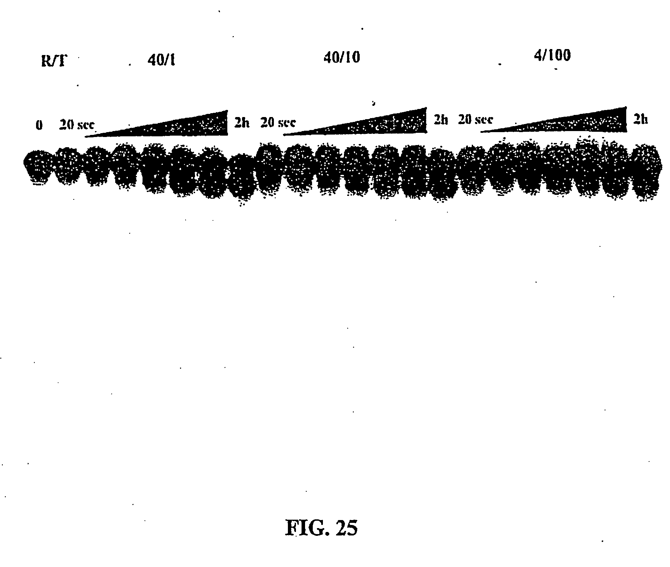

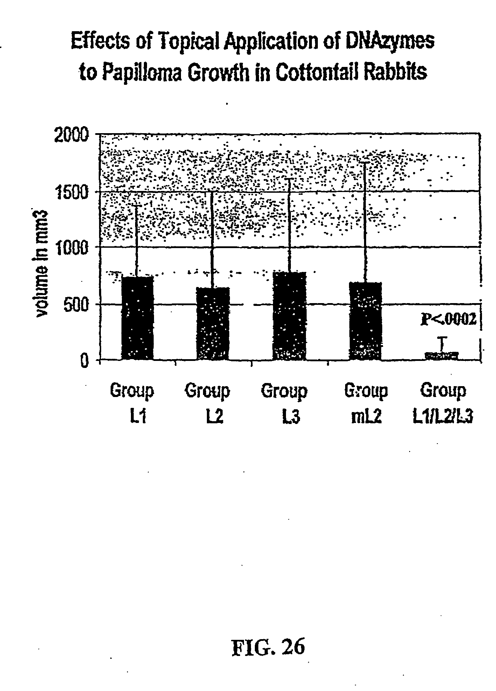

DNAzymes were also tested in the model cottontail rabbit system. Sections of cottontail rabbit skin were lightly abraded, and a suspension of Shope Papilloma Virus was applied. After 10 days, DNAzymes in saline solution were applied topically at 30µg/day for 4 weeks, and then at 60 µg/day for 2 additional weeks. The following DNAzymes were tested individual and as mixture: Group L1 (SBQ ID NO WW); Group L2 (SEQ ID NO XX); Group L3 (SEQ ID NO YY). The target sites for the DNAzymes were selected by homology to previously-identified HPV sites. A catalytically-defective DNAzyme (Group mL2, SBQ ID NO ZZ) was also tested. Papillomas were then measured, and mean volumes calculated. As shown in Figure 25, the individual DNAzymes and the catalytically defective DNAzyme were not effective in inhibiting papilloma growth. However, a mixture of all three DNAzymes (L1/L2/L3) was highly effective in reducing papilloma growth.
TGCCGGGAGGCTAGCTACAACGATCGGGGCT (SEQ ID NO WW) is LSI DNAzyme to Shope Papilloma Virus (GenBank® Accession No. AJ404003) with cleavage site at nucleotide 614.
CACAGAAAGGCTAGCTACAACGAAGACTGAA (SEQ ID NO XX) is LS2 DNAzyme to Shope Papilloma Virus (GenBank® Accession No. AJ404003) with cleavage site at nucleotide 935.
TATAGAAGGGCTAGCTACAACGAAGCCCTGC (SEQ ID NO YY) is LS3, a DNAzyme to Shope Papilloma Virus (GenBank® Accession No. AJ404003) with cleavage site at nucleotide 1006.
CACAGAAAGCCTAGCTACTACGAAGACTGAA (SEQ ID NO ZZ) is mLS2, a catalytically defective DNAzyme to Shope Papilloma Virus (GenBank® Accession No. AJ404003).

### 13. EXAMPLE: DELIVERY AND IN VIVO TESTING

### Biologic Delivery

The toxic agents and/or ribozymes of the present invention may be delivered by a wide variety of viral vectors and bacteriophage as described herein, and exemplified herein above.

In one embodiment of the invention, a toxic agent is encoded in a Transfer plasmid, and is used in connection with a P1 bacteriophage delivery system. Such Transfer plasmid preferably contains 1) an origin or replication 2) selectable marker 3) P1 PAC site and PAC ABC genes 4) P1 lytic replicon 5) nucleic acids encoding one or more toxic agents of the invention. In a preferred embodiment of the invention, the bacteriophage P1 prophage (P1 plasmid) is engineered such that viral DNA can not be packaged into virions, such as, for example, by deletion of the PAC site from the P1 plasmid.

In another embodiment, the toxic agents and/or ribozymes may be delivered via a plasmid encoding the toxic agents and/or ribozymes, a plasmid origin of replication, a selectable marker for plasmid maintenance, the minimal lambda origin of replication, and cos sites, which are required for packaging of DNA into lambda virions. This plasmid is maintained in a lambda lysogen that is defective in integration/excision and recombination functions. The defective lysogen provides all of the replication factors needed to activate the lambda origin of replication on the plasmid and all of the structural components needed to form mature virions; however, the lysogen is not able to replicate and package its own DNA into the virions. The lysogen also carries the cI⁸⁵⁷ temperature-sensitive repressor mutation. Induction of the lysogen by temperature shift to 42 ° C or by other means, such as exposure to 5J/m2 of ultraviolet radiation will mobilize the plasmid and result in its replication and packaging into lambda virions. The virions can then be harvested, purified free of *E. coli* proteins and be used to deliver the toxic agents and/or ribozyme gene(s) to *E*. *coli.* Similar methods are performed for *Pseudomonas aeruginosa* in order to deliver a toxic agent and/or ribozyme to P. *aeruginosa.*

### Abiologic Delivery

Abiologic delivery of the toxic agent and/or ribozymes is accomplished with constructs that have been engineered to be expressed within the targeted tissue or pathogen. Briefly, the genetic element containing the promoter and the toxic agent and/or ribozyme(s) are complexed with cationic liposomes (Lipofectamine-Gibco BRL) in a 1:10 ratio and are introduced into test animals by either single or multiple injection of 0.2 ml total volume nucleic acid-liposome mixture.

### Prophylactic Administration and Prevention of Acute Baderial Infection

Following the demonstration that toxic agents and/or ribozymes of the present invention have an *in vitro* biological activity (either directly on bacterial cultures or in an infectious tissue culture cell assay system), the effectiveness of the toxic agents and/or ribozymes, is shown in *in vivo* model systems, *e.g.,* as described above. To demonstrate the efficacy of toxic agents and/or ribozymes of the invention *in vivo,* experimental animal model systems are utilized, such as those described herein. For an initial demonstration of the efficacy of the toxic agents and/or ribozymes in *vivo,* mice are infected with a microbial pathogen which has previously been shown to be sensitive to the toxic agents and/or ribozymes construct(s) and the effect of toxic agents and/or ribozymes administered *in vivo* is determined. In the first series of *in vivo* trials, one determines the effectiveness of toxic agents and/or ribozymes at preventing an acute infection in a murine model system when the toxic agents and/or ribozymes is added directly to the microbe prior to administration *in vivo.*

The next series of trials demonstrates that the administration of toxic agents and/or ribozymes after infection is effective at preventing an acute bacterial infections. In addition to the clinical status of infected mice, tissues obtained at necropsy are examined histologically and the presence of replicating microorganism in tissue samples is determined by standard methodology. Animals can be infected by various routes (systemic and/or mucosal) and the toxic agents and/or ribozymes are delivered over time after infection by systemic, mucosal, or topical routes. Both abiologic as well as biological delivery of the toxic agents and/or ribozymes is used. The demonstration of a positive effect of the toxic agents and/or ribozymes in controlled experimental model system provides compelling evidence for the efficacy of the preparation and determines whether or not the preparation warrants evaluation under conditions of standard clinical trials.

The present invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

Throughout this application various publications are referenced. The disclosures of these publications in their entireties are hereby incorporated by reference into this application in order to more fully describe the state of the art to which this invention pertains.

## Claims

1. A purified preparation of at least one nucleic acid molecule that specifically hybridizes under physiological conditions to mRNA encoding at least one viral protein associated with transformation or plasmid copy number control or which hybridizes to a viral polyadenylation signal or a core, pre-core or polymerase encoding sequence, wherein at least one sequence is selected from the group consisting of SEQ ID NOs: AH, AA to AG, AI to AZ and BA to BD or a homologous sequence thereof.

2. A purified preparation of at least one nucleic acid molecule of claim 1, wherein said mRNA is E6/E7 mRNA.

3. A purified preparation of at least one nucleic acid molecule of claims 1 or 2, wherein said mRNA is papilloma viral RNA or hepatitis B viral RNA.

4. A purified preparation of at least one nucleic acid molecule that specifically hybridizes under physiological conditions to a sequence selected from the group consisting of SEQ ID NOs: AH, AA to AG, AI to AZ and BA to BD or a homologous sequence in a related HPV or HBV strain.

5. A purified preparation of at least one nucleic acid molecule of any one of claims 1-4, wherein said nucleic acid molecules are DNAzymes, antisense oligonucleotides or ribozymes.

6. A purified preparation of at least one nucleic acid molecule of any one of claims 1-4, wherein said nucleic acid molecules are DNAzymes.

7. A purified preparation of at least one nucleic acid molecule of any one of claims 1-4, wherein said nucleic acid molecules are antisense oligonucleotides.

8. A purified preparation of at least one nucleic acid molecule of any one of claims 1-4, wherein said nucleic acid molecules are ribozymes.

9. A purified preparation of at least one nucleic acid molecule of any one of claims 1-8, wherein said nucleic acid molecules are resistant to nuclease degradation.

10. A purified preparation of at least one nucleic acid molecule of claim 9, wherein said nucleic acid molecules are modified at their 3'ends, are resistant to nuclease degradation and have a 3'-3' inverted T at their 3' ends.

11. A pharmaceutical composition comprising any of the nucleic acid molecules of claims 1-10 and a pharmaceutically acceptable carrier.

12. A method of treating papillomavirus-induced conditions or hepatitis virus-induced conditions comprising administering to a subject a pharmaceutical composition of claim 11.

13. A method of claim 12, wherein said administration comprises topical application to the cervix.

14. A method of claim 12, wherein said administration comprises topical application to the epidermis.

15. A pharmaceutical composition of claim 11, wherein said nucleic acid molecules are formulated as a cosmetic formulation.

16. A pharmaceutical composition of claims 11 or 15, wherein said nucleic acid molecules are formulated in an amount sufficient to produce a cytotoxic or cytostatic effect in cells infected with papillomavirus or heptatis B virus.

17. A pharmaceutical composition of claim 16, wherein said cells are transformed by a papillomavirus.

18. A pharmaceutical composition of claim 16, wherein said cells are transformed by a hepatitis B virus.

19. A pharmaceutical composition of claims 16-18, wherein said nucleic acid molecules are formulated for topical administration.

20. A pharmaceutical composition of claim 19, wherein said nucleic acids are formulated into an ointment, salve, gel, cream or lotion.

21. A pharmaceutical composition of any of claims 11 or 15-20, wherein said nucleic acid molecules are formulated into a liposome preparation or a lipid preparation.

22. A method of any one of claims 12-14, wherein said papillomavirus-induced condition is selected from the group consisting of warts, cervical dysplasia, cervical carcinoma, carcinoma in situ and laryngeal papilloma.

23. A method of claim 22, wherein said administration is to epithelial cells.

24. A method of claim 23, wherein said epithelial cells are selected from the group consisting of squamous epithelia, cutaneous epithelia and mucosal epithelia.

25. The pharmaceutical composition of any of claims 11 and 15-21 wherein said one or more nucleic acids are formulated for delivery in a liposome.

26. The pharmaceutical composition of claim 25 wherein said liposome is capable of tissue-specific uptake in the liver.

27. The pharmaceutical composition of claim 26 wherein said liposome is modified using asialofetuin or one or more sugars.
